# EUROPEAN PATENT APPLICATION

(11) **EP 3 527 578 A1**
(43) Date of publication of application: **21.08.2019**
(21) Application number: 17859723.3
(22) Date of filing: 13.10.2017
(51) Int. Cl.: C07H 19/067, C07C 319/14, C07C 319/20, C07C 323/12, C07H 19/167, C07H 21/02, C07H 23/00

(54) **NOVEL GLYCOSIDE COMPOUND AND PRODUCTION METHOD THEREFOR**

(30) Priority: 14.10.2016 JP 2016202633; 17.03.2017 JP 2017053633
(71) Applicant: Bonac Corporation, Kurume-shi, Fukuoka 839-0861 (JP)
(72) Inventor: OHGI, Tadaaki, Kurume-shi Fukuoka 839-0861 (JP); AOKI, Eriko, Kurume-shi Fukuoka 839-0861 (JP); KINOSHITA, Takashi, Kurume-shi Fukuoka 839-0861 (JP); ITOH, Akihiro, Kurume-shi Fukuoka 839-0861 (JP); EMURA, Chisato, Kurume-shi Fukuoka 839-0861 (JP)
(74) Representative: J A Kemp
(86) International application number: PCT/JP2017/037279
(87) International publication number: WO 2018/070543

(57) **Abstract**

The present invention aims to provide a method of producing, more efficiently at a high purity, a phosphoramidite preferable for the production (synthesis) of a nucleic acid. Using a coupling reaction of an ether represented by the following chemical formula (105), an enantiomer, tautomer or stereoisomer thereof, or a salt thereof, and a glycoside compound, phosphoramidite that enables efficient synthesis of nucleic acid can be obtained: 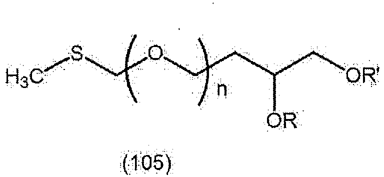 wherein n is a positive integer, and R and R' are the same or different and each is a hydrogen atom or a hydroxyl-protecting group.

SEQUENCE LISTING

<110>  BONAC CORPORATION

<120>  NEW GLYCOSIDE COMPOUND AND PRODUCING METHOD THEREOF

<130>  092661

<140>  JP2016-202633
<141>  2016-10-14

<140>  JP2017-053633
<141>  2017-03-17


<160>  3

<170>  PatentIn version 3.5

<210>  1
<211>  21
<212>  RNA
<213>  Artificial Sequence

<220>
<223>  nucleic acid molecule

<400>  1
UUUUUUUUUU UUUUUUUUUU T                                              21

<210>  2
<211>  50
<212>  RNA
<213>  Artificial Sequence

<220>
<223>  nucleic acid molecule

<400>  2
UUUUUUUUUU UUUUUUUUUU UUUUUUUUUU UUUUUUUUUU UUUUUUUUUT   50

<210>  3
<211>  21

<212>  RNA
<213>  Artificial Sequence

<220>
<223>  nucleic acid molecule

<400>  3
GUGUGUGUGU GUGUGUGUGU T                                              21

## Description

### [Technical Field]

The present invention relates to a glycoside compound and a production method thereof.

### [Background Art]

As a production (synthesis) method of nucleic acids such as DNA, RNA and the like, for example, a phosphoramidite method and the like are used. As a starting material for the nucleic acid synthesis by the phosphoramidite method, phosphoramidite of nucleoside (hereinafter to be simply referred to as "phosphoramidite") is used. Examples of the protecting group at the 2'-position of the aforementioned phosphoramidite include many protecting groups such as TBDMS (tert-butyldimethylsilyl) group, TOM (triisopropylsilyloxymethyl) group, ACE (bis(2-acetoxyethoxy)methyl) group and the like.

However, since the production cost of conventional phosphoramidites such as TOM amidite, ACE amidite and the like is high, they are not convenient as starting materials for the synthesis of pharmaceutical products and the like. In addition, the yield and purity of nucleic acid are sometimes not very high when nucleic acid is synthesized by a coupling (condensation) reaction using TBDMS amidite.

Thus, the development of a protecting group capable of providing a phosphoramidite which can be produced at a low cost and can produce a nucleic acid in a high yield with high purity has been tried (patent documents 1 and 2).

### [Document List]

### [Patent documents]

patent document 1: WO2013/027843
patent document 2: WO2008/090829

### [SUMMARY OF THE INVENTION]

### [Problems to be Solved by the Invention]

The present invention aims to provide a method of producing, more efficiently at a high purity, a phosphoramidite preferable for the production (synthesis) of a nucleic acid.

### [Means of Solving the Problems]

The present inventors have conducted intensive studies in an attempt to solve the aforementioned problems and found that phosphoramidite can be produced more efficiently at a high purity as compared to conventional methods by using a 3,4-diacetoxybutoxymethyl (hereinafter to be referred to as DBM) group as the 2'-position protecting group of phosphoramidite, which resulted in the completion of the present invention.

Accordingly, the present invention provides the following.
[1] A glycoside compound represented by the following chemical formula (1),
   an enantiomer, tautomer or stereoisomer thereof or a salt thereof: 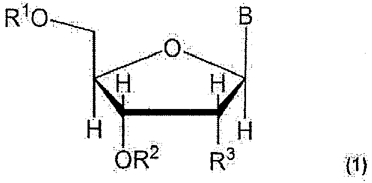 in the aforementioned chemical formula (1),
   B is an atomic group having a nucleic acid base skeleton, and optionally having a protecting group,
   R¹ and R² are each a hydrogen atom or a protecting group,
   or R¹ and R² in conjunction optionally form an atomic group represented by the following chemical formula (R¹R²A) or (R¹R²B) : 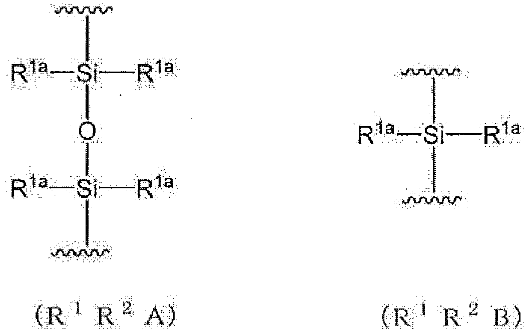
      each R^{1a} is a hydrogen atom, a straight chain or branched alkyl group, or a straight chain or branched alkoxy group, which may be the same or different,
      R³ is a group represented by the following chemical formula (R³) : 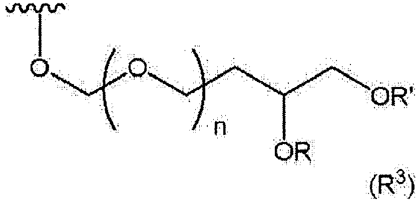 in the aforementioned chemical formula (R³),
         n is a positive integer,
         R and R' are the same or different and each is a hydrogen atom or a hydroxyl-protecting group.
[2] The glycoside compound of the above-mentioned [1], wherein R and R' are the same or different and each is an acyl group, an enantiomer, tautomer or stereoisomer thereof or a salt thereof.
[3] The glycoside compound of the above-mentioned [1], wherein R and R' are acetyl groups,
   an enantiomer, tautomer or stereoisomer thereof or a salt thereof.
[4] The glycoside compound of any of the above-mentioned [1] to [3], wherein, in the aforementioned chemical formula (1),
   R¹ is a hydrogen atom, or a substituent represented by any of the following chemical formulas (R¹A), (R¹B), (R¹C) and (R¹D),
   an enantiomer, tautomer or stereoisomer thereof or a salt thereof: 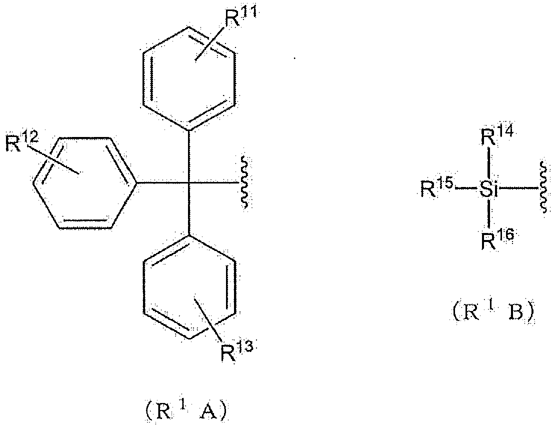 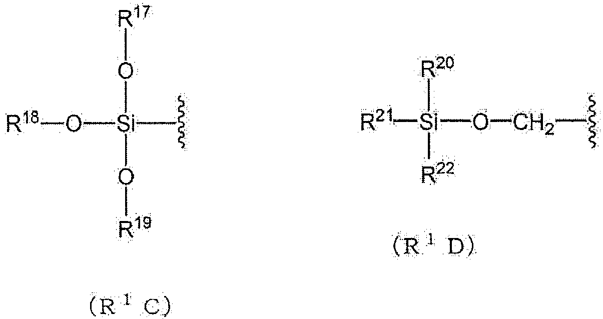 in the aforementioned chemical formula (R¹A),
   R¹¹ - R¹³ may be the same or different and each is a straight chain or branched alkoxy group, or a straight chain or branched alkyl group, or absent,
   R¹¹ - R¹³ are, when they are present, respectively present singly or in plurality, and when present in plurality, they may be the same or different,
   in the aforementioned chemical formula (R¹B),
   R¹⁴ - R¹⁶ may be the same or different and each is a hydrogen atom, a straight chain or branched alkyl group, or a straight chain or branched alkoxy group,
   in the aforementioned chemical formula (R¹C),
   R¹⁷ - R¹⁹ are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which may be the same or different,
   in the aforementioned chemical formula (R¹D),
   R²⁰ - R²² may be the same or different and each is a hydrogen atom, or a straight chain or branched alkyl group.
[5] The glycoside compound of any of the above-mentioned [1] to [3], wherein, in the aforementioned chemical formula (1),
   R¹ is a hydrogen atom, or a substituent represented by the following chemical formula (R¹Aa), (R¹Ba), (R¹Ca), (R¹Cb) or (R¹Da),
   an enantiomer, tautomer or stereoisomer thereof or a salt thereof: 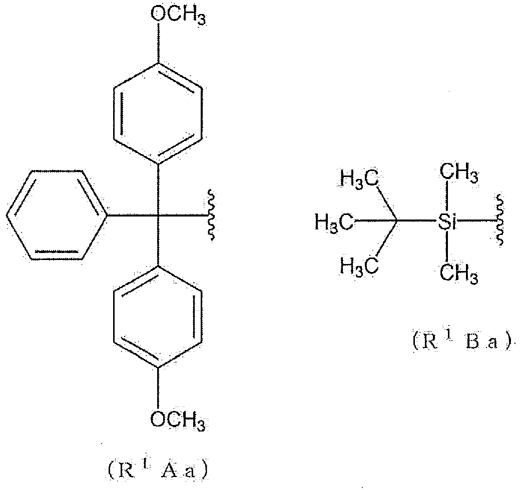 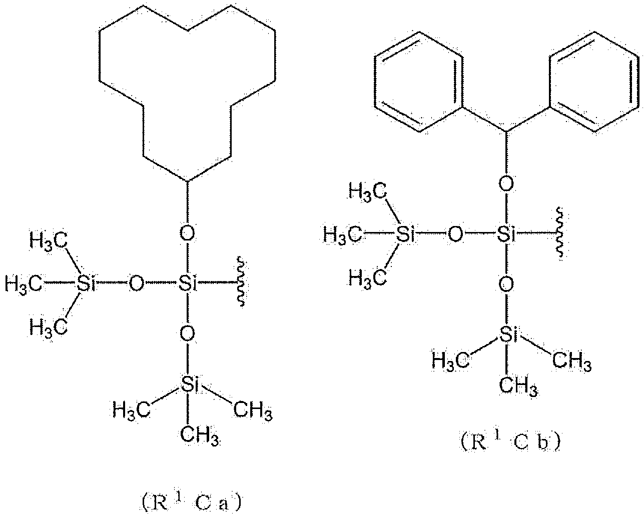 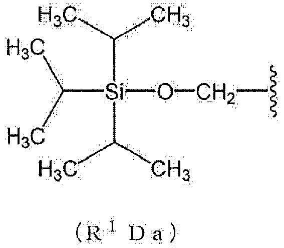
[6] The glycoside compound of any of the above-mentioned [1] to [3], wherein, in the aforementioned chemical formula (1),
   R¹ and R² in conjunction optionally form an atomic group represented by the aforementioned chemical formula (R¹R²A) or (R¹R²B) :
   in the aforementioned chemical formula (R¹R²A) and (R¹R²B),
   each R^{1a} is a hydrogen atom, a straight chain or branched alkyl group, or a straight chain or branched alkoxy group, which may be the same or different,
   an enantiomer, tautomer or stereoisomer thereof or a salt thereof.
[7] The glycoside compound of any of the above-mentioned [1] to [5], wherein the glycoside compound represented by the aforementioned chemical formula (1) is a glycoside compound represented by the following chemical formula (2),
   an enantiomer, tautomer or stereoisomer thereof or a salt thereof: 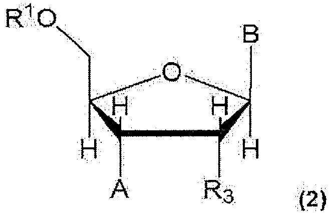 in the aforementioned chemical formula (2),
   B, R¹ and R³ are the same as those in the aforementioned chemical formula (1),
   R¹ is a protecting group,
   A is a group represented by the following chemical formula (2-1), (2-2-1), (2-2-2) or (2-2-3): 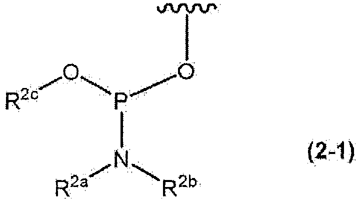 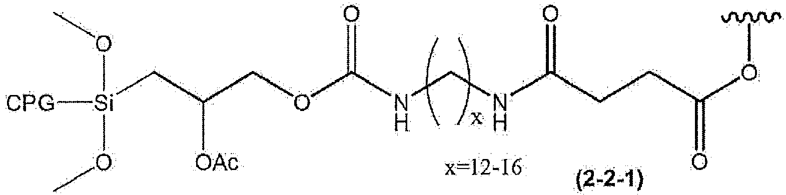 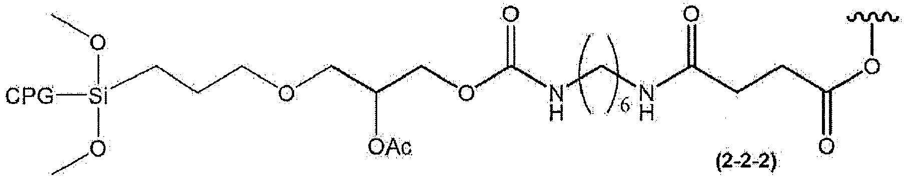 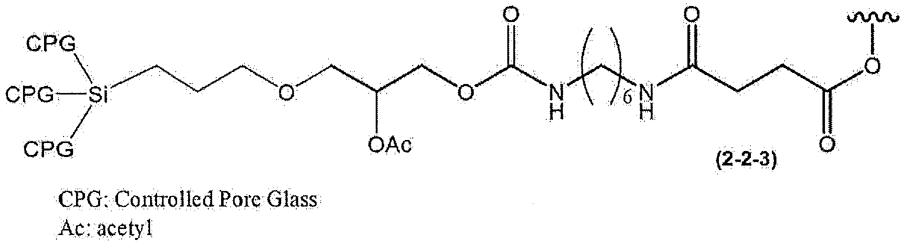
      R^{2a} and R^{2b} may be the same or different and each is a hydrogen atom or any substituent,
      alternatively, R^{2a} and R^{2b} may form, in conjunction with the nitrogen atom bonded thereto, a non-aromatic ring, wherein the aforementioned non-aromatic ring may or may not have a nitrogen atom, an oxygen atom or a sulfur atom besides the aforementioned nitrogen atom, and may or may not further have a substituent, and
      R^{2c} is a hydrogen atom, an electron-withdrawing group or any substituent, which may be optionally substituted by an electron-withdrawing group [D²].
[8] The glycoside compound of the above-mentioned [7], wherein, in the aforementioned chemical formula (2-1),
   R^{2a} and R^{2b} are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which is optionally further substituted or not substituted by an electron-withdrawing group,
   alternatively, R^{2a} and R^{2b} may form, in conjunction with the nitrogen atom bonded thereto, a 5- or 6-membered non-aromatic ring, wherein the aforementioned non-aromatic ring may or may not have a nitrogen atom, an oxygen atom or a sulfur atom besides the aforementioned nitrogen atom, and may or may not further have a substituent,
   an enantiomer, tautomer or stereoisomer thereof or a salt thereof.
[9] The glycoside compound of the above-mentioned [7], wherein, in the aforementioned chemical formula (2-1),
   R^{2a} and R^{2b} are each a methyl group, an ethyl group, an isopropyl group, or a t-butyl group, or
   R^{2a} and R^{2b} form, in conjunction with a nitrogen atom bonded thereto, a piperidyl group, a morpholino group, a pyrrolidyl group, a thiomorpholino group, or other nitrogen-containing alicyclic group,
   an enantiomer, tautomer or stereoisomer thereof or a salt thereof.
[10] The glycoside compound of any of the above-mentioned [7] to [9], wherein,
   in the aforementioned chemical formula (2-1),
   R^{2c} is a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, and further, may or may not be substituted by an electron-withdrawing group [D²], an enantiomer, tautomer or stereoisomer thereof or a salt thereof.
[11] The glycoside compound of any of the above-mentioned [7] to [9], wherein, in the aforementioned chemical formula (2-1),
   R^{2c} is a straight chain or branched alkyl group substituted by an electron-withdrawing group [D²], an enantiomer, tautomer or stereoisomer thereof or a salt thereof.
[12] The glycoside compound of any of the above-mentioned [7] to [11], wherein, in the aforementioned chemical formula (2-1),
   the aforementioned electron-withdrawing group [D²] for R^{2c} is a cyano group, a nitro group, an alkylsulfonyl group, halogen, an arylsulfonyl group, or a trihalomethyl group, an enantiomer, tautomer or stereoisomer thereof or a salt thereof.
[13] The glycoside compound of any of the above-mentioned [7] to [11], wherein,
   in the aforementioned chemical formula (2-1),
   R^{2c} is an alkenyl group or an ethynyl group, or substituted by an electron-withdrawing group [D²] and form, together with [D²], a cyanoethyl group,
   an enantiomer, tautomer or stereoisomer thereof or a salt thereof.
[14] The glycoside compound of any of the above-mentioned [1] to [5] and [7] to [13], wherein,
   the glycoside compound represented by the aforementioned chemical formula (1) is a glycoside compound represented by the following chemical formula (3),
   an enantiomer, tautomer or stereoisomer thereof or a salt thereof: 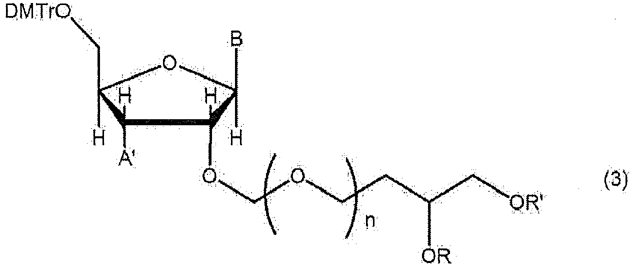 in the aforementioned chemical formula (3),
   R, R', B and n are as defined for the aforementioned chemical formula (1), and
   DMTr is a 4,4'-dimethoxy(triphenylmethyl) group, and
   A' is a group represented by the following chemical formula (3-1), (3-2-1) or (3-2-2): 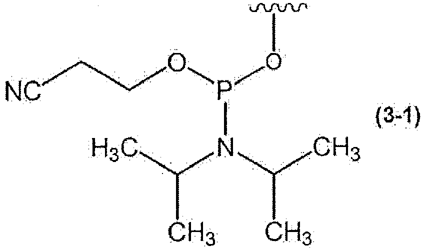 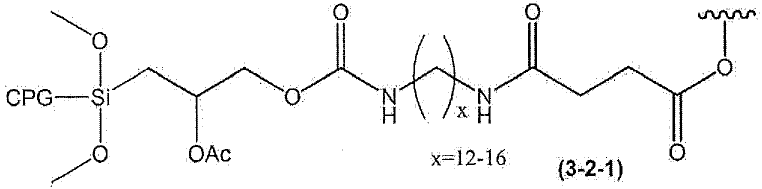 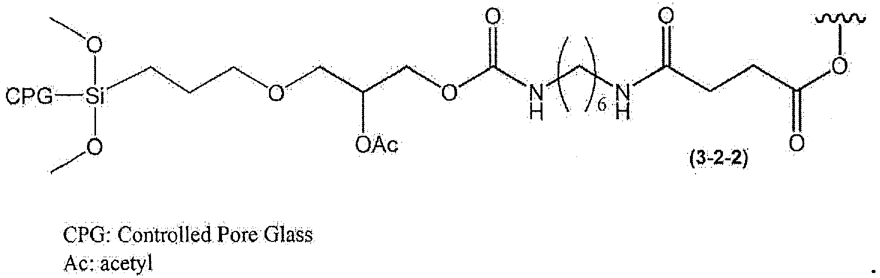
[15] The glycoside compound of the above-mentioned [1], wherein the glycoside compound represented by the aforementioned chemical formula (1) is a glycoside compound represented by the following chemical formula (AA5), (AB5), (C5), (G5) or (U5),
   an enantiomer, tautomer or stereoisomer thereof or a salt thereof: 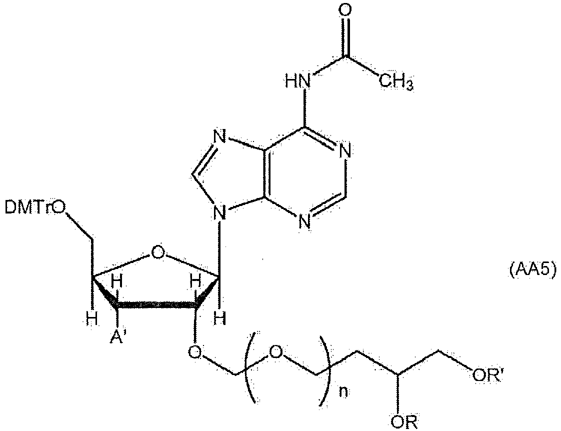 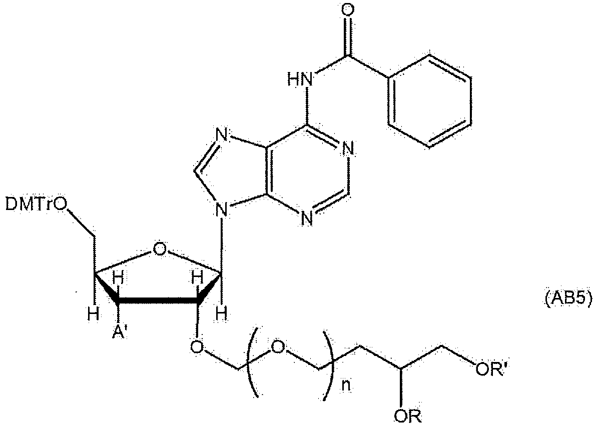 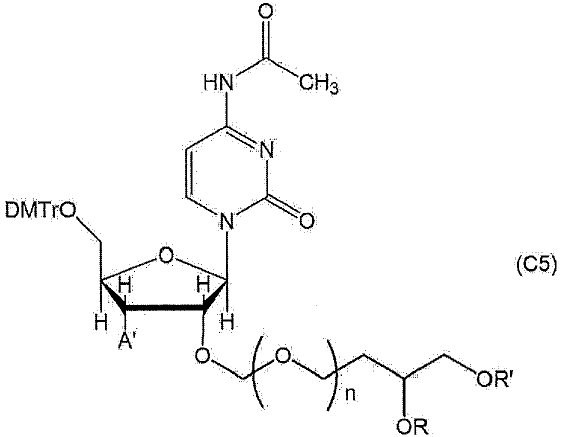 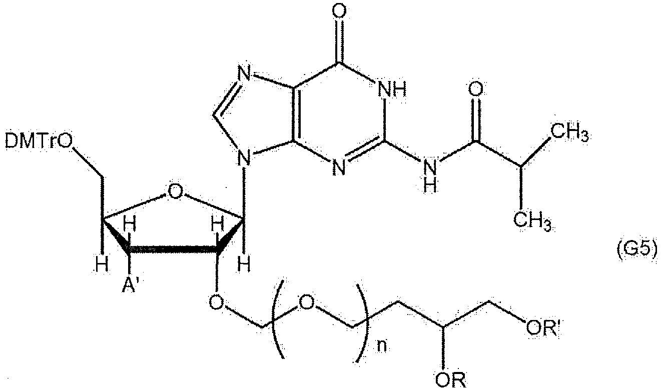 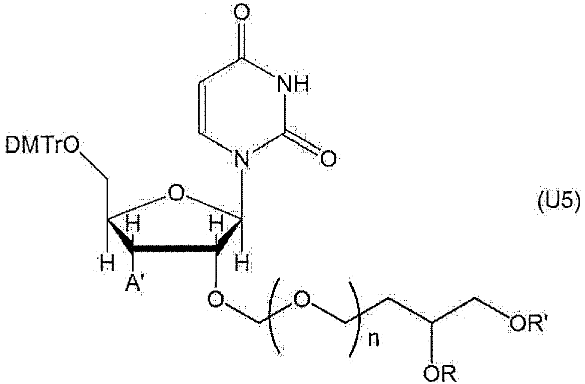 in the aforementioned chemical formulas (AA5), (AB5), (C5), (G5) and (U5), R, R' and n are as defined for the aforementioned chemical formula (1), DMTr is a 4,4'-dimethoxy(triphenylmethyl) group, A' is a group represented by the following chemical formula (3-1), (3-2-1) or (3-2-2): 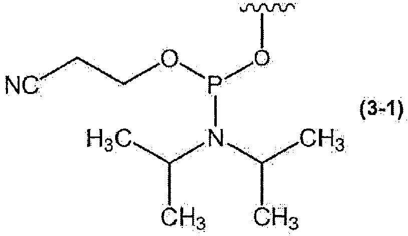 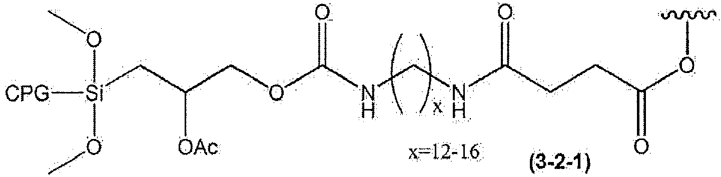 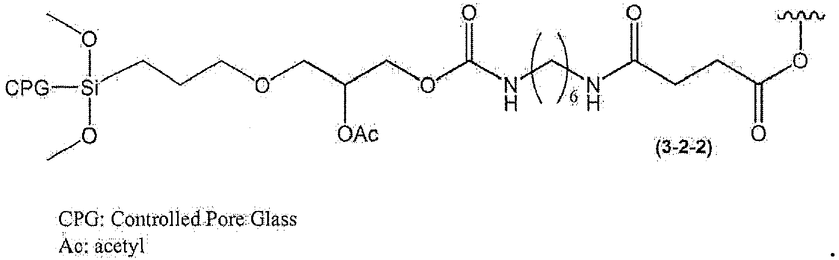
[16] The glycoside compound of any of the above-mentioned [1] to [15], wherein,
   in the aforementioned chemical formula (1), n=1, an enantiomer, tautomer or stereoisomer thereof or a salt thereof.
[17] A method for producing an ether represented by the following chemical formula (105), comprising
   (1) methylthiomethylating a compound represented by the following chemical formula (102) to produce a compound represented by the following chemical formula (103);
   (2) subjecting the compound represented by the following chemical formula (103) to a ring opening reaction to produce a compound represented by the following chemical formula (104); and
   (3) protecting a hydroxyl group of the compound represented by the following chemical formula (104), and repeating, where necessary, a coupling reaction with methylthiomethanol in the presence of a halogenating agent and a Lewis acid: 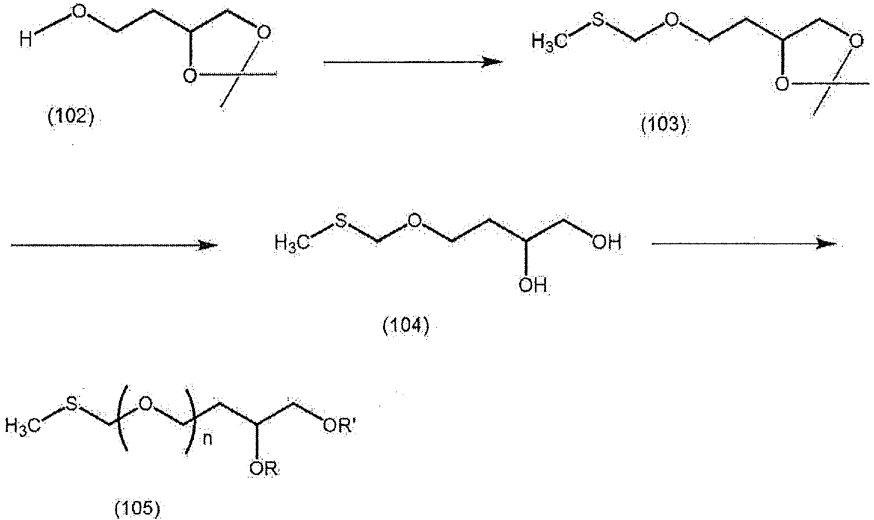 in each formula,
      n is a positive integer, and
      R and R' are the same or different and each is a hydrogen atom or a hydroxyl-protecting group.
[18] The method of the above-mentioned [17], wherein the alcohol represented by the chemical formula (102) is obtained by protecting a diol represented by the following chemical formula (101) by cyclization: 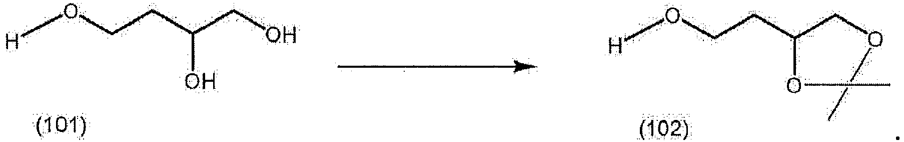
[19] A method for producing a compound represented by the following chemical formula (102), comprising
   (1) alkaline hydrolyzing a compound represented by the following chemical formula (101-1) to produce an alcohol represented by the following chemical formula (101-2);
   (2) producing a compound represented by the following chemical formula (101-3) by protecting a hydroxyl group of the alcohol represented by the chemical formula (101-2);
   (3) oxidizing the compound represented by the following chemical formula (101-3) to produce a compound represented by the following chemical formula (101-4);
   (4) isopropylidenating the compound represented by the following chemical formula (101-4) to produce a compound represented by the following chemical formula (101-5); and
   (5) deprotecting the compound represented by the following chemical formula (101-5): 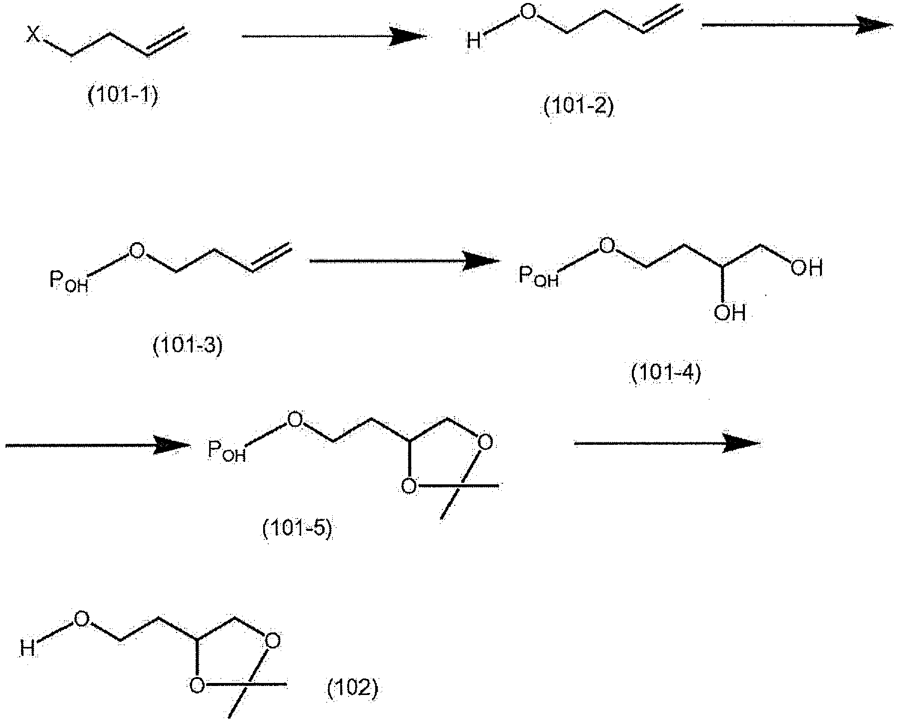 in each formula,
      P_{OH} is a hydroxyl-protecting group, and
      X is a halogen atom, an acetyloxy group, an optionally substituted carboxyl group or an optionally substituted carbamoyl group.
[20] A method for producing ether represented by the following chemical formula (105), comprising
   (1) protecting the 4-position hydroxyl group of a compound represented by the following chemical formula (104-1) to produce hydroxycarboxylic acid represented by the following chemical formula (104-2);
   (2) protecting a hydroxyl group of the hydroxycarboxylic acid represented by the following chemical formula (104-2), and acylating same to produce a compound represented by the following chemical formula (104-3);
   (3) deprotecting the 4-position of the compound represented by the following chemical formula (104-3) to produce a compound represented by the following chemical formula (104-4); and
   (4) methylthiomethyling the compound represented by the following chemical formula (104-4), and repeating, where necessary, a coupling reaction with methylthiomethanol in the presence of a halogenating agent and a Lewis acid: 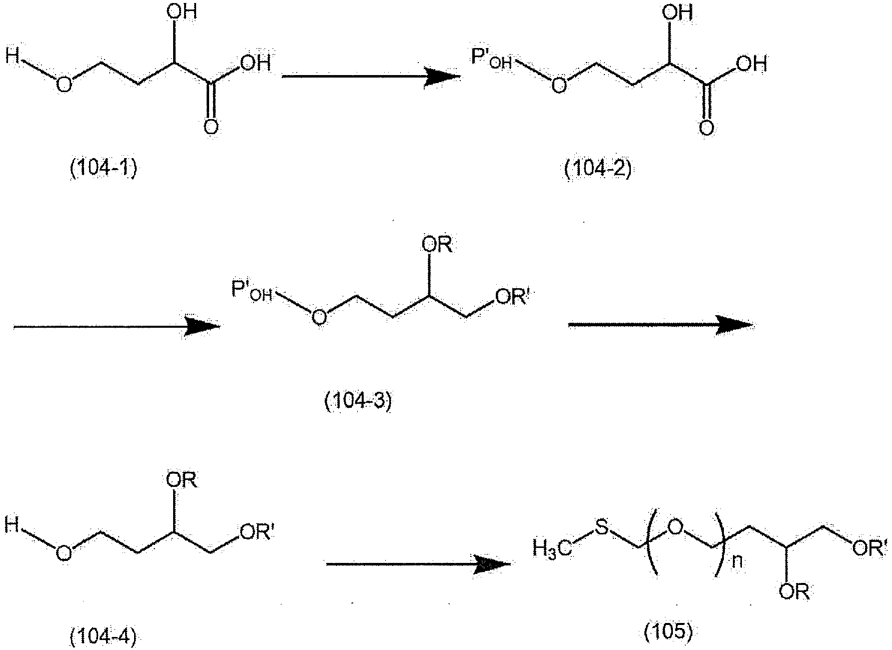
   in each formula,
   n is a positive integer,
   P'_{OH} is a hydroxyl-protecting group, and
   R and R' are the same or different and each is a hydrogen atom or a hydroxyl-protecting group.
[21] The production method of the above-mentioned [17] or [20], wherein R and R' are acetyl groups.
[22] Ether represented by the following chemical formula (105), an enantiomer, tautomer or stereoisomer thereof or a salt thereof: 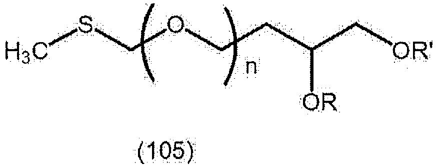 wherein,
   n is a positive integer, and
   R and R' are the same or different and each is a hydrogen atom or a hydroxyl-protecting group.
[23] A production method of the glycoside compound of any of the above-mentioned [1] to [16], an enantiomer, tautomer or stereoisomer thereof or a salt thereof, comprising a coupling step for producing a glycoside compound represented by the following chemical formula (1a) by subjecting a glycoside compound represented by the following chemical formula (106) and ether represented by the following chemical formula (105) to a coupling reaction in the presence of a halogenating agent and a Lewis acid, wherein
   the glycoside compound represented by the following chemical formula (1a) is the glycoside compound wherein R¹ and R² in the aforementioned chemical formula (1) in conjunction form an atomic group represented by the aforementioned chemical formula (R¹R²A) or R¹R²B : 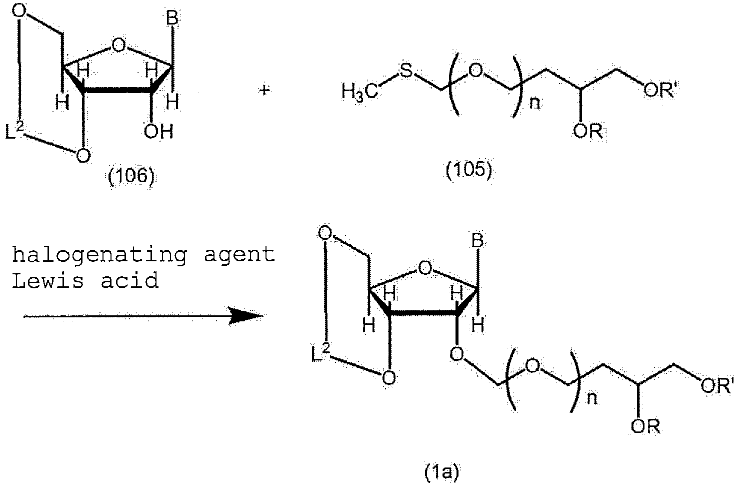 in the aforementioned chemical formulas (106) and (1a),
   L² is an atomic group represented by the aforementioned chemical formula (R¹R²A) or (R¹R²B),
   in the aforementioned chemical formulas (105), (106) and (1a),
   B, n, R and R' are as defined for the aforementioned chemical formula (1).
[24] The production method of the above-mentioned [23], further comprising a deprotection step for producing a glycoside compound represented by the following chemical formula (1b) by removing the aforementioned atomic group L² from the glycoside compound represented by the aforementioned chemical formula (1a), wherein
   the glycoside compound represented by the following chemical formula (1b) is a glycoside compound of the aforementioned chemical formula (1) wherein R¹ and R² are hydrogen atoms: 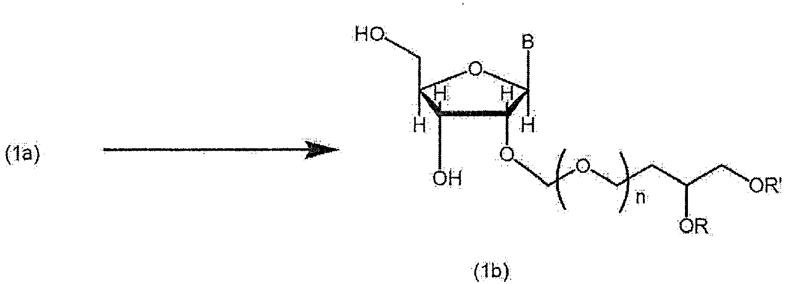 in the aforementioned chemical formula (1b),
   B, n, R and R' are as defined for the aforementioned chemical formula (1).
[25] The production method of the above-mentioned [24], further comprising a protecting group introduction step for producing a glycoside compound represented by the following chemical formula (1c) by introducing protecting groups R¹ and R² into the aforementioned chemical formula (1b), wherein
   the glycoside compound represented by the following chemical formula (1c) is a glycoside compound of the aforementioned chemical formula (1) wherein R¹ and R² are other than a hydrogen atom and the aforementioned chemical formulas (R¹R²A) and (R¹R²B) : 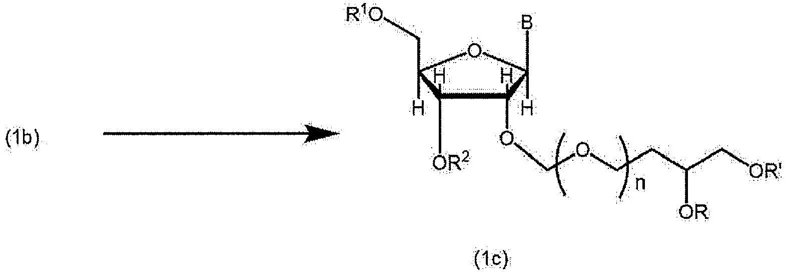 in the aforementioned chemical formula (1c),
   R¹ and R² are R¹ and R² in the aforementioned chemical formula (1) and other than a hydrogen atom and the aforementioned chemical formulas (R¹R²A) and (R¹R²B), and
   B, n, R and R' are as defined for the aforementioned chemical formula (1).
[26] The production method of any of the above-mentioned [23] to [25], wherein,
   the aforementioned halogenating agent is at least one selected from the group consisting of N-chlorosuccinimide, N-bromosuccinimide, N-iodosuccinimide, iodine, bromine and chlorine.
[27] The production method of any of the above-mentioned [23] to [26], wherein the aforementioned Lewis acid is at least one selected from the group consisting of perfluoroalkyl carboxylic acid, perfluoroalkyl sulfonic acid, alkyl sulfonic acid and a salt thereof.
[28] The production method of any of the above-mentioned [23] to [27], wherein the aforementioned Lewis acid is a trifluoromethanesulfonic acid or methanesulfonic acid.
[29] The production method of any of the above-mentioned [23] to [28], wherein the aforementioned coupling reaction is performed in the co-presence of a molecular sieve.
[30] A nucleic acid comprising a structure represented by the following chemical formula (I), an enantiomer, tautomer or stereoisomer thereof: 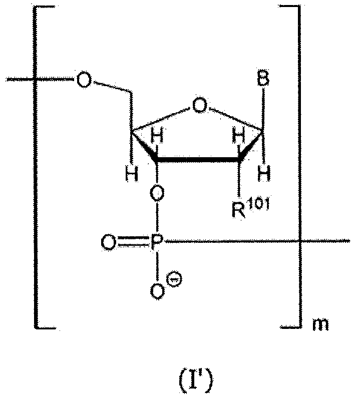 in the aforementioned chemical formula (I'), B is the same as the aforementioned chemical formula (1), (2) or (3),
   R¹⁰¹ is a group represented by the following chemical formula (R³) or (R⁴), 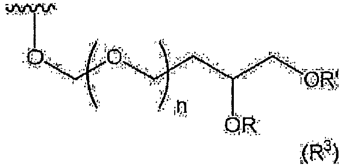 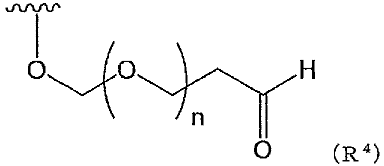 in the aforementioned chemical formula,
   n is a positive integer,
   R and R' are the same or different and each is a hydrogen atom or a hydroxyl-protecting group,
      each B may be the same or different and each R¹⁰¹ may be the same or different, and
      m is a positive integer.
[31] A method for producing a nucleic acid comprising a structure represented by the following chemical formula (I), comprising
   a condensation step for performing a condensation reaction of the glycoside compound of any of the above-mentioned [7] to [16]: 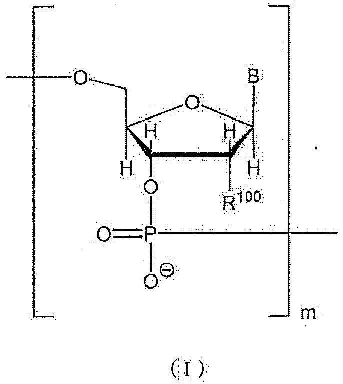 in the aforementioned chemical formula (I), B is the same as the aforementioned chemical formula (1), (2) or (3),
   R¹⁰⁰ is a hydrogen atom or a hydroxyl group, or a group represented by the following chemical formula (R³) or (R⁴): 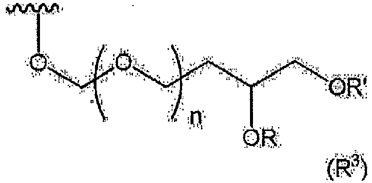 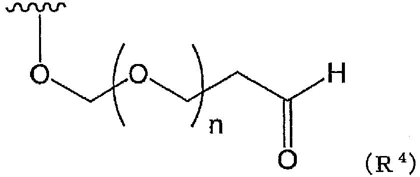 in the aforementioned chemical formula,
   n is a positive integer,
   R and R' are the same or different and each is a hydrogen atom or a hydroxyl-protecting group,
      each B may be the same or different, and each R¹⁰⁰ may be the same or different, and
      m is a positive integer.
[32] The method of the above-mentioned [31], comprising a step for deprotecting the 2'-position of the condensation compound obtained by the condensation reaction.
[33] The method of the above-mentioned [32], wherein the deprotection is performed by (i) an ammonia treatment step, (ii) a periodic acid treatment step, and (iii) a tetraalkylammonium halide treatment step.
[34] The method of the above-mentioned [33], comprising a step for protecting the 2'-position formyl group between (ii) the periodic acid treatment step and (iii) the tetraalkylammonium halide treatment step.
[35] The method of the above-mentioned [34], wherein an amine compound is reacted in the step for protecting the 2'-position formyl group.
[36] The method of the above-mentioned [35], wherein the amine compound is a benzylamine compound having an electron-withdrawing group.
[37] The method of any of the above-mentioned [31] to [36], wherein, in the aforementioned chemical formula (I), each R¹⁰⁰ is a hydroxyl group.
[38] The method of any of the above-mentioned [31] to [36], wherein, in the aforementioned chemical formula (I), each R¹⁰⁰ is a hydrogen atom, and the method further comprises
   a reverse transcription step for producing a nucleic acid represented by the aforementioned chemical formula (I) by reverse transcription from the nucleic acid obtained the aforementioned condensation step.
[39] The method of claim 31, comprising a step for obtaining the nucleic acid comprising a structure represented by the aforementioned chemical formula (I) wherein each R¹⁰⁰ is a hydroxyl group, by treating a nucleic acid comprising a structure represented by the aforementioned chemical formula (I) wherein each R¹⁰⁰ is a group represented by the chemical formula (R⁴) with tetraalkylammoniumhalide.

### [Effect of the Invention]

According to the glycoside compound, ether, the production method of ether, and the production method of a glycoside compound, of the present invention, phosphoramidite that can be produced at a low cost and can produce a nucleic acid in a high yield and with high purity can be provided. Moreover, according to the production method of a nucleic acid in the present invention, a nucleic acid can be produced in a high yield and with high purity by using the aforementioned phosphoramidite.

### [Brief Description of the Drawings]

Fig. 1 is an HPLC chart of the nucleic acid (reaction mixture) produced in Example 7.
Fig. 2 is a mass spectrum of the nucleic acid (reaction mixture) produced in Example 7.
Fig. 3 is an HPLC chart of the nucleic acid (reaction mixture) produced in Example 8.
Fig. 4 is a mass spectrum of the nucleic acid (reaction mixture) produced in Example 8.
Fig. 5 is an HPLC chart of the nucleic acid (reaction mixture) produced in Example 10.
Fig. 6 is a mass spectrum of the nucleic acid (reaction mixture) produced in Example 10.

### [Description of Embodiments]

The present invention is explained in detail by way of Examples. However, the present invention is not limited by the following explanation.

Unless particularly specified, the terms used in the present specification can be used in the meanings generally adopted in the pertinent technical field.

The "atomic group having a nucleic acid base skeleton" means a functional group having a nucleic acid base skeleton in the whole or a part of the structure. The "nucleic acid base skeleton" here may be a natural nucleic acid base skeleton or an artificial nucleic acid base skeleton, and preferably a natural nucleic acid base skeleton.

The natural nucleic acid base is more preferably adenine, cytosine, guanine, uracil, thymine or other nitrogen-containing aromatic ring (e.g., 5-alkylpyrimidine, 5-halogenopyrimidine, deazapurine, deazapyrimidine, azapurine, azapyrimidine).

The "halogen" is, for example, a fluorine atom, a chlorine atom, a bromine atom or an iodine atom.

The "electron-withdrawing group" is a group which easily attracts an electron from the bonded atom side as compared to a hydrogen atom. Specifically, cyano, nitro, alkylsulfonyl (e.g., methylsulfonyl, ethylsulfonyl), halogen (fluorine atom, chlorine atom, bromine atom or iodine atom), arylsulfonyl (e.g., phenylsulfonyl, naphthylsulfonyl), trihalomethyl (e.g., trichloromethyl, trifluoromethyl), tetraalkylammonium (e.g., tetramethylammonium) and the like can be mentioned.

The "hydroxyl-protecting group" means a general hydroxyl-protecting group known to those of ordinary skill in the art, which is introduced to prevent a reaction of the hydroxyl group. For example, the protecting groups described in Protective Groups in Organic Synthesis, published by John Wiley and Sons (1980) and the like, specifically, acyl-protecting groups such as acetyl, benzoyl and the like, alkyl-protecting groups such as trityl, 4-methoxytrityl, 4,4'-dimethoxytrityl, benzyl and the like, silyl-protecting group such as trimethylsilyl, triisopropylsilyl, tert-butyldimethylsilyl, tert-butyldiphenylsilyl and the like can be mentioned.

In the present invention, "alkyl" includes, for example, linear or branched alkyl. The carbon number of the aforementioned alkyl is not particularly limited and, for example, 1 - 30, preferably 1 - 12, 1 - 6 or 1 - 4. Examples of the aforementioned alkyl include methyl, ethyl, n-propyl, isopropyl, n-butyl, isobutyl, sec-butyl and tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl and the like. Preferably, for example, methyl, ethyl, n-propyl, isopropyl, n-butyl, isobutyl, sec-butyl, tert-butyl, n-pentyl, isopentyl, neopentyl, n-hexyl, isohexyl and the like can be mentioned. The same applies to a group containing an alkyl group in the structure (alkylamino group, alkoxy group etc.), and a group induced from an alkyl group (haloalkyl group, hydroxyalkyl group, aminoalkyl group, alkanoyl group etc.).

In the present invention, "alkenyl" includes, for example, linear or branched alkenyl. The aforementioned alkenyl is, for example, the aforementioned alkyl containing one or plural double bonds and the like. The carbon number of the aforementioned alkenyl is not particularly limited and, for example, the same as for the aforementioned alkyl and preferably 2 - 12 or 2 - 8. Examples of the aforementioned alkenyl include vinyl, 1-propenyl, 2-propenyl, 1-butenyl, 2-butenyl, 3-butenyl, 1,3-butadienyl, 3-methyl-2-butenyl and the like.

In the present invention, "alkynyl" includes, for example, linear or branched alkynyl. The aforementioned alkynyl is, for example, the aforementioned alkyl containing one or plural triple bonds and the like. The carbon number of the aforementioned alkynyl is not particularly limited and, for example, the same as for the aforementioned alkyl and preferably 2 - 12 or 2 - 8. Examples of the aforementioned alkynyl include ethynyl, propynyl, butynyl and the like. The aforementioned alkynyl may further have, for example, one or plural double bonds.

In the present invention, "aryl" includes, for example, a monocyclic aromatic hydrocarbon group and a polycyclic aromatic hydrocarbon group. Examples of the aforementioned monocyclic aromatic hydrocarbon group include phenyl and the like. Examples of the aforementioned polycyclic aromatic hydrocarbon group include 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl and the like. Preferably, for example, phenyl, naphthyl such as 1-naphthyl and 2-naphthyl and the like, and the like can be mentioned.

In the present invention, "heteroaryl" includes, for example, a monocyclic aromatic heterocyclic group and a fused aromatic heterocyclic group. Examples of the aforementioned heteroaryl include furyl (e.g., 2-furyl, 3-furyl), thienyl (e.g., 2-thienyl, 3-thienyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), triazolyl (e.g., 1,2,4-triazol-1-yl, 1,2,4-triazol-3-yl, 1,2,4-triazol-4-yl), tetrazolyl (e.g., 1-tetrazolyl, 2-tetrazolyl, 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), furazanyl (e.g., 3-furazanyl), pyrazinyl (e.g., 2-pyrazinyl), oxadiazolyl (e.g., 1,3,4-oxadiazol-2-yl), benzofuryl (e.g., 2-benzo[b]furyl, 3-benzo[b]furyl, 4-benzo[b]furyl, 5-benzo[b]furyl, 6-benzo[b]furyl, 7-benzo[b]furyl), benzothienyl (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyl (e.g., 1-benzimidazolyl, 2-benzimidazolyl, 4-benzimidazolyl, 5-benzimidazolyl), dibenzofuryl, benzoxazolyl, benzothiazolyl, quinoxalyl (e.g., 2-quinoxalinyl, 5-quinoxalinyl, 6-quinoxalinyl), cinnolinyl (e.g., 3-cinnolinyl, 4-cinnolinyl, 5-cinnolinyl, 6-cinnolinyl, 7-cinnolinyl, 8-cinnolinyl), quinazolyl (e.g., 2-quinazolinyl, 4-quinazolinyl, 5-quinazolinyl, 6-quinazolinyl, 7-quinazolinyl, 8-quinazolinyl), quinolyl (e.g., 2-quinolyl, 3-quinolyl, 4-quinolyl, 5-quinolyl, 6-quinolyl, 7-quinolyl, 8-quinolyl), phthalazinyl (e.g., 1-phthalazinyl, 5-phthalazinyl, 6-phthalazinyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), puryl, pteridinyl (e.g., 2-pteridinyl, 4-pteridinyl, 6-pteridinyl, 7-pteridinyl), carbazolyl, phenanthridinyl, acridinyl (e.g., 1-acridinyl, 2-acridinyl, 3-acridinyl, 4-acridinyl, 9-acridinyl), indolyl (e.g., 1-indolyl, 2-indolyl, 3-indolyl, 4-indolyl, 5-indolyl, 6-indolyl, 7-indolyl), isoindolyl, phenazinyl (e.g., 1-phenazinyl, 2-phenazinyl) or phenothiazinyl (e.g., 1-phenothiazinyl, 2-phenothiazinyl, 3-phenothiazinyl, 4-phenothiazinyl) and the like.

In the present invention, "cycloalkyl" is, for example, a cyclic saturated hydrocarbon group, and the carbon number is not particularly limited and is, for example, 3 - 24 or 3 - 15. Examples of the aforementioned cycloalkyl include cyclopropyl, cyclobutyl, cyclopentyl, cyclohexyl, cycloheptyl, cyclooctyl, bridged cyclic hydrocarbon group, spirohydrocarbon group and the like, preferably, cyclopropyl, cyclobutyl, cyclopentyl, cyclohexyl, bridged cyclic hydrocarbon group and the like.

In the present invention, the "bridged cyclic hydrocarbon group" is, for example, bicyclo[2.1.0]pentyl, bicyclo[2.2.1]heptyl, bicyclo[2.2.2]octyl and bicyclo[3.2.1]octyl, tricyclo[2.2.1.0]heptyl, bicyclo[3.3.1]nonane, 1-adamantyl, 2-adamantyl or the like.

In the present invention, the "spirohydrocarbon group" is, for example, spiro[3.4]octyl or the like.

In the present invention, "cycloalkenyl" includes, for example, a cyclic unsaturated aliphatic hydrocarbon group, and the carbon number is, for example, 3 - 24 or 3 - 7. Examples of the aforementioned group include cyclopropenyl, cyclobutenyl, cyclopentenyl, cyclohexenyl, cycloheptenyl and the like, preferably, cyclopropenyl, cyclobutenyl, cyclopentenyl, cyclohexenyl and the like. The aforementioned cycloalkenyl includes, for example, a bridged cyclic hydrocarbon group and a spirohydrocarbon group having an unsaturated bond in the ring.

In the present invention, "arylalkyl" is, for example, benzyl, 2-phenethyl, naphthalenylmethyl or the like, "cycloalkylalkyl" or "cyclylalkyl" is, for example, cyclohexylmethyl, adamantylmethyl or the like, and "hydroxyalkyl" is, for example, hydroxymethyl and 2-hydroxyethyl or the like.

In the present invention, "alkoxy" includes, for example, the aforementioned alkyl-O- group and, for example, methoxy, ethoxy, n-propoxy, isopropoxy, n-butoxy and the like can be mentioned, and "alkoxyalkyl" is, for example, methoxymethyl or the like, and "aminoalkyl" is, for example, 2-aminoethyl or the like.

In the present invention, "cyclyl" is any cyclic atomic group, and is preferably a non-aromatic saturated or unsaturated cyclic substituent. The carbon number thereof is not particularly limited and is, for example, 3 - 24.

In the present invention, "heterocyclyl" is, for example, 1-pyrrolinyl, 2-pyrrolinyl, 3-pyrrolinyl, 1-pyrrolidinyl, 2-pyrrolidinyl, 3-pyrrolidinyl, pyrrolidinone, 1-imidazolinyl, 2-imidazolinyl, 4-imidazolinyl, 1-imidazolidinyl, 2-imidazolidinyl, 4-imidazolidinyl, imidazolidinone, 1-pyrazolinyl, 3-pyrazolinyl, 4-pyrazolinyl, 1-pyrazolidinyl, 3-pyrazolidinyl, 4-pyrazolidinyl, piperidinone, piperidino, 2-piperidinyl, 3-piperidinyl, 4-piperidinyl, 1-piperazinyl, 2-piperazinyl, piperazinone, 2-morpholinyl, 3-morpholinyl, morpholino, tetrahydropyranyl, tetrahydrofuranyl or the like.

In the present invention, "heterocyclylalkyl" includes, for example, piperidinylmethyl, piperazinylmethyl and the like, "heterocyclylalkenyl" includes, for example, 2-piperidinylethenyl and the like, and "heteroarylalkyl" includes, for example, pyridylmethyl, quinolin-3-ylmethyl and the like.

In the present invention, "silyl" includes, a group represented by the formula R₃Si-, wherein R is, independently, selected from the aforementioned alkyl, aryl and cycloalkyl and, for example, a trimethylsilyl group, a triisopropylsilyl group, a tert-butyldimethylsilyl group and the like can be mentioned. The "silyloxy" is, for example, a trimethylsilyloxy group and the like, and "silyloxyalkyl", for example, trimethylsilyloxymethyl or the like.

In the present invention, "acyl" is not particularly limited and, for example, formyl, acetyl, propionyl, isobutyryl, valeryl, isovaleryl, pivaloyl, hexanoyl, cyclohexanoyl, benzoyl, ethoxycarbonyl, and the like can be mentioned. The same applies to a group containing an acyl group in the structure (acyloxy group, alkanoyloxy group etc.). In the present invention, moreover, the carbon number of the acyl group contains carbonyl carbon and, for example, an alkanoyl group (acyl group) having a carbon number 1 means a formyl group.

In the present invention, "perfluoroalkyl" is not particularly limited and, for example, a perfluoroalkyl group induced from a straight chain or branched alkyl group having 1 - 30 carbon atoms can be mentioned. The aforementioned "perfluoroalkyl" is more specifically, for example, a perfluoroalkyl group induced from a group such as methyl, ethyl, n-propyl, isopropyl, n-butyl, isobutyl, sec-butyl and tert-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl and the like. The same applies to a group containing a perfluoroalkyl group in the structure (perfluoroalkylsulfonyl group, perfluoroacyl group etc.).

In the present invention, the aforementioned various groups are optionally substituted. Examples of the aforementioned substituent include hydroxy, carboxy, halogen, alkyl halide (e.g., CF₃, CH₂CF₃, CH₂CCl₃), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g., morpholylmethyl), alkoxy (e.g., methoxy, ethoxy, propoxy, butoxy), halogenated alkoxy (e.g., OCF₃), alkenyloxy (e.g., vinyloxy, allyloxy), aryloxy (e.g., phenyloxy), alkyloxycarbonyl (e.g., methoxycarbonyl, ethoxycarbonyl, tert-butoxycarbonyl), arylalkyloxy (e.g., benzyloxy), amino[alkylamino (e.g., methylamino, ethylamino, dimethylamino), acylamino (e.g., acetylamino, benzoylamino), arylalkylamino (e.g., benzylamino, tritylamino), hydroxyamino], alkylaminoalkyl (e.g., diethylaminomethyl), sulfamoyl, oxo and the like.

In the present invention, when the aforementioned various groups are heterocycles or contain a heterocycle, the "carbon number" also includes the number of hetero atoms constituting the aforementioned heterocycle.

In the present invention, examples of the "substituent" include
(1) halogen (fluorine atom, chlorine atom, bromine atom or iodine atom);
(2) alkyl group (mentioned above);
(3) alkenyl group (mentioned above);
(4) alkynyl group (mentioned above);
(5) haloalkyl group (e.g., chloromethyl, fluoromethyl, dichloromethyl, difluoromethyl, dichlorofluoromethyl, trifluoromethyl, pentafluoroethyl etc.);
(6) aryl group (mentioned above);
(7) heteroaryl group (mentioned above);
(8) aralkyl group (mentioned above);
(9) cycloalkyl group (mentioned above);
(10) cycloalkenyl group (mentioned above);
(11) cycloalkylalkyl group (mentioned above);
(12) cycloalkenylalkyl group (e.g., cyclopentenylethyl, cyclohexenylethyl, cyclohexenylbutyl etc.);
(13) hydroxyalkyl group (e.g., hydroxymethyl, hydroxyethyl, hydroxypropyl, hydroxybutyl etc.);
(14) alkoxyalkyl group (mentioned above);
(15) aminoalkyl group (e.g., aminomethyl, aminoethyl, aminopropyl etc.);
(16) heterocyclyl group (e.g., 1-pyrrolinyl, 2-pyrrolinyl, 3-pyrrolinyl, 1-pyrrolidinyl, 2-pyrrolidinyl, 3-pyrrolidinyl, pyrrolidinone, 1-imidazolinyl, 2-imidazolinyl, 4-imidazolinyl, 1-imidazolidinyl, 2-imidazolidinyl, 4-imidazolidinyl, imidazolidinone, 1-pyrazolinyl, 3-pyrazolinyl, 4-pyrazolinyl, 1-pyrazolidinyl, 3-pyrazolidinyl, 4-pyrazolidinyl, piperidinone, piperidino, 2-piperidinyl, 3-piperidinyl, 4-piperidinyl, 1-piperazinyl, 2-piperazinyl, piperazinone, 2-morpholinyl, 3-morpholinyl, morpholino, tetrahydropyranyl, tetrahydrofuranyl or the like);
(17) heterocyclylalkenyl group (e.g., 2-piperidinylethenyl etc.);
(18) heterocyclylalkyl group (e.g., piperidinylmethyl, piperazinylmethyl etc.);
(19) heteroarylalkyl group (e.g., pyridylmethyl, quinolin-3-ylmethyl etc.);
(20) silyl group;
(21) silyloxyalkyl group (e.g., silyloxymethyl, silyloxyethyl etc.);
(22) mono-, di- or tri-alkylsilyl group (e.g., methylsilyl, ethylsilyl etc.);
(23) mono-, di- or tri-alkylsilyloxyalkyl group (e.g., trimethylsilyloxymethyl etc.)
and the like. Each of these substituents may or may not be further substituted by an electron-withdrawing group (mentioned above).

According to the present invention, for example, one or more effects from the following [1] - [4] can be obtained. However, these effects are exemplary and do not limit the present invention.
[1] The production method of a nucleic acid in the present invention can produce a nucleic acid with high purity and in a high yield by using the glycoside compound represented by the aforementioned chemical formula (2) in the present invention. Specifically, for example, it is possible to produce a nucleic acid by using inexpensive nucleic acid synthesis reagents and strong deprotection conditions similar to those used for DNA oligo synthesis.
[2] Inexpensive starting materials/reagents can be used for the ether and glycoside compound of the present invention, and they can be produced at a lower cost than conventional ACE amidite, TOM amidite and the like.
[3] According to the method of the present invention, the intermediate oligomer is stable and handling is easy.
[4] According to the method of the present invention, since the target nucleic acid can be produced at low cost and high yield, industrialization is easy.

### 1. Glycoside compound

The glycoside compound of the present invention is, as mentioned above,
a glycoside compound represented by the following chemical formula (1) (hereinafter to be also simply referred to as glycoside compound (1)), an enantiomer, tautomer or stereoisomer thereof or a salt thereof: 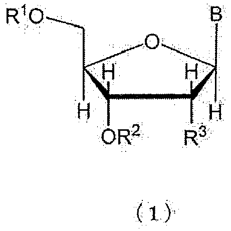 in the aforementioned chemical formula (1),
B is an atomic group having a nucleic acid base skeleton, and optionally having a protecting group,
R¹ and R² are each a hydrogen atom or a protecting group,
or R¹ and R² in conjunction optionally form an atomic group represented by the following chemical formula (R¹R²A) or (R¹R²B) : 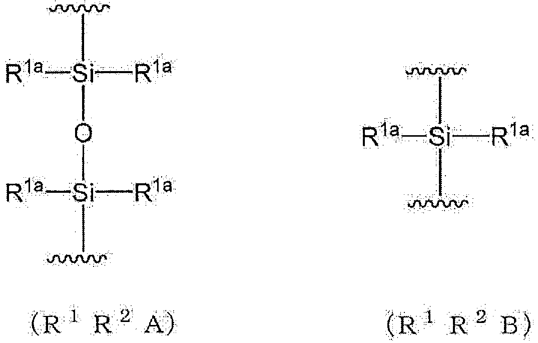
   each R^{1a} is a hydrogen atom, a straight chain or branched alkyl group, or a straight chain or branched alkoxy group, which may be the same or different,
   R³ is a group represented by the following chemical formula (R³) : 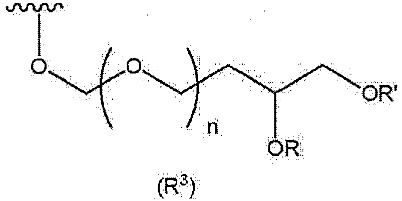 in the aforementioned chemical formula (R³),
      n is a positive integer, and
      R and R' are the same or different and each is a hydrogen atom or a hydroxyl-protecting group.

In the aforementioned chemical formula (R³), the hydroxyl-protecting group is, for example, an acyl-protecting group such as acetyl, benzoyl and the like, an alkyl-protecting group such as trityl, 4-methoxytrityl, 4,4'-dimethoxytrityl, benzyl and the like, or a silyl-protecting group such as trimethylsilyl, triisopropylsilyl, tert-butyldimethylsilyl, tert-butyldiphenylsilyl and the like, preferably an acyl group, more preferably an acetyl group. In the aforementioned chemical formula (R³), n is not particularly limited and, for example, it is within the range of 1 - 30, preferably 1 - 20, and n is particularly preferably 1.

In the aforementioned chemical formula (1), R¹ is, as mentioned above, a hydrogen atom or a protecting group. The protecting group R¹ is not particularly limited and is, for example, a substituent represented by any of the following chemical formulas (R¹A), (R¹B), (R¹C) and (R¹D) :

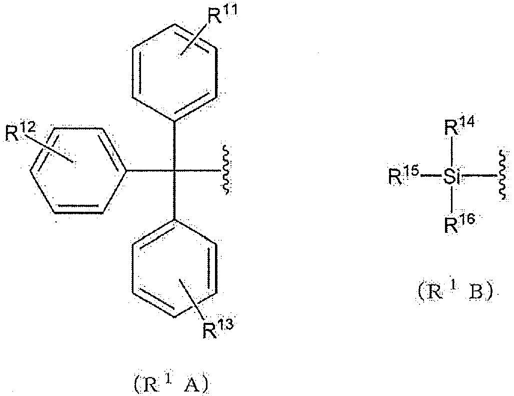

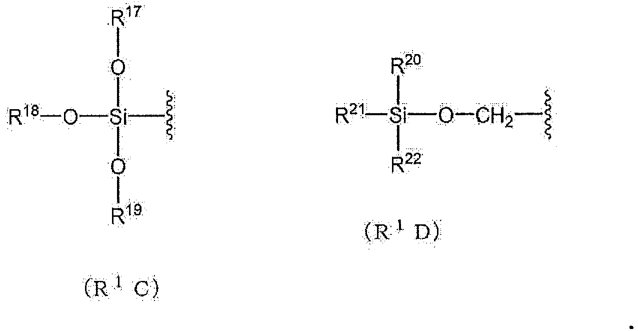

In the aforementioned chemical formula (R¹A),
R¹¹ - R¹³ may be the same or different and each is a straight chain or branched alkoxy group, or a straight chain or branched alkyl group, or absent,
R¹¹ - R¹³ are, when they are present, respectively present singly or in plurality, and when present in plurality, they may be the same or different,
in the aforementioned chemical formula (R¹B),
R¹⁴ - R¹⁶ may be the same or different and each is a hydrogen atom, a straight chain or branched alkyl group, or a straight chain or branched alkoxy group,
in the aforementioned chemical formula (R¹C),
R¹⁷ - R¹⁹ are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which may be the same or different,
in the aforementioned chemical formula (R¹D),
R²⁰ - R²² may be the same or different and each is a hydrogen atom, or a straight chain or branched alkyl group.

In the aforementioned chemical formula (R¹A), preferably, R¹¹ - R¹³ may be the same or different and each is a straight chain or branched alkoxy group having 1 - 12 carbon atoms, or a straight chain or branched alkyl group having 1 - 12 carbon atoms, or absent. As mentioned above, R¹¹ - R¹³ are, when they are present, respectively present singly or in plurality, and when present in plurality, they may be the same or different. In the aforementioned chemical formula (R¹B), preferably, R¹⁴ - R¹⁶ may be the same or different and each is a hydrogen atom, a straight chain or branched alkyl group having 1 - 12 carbon atoms, or a straight chain or branched alkyl group having 1 - 12 carbon atoms. In the aforementioned chemical formula (R¹C), preferably, R¹⁷ - R¹⁹ are each a hydrogen atom, halogen, a straight chain or branched alkyl group having 1 - 12 carbon atoms, a straight chain or branched alkenyl group having 2 - 12 carbon atoms, a straight chain or branched alkynyl group having 2 - 12 carbon atoms, a straight chain or branched haloalkyl group having 1 - 12 carbon atoms, an aryl group having 5 - 24 carbon atoms, a heteroaryl group having 5 - 24 carbon atoms, a straight chain or branched arylalkyl group having 6 - 30 carbon atoms, a cycloalkyl group having 3 - 24 carbon atoms, a cycloalkenyl group having 3 - 24 carbon atoms, a straight chain or branched cycloalkylalkyl group having 4 - 30 carbon atoms, a straight chain or branched cyclylalkyl group having 4 - 30 carbon atoms, a straight chain or branched hydroxyalkyl group having 1 - 12 carbon atoms, a straight chain or branched alkoxyalkyl group having 1 - 12 carbon atoms, a straight chain or branched aminoalkyl group having 1 - 12 carbon atoms, a straight chain or branched heterocyclylalkenyl group having 5 - 30 carbon atoms, a straight chain or branched heterocyclylalkyl group having 4 - 30 carbon atoms, a straight chain or branched heteroarylalkyl group having 6 - 30 carbon atoms, a silyl group, a silyloxyalkyl group having 1 - 12 carbon atoms, a mono-, di- or trialkylsilyl group having alkyl carbon number 1 - 12, or an alkyl group having 1 - 12 carbon atoms and substituted by a mono-, di- or trialkylsilyloxy group having alkyl carbon number 1 - 12, which may be the same or different. In the aforementioned chemical formula (R¹D), preferably, R²⁰ - R²² may be the same or different and each is a hydrogen atom, or a straight chain or branched alkyl group having 1 - 12 carbon atoms.

In the glycoside compound of the present invention, the substituent represented by the aforementioned chemical formula (R¹A) is preferably a substituent represented by the following chemical formula (R¹A2):

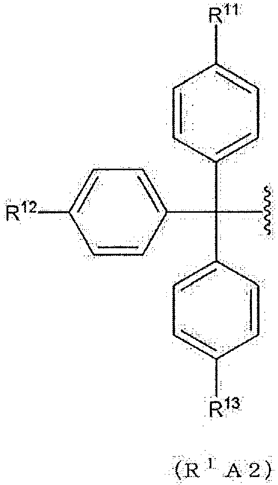

In the aforementioned chemical formula (R¹A2),
R¹¹ - R¹³ may be the same or different and each is a hydrogen atom, a straight chain or branched alkoxy group, or a straight chain or branched alkyl group.

In the aforementioned chemical formula (R¹A2), more preferably, R¹¹ - R¹³ may be the same or different and each is a hydrogen atom, a straight chain or branched alkoxy group having 1 - 12 carbon atoms, or a straight chain or branched alkyl group having 1 - 12 carbon atoms.

In the glycoside compound of the present invention, R¹ in the aforementioned chemical formula (1) is more preferably a hydrogen atom, or a substituent represented by the following chemical formula (R¹Aa), (R¹Ba), (R¹Ca), (R¹Cb) or (R¹Da) :

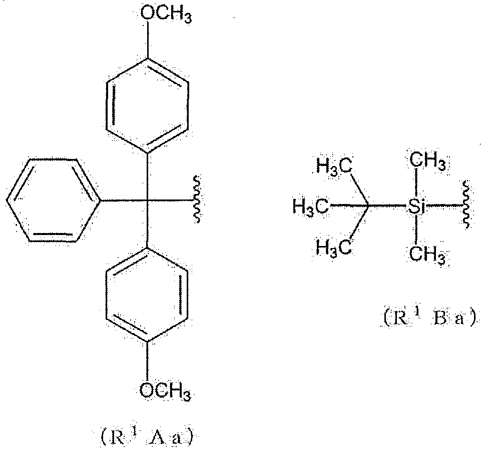

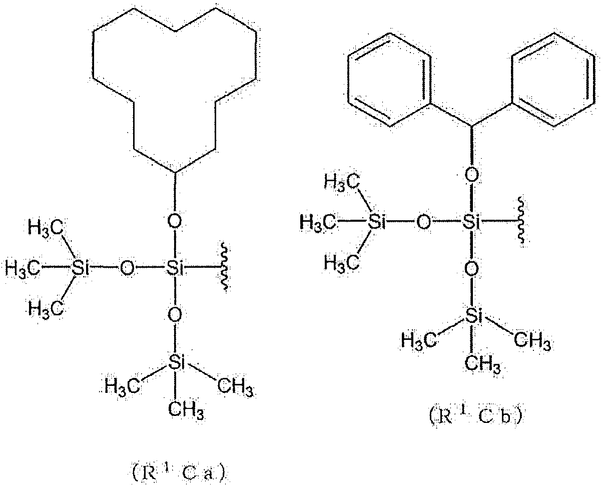

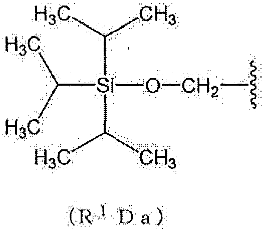

In the aforementioned chemical formulas (R¹R²A) and
(R¹R²B) in the glycoside compound of the present invention, respective R^{1a} may be the same or different, as mentioned above, and each is a hydrogen atom, a straight chain or branched alkyl group, or a straight chain or branched alkoxy group. The aforementioned straight chain or branched alkyl group is more preferably a straight chain or branched alkyl group having 1 - 12 carbon atoms. The aforementioned straight chain or branched alkoxy group is more preferably a straight chain or branched alkoxy group having 1 - 12 carbon atoms.

In the glycoside compound of the present invention, the glycoside compound represented by the aforementioned chemical formula (1) is preferably the glycoside compound represented by the aforementioned chemical formula (2) (hereinafter to be also simply referred to as glycoside compound (2)):

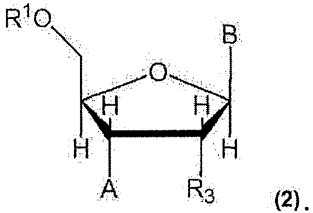

In the aforementioned chemical formula (2),
B, R¹ and R³ are as defined for the aforementioned chemical formula (1),
provided that R¹ is a protecting group,
A is a group represented by the following chemical formula (2-1), (2-2-1), (2-2-2) or (2-2-3):

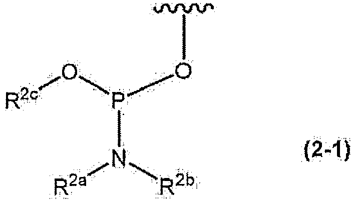

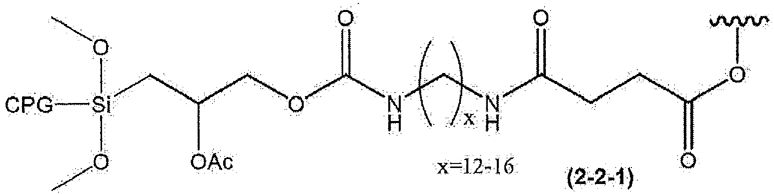

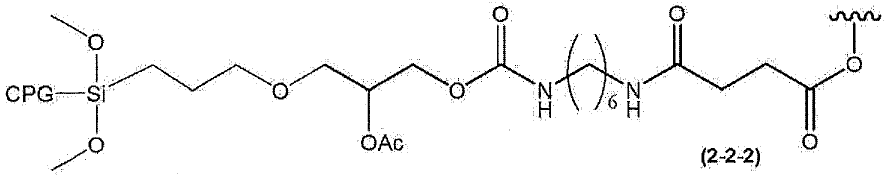

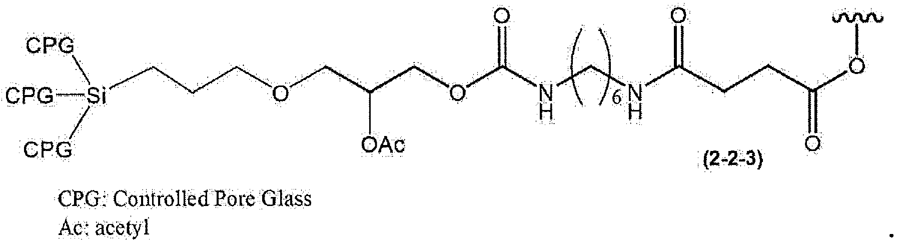

R^{2a} and R^{2b} may be the same or different and each is a hydrogen atom or any substituent,
or R^{2a} and R^{2b} optionally form a nonaromatic ring, in conjunction with a nitrogen atom to which they are bonded, the aforementioned nonaromatic ring optionally has a nitrogen atom, an oxygen atom or a sulfur atom, besides the aforementioned nitrogen atom, and optionally has a substituent, and
R^{2c} is a hydrogen atom, an electron-withdrawing group or any substituent, which may be optionally substituted by an electron-withdrawing group [D²].

In the aforementioned chemical formula (2-1),
R^{2a} and R^{2b} may be the same or different and each is a hydrogen atom or any substituent,
or R^{2a} and R^{2b} optionally form a non-aromatic ring, in conjunction with a nitrogen atom to which they are bonded, the aforementioned non-aromatic ring optionally has a nitrogen atom, an oxygen atom or a sulfur atom, besides the aforementioned nitrogen atom, and optionally has a substituent, and
R^{2c} is a hydrogen atom, an electron-withdrawing group or any substituent, which may be optionally substituted by an electron-withdrawing group [D²].

In the aforementioned chemical formula (2-1), R^{2a} and R^{2b} are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which is preferably optionally further substituted or not substituted by an electron-withdrawing group. Alternatively, R^{2a} and R^{2b} may form, in conjunction with the nitrogen atom bonded thereto, a 5- or 6-membered non-aromatic ring, wherein the aforementioned non-aromatic ring may or may not have a nitrogen atom, an oxygen atom or a sulfur atom besides the aforementioned nitrogen atom, and may or may not further have a substituent.

In the aforementioned chemical formula (2-1), more preferably, R^{2a} and R^{2b} are each a hydrogen atom, halogen, a straight chain or branched alkyl group having 1 - 12 carbon atoms, a straight chain or branched alkenyl group having 2 - 12 carbon atoms, a straight chain or branched alkynyl group having 2 - 12 carbon atoms, a straight chain or branched haloalkyl group having 1 - 12 carbon atoms, an aryl group having 5 - 24 carbon atoms, a heteroaryl group having 5 - 24 carbon atoms, a straight chain or branched arylalkyl group having 6 - 30 carbon atoms, a cycloalkyl group having 3 - 24 carbon atoms, a cycloalkenyl group having 3 - 24 carbon atoms, a straight chain or branched cycloalkylalkyl group having 4 - 30 carbon atoms, a straight chain or branched cyclylalkyl group having 4 - 30 carbon atoms, a straight chain or branched hydroxyalkyl group having 1 - 12 carbon atoms, a straight chain or branched alkoxyalkyl group having 2 - 12 carbon atoms, a straight chain or branched aminoalkyl group having 1 - 12 carbon atoms, a straight chain or branched heterocyclylalkenyl group having 5 - 30 carbon atoms, a straight chain or branched heterocyclylalkyl group having 4 - 30 carbon atoms, a straight chain or branched heteroarylalkyl group having 6 - 30 carbon atoms, a silyl group, a silyloxyalkyl group having 1 - 12 carbon atoms, a mono-, di- or trialkylsilyl group having alkyl carbon number 1 - 12, or an alkyl group having 1 - 12 carbon atoms and substituted by a mono-, di- or trialkylsilyloxy group having alkyl carbon number 1 - 12, which may be further substituted or not substituted by an electron-withdrawing group. Alternatively, R^{2a} and R^{2b} may form, in conjunction with the nitrogen atom bonded thereto, a 5- or 6-membered non-aromatic ring. The aforementioned non-aromatic ring may or may not have a nitrogen atom, an oxygen atom or a sulfur atom besides the aforementioned nitrogen atom, and may or may not further have a substituent.

In the aforementioned chemical formula (2-1), more preferably, R^{2a} and R^{2b} are each a methyl group, an ethyl group, an isopropyl group, or a t-butyl group, or R^{2a} and R^{2b} form, in conjunction with a nitrogen atom bonded thereto, a piperidyl group, a morpholino group, a pyrrolidyl group, a thiomorpholino group, or other nitrogen-containing alicyclic group. More specifically, for example, in the aforementioned chemical formula (2-1), -NR^{2a}R^{2b} is more preferably a diisopropylamino group, a diethylamino group, an ethylmethylamino group, a pyrrolidyl (particularly, pyrrolidin-1-yl) group, a piperidyl (particularly, piperidin-1-yl) group, a morpholino (particularly, morpholin-1-yl) group, a thiomorpholino (particularly, thiomorpholin-1-yl) group, or an arylamino group.

In the aforementioned chemical formula (2-1), R^{2c} is a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, and further preferably may or may not be substituted by an electron-withdrawing group [D²] .

In the aforementioned chemical formula (2-1), R^{2c} is a hydrogen atom, halogen, a straight chain or branched alkyl group having 1 - 12 carbon atoms, a straight chain or branched alkenyl group having 2 - 12 carbon atoms, a straight chain or branched alkynyl group having 2 - 12 carbon atoms, a straight chain or branched haloalkyl group having 1 - 12 carbon atoms, an aryl group having 5 - 24 carbon atoms, a heteroaryl group having 5 - 24 carbon atoms, a straight chain or branched arylalkyl group having 6 - 30 carbon atoms, a cycloalkyl group having 3 - 24 carbon atoms, a cycloalkenyl group having 3 - 24 carbon atoms, a straight chain or branched cycloalkylalkyl group having 4 - 30 carbon atoms, a straight chain or branched cyclylalkyl group having 4 - 30 carbon atoms, a straight chain or branched hydroxyalkyl group having 1 - 12 carbon atoms, a straight chain or branched alkoxyalkyl group having 2 - 12 carbon atoms, a straight chain or branched aminoalkyl group having 1 - 12 carbon atoms, a straight chain or branched heterocyclylalkenyl group having 6 - 30 carbon atoms, a straight chain or branched heterocyclylalkyl group having 4 - 30 carbon atoms, a straight chain or branched heteroarylalkyl group having 6 - 30 carbon atoms, a silyl group, a silyloxyalkyl group having 1 - 12 carbon atoms, a mono-, di- or trialkylsilyl group having alkyl carbon number 1 - 12, or an alkyl group having 1 - 12 carbon atoms and substituted by a mono-, di- or trialkylsilyloxy group having alkyl carbon number 1 - 12, and more preferably may or may not be further substituted by an electron-withdrawing group [D²] .

In the aforementioned chemical formula (2-1), R^{2c} is more preferably a straight chain or branched alkyl group substituted by an electron-withdrawing group [D²] . In the aforementioned chemical formula (2-1), R^{2c} is more preferably a straight chain or branched alkyl group having 1 - 12 carbon atoms and substituted by an electron-withdrawing group [D²].

In the aforementioned chemical formula (2-1), the aforementioned electron-withdrawing group [D²] for R^{2c} is preferably a cyano group, a nitro group, an alkylsulfonyl group, halogen, an arylsulfonyl group, a trihalomethyl group, or a tetraalkylammonium group. The aforementioned trihalomethyl group is, for example, a trichloromethyl group, a trifluoromethyl group or the like.

In the aforementioned chemical formula (2-1), R^{2c} is particularly preferably an alkenyl group or an ethynyl group, or substituted by an electron-withdrawing group [D²] and form, together with [D²], a cyanoethyl group.

In the glycoside compound of the present invention, the glycoside compound represented by the aforementioned chemical formula (1) is more preferably a glycoside compound represented by the following chemical formula (3) (hereinafter to be also simply referred to as glycoside compound (3)).

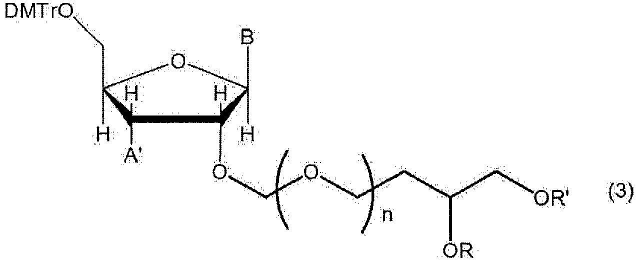

In the aforementioned chemical formula (3),
R, R', B and n are as defined for the aforementioned chemical formula (1),
DMTr is a 4,4'-dimethoxy(triphenylmethyl) group, and
A' is a group represented by the following chemical formula (3-1), (3-2-1) or (3-2-2):

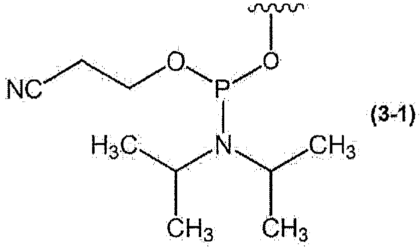

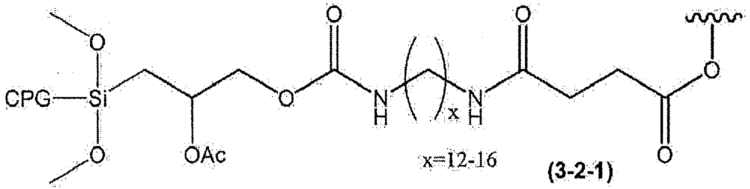

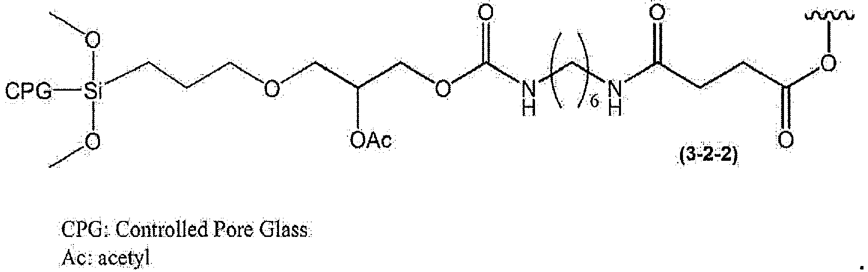

In the glycoside compound of the present invention, the nucleic acid base for B in the aforementioned chemical formula (1) is not particularly limited, but is preferably an atomic group having a natural nucleic acid base skeleton. The aforementioned natural nucleic acid base may or may not have a protecting group. The aforementioned natural nucleic acid base is more preferably adenine, cytosine, guanine, uracil, thymine, or other nitrogen-containing aromatic ring. In the aforementioned chemical formula (1), B is more preferably bonded to the D-ribose skeleton in the aforementioned chemical formula (1) at the 9-position nitrogen of adenine, the 1-position nitrogen of cytosine, 9-position nitrogen of guanine, the 1-position nitrogen of uracil or the 1-position nitrogen of thymine. In addition, as for the nucleic acid base for B, the nucleic acid base (e.g., the aforementioned nucleic bases such as adenine, cytosine, guanine, uracil, thymine and the like) may be substituted or not substituted by any substituent. Examples of the aforementioned substituent include halogen, an acyl group, an alkyl group, an arylalkyl group, an alkoxy group, an alkoxyalkyl group, a hydroxy group, an amino group, a monoalkylamino group, a dialkylamino group, a carboxy group, a cyano group, a nitro group and the like. These substituents may be 0, 1 or plural (e.g., 2 - 3). When they are in plurality, the kind thereof may be one or plural.

As mentioned above, B may or may not have a protecting group. For example, when the aforementioned nucleic acid base for B has an amino group (amino substituent) outside the ring (e.g., the aforementioned nucleic acid base is adenine, guanine, cytosine etc.), the aforementioned amino group may be protected by a protecting group. The aforementioned amino-protecting group is not particularly limited and, for example, may be the same as the protecting group etc. used in known nucleic acids chemistry. Examples of the aforementioned amino-protecting group include acyl group. Examples of the aforementioned acyl group include benzoyl group, 4-methoxybenzoyl group, acetyl group, propionyl group, butyryl group, isobutyryl group, phenylacetyl group, phenoxyacetyl group, 4-tert-butylphenoxyacetyl group, 4-isopropylphenoxyacetyl group and the like. Other than acyl group, for example, a (dimethylamino)methylene group and the like.

In the glycoside compound of the present invention, the glycoside compound represented by the aforementioned chemical formula (1) is more preferably a glycoside compound represented by the following chemical formula (AA5), (AB5), (C5), (G5) or (U5) (hereinafter to be also simply referred to as glycoside compound (AA5), (AB5), (C5), (G5) or (U5), respectively):

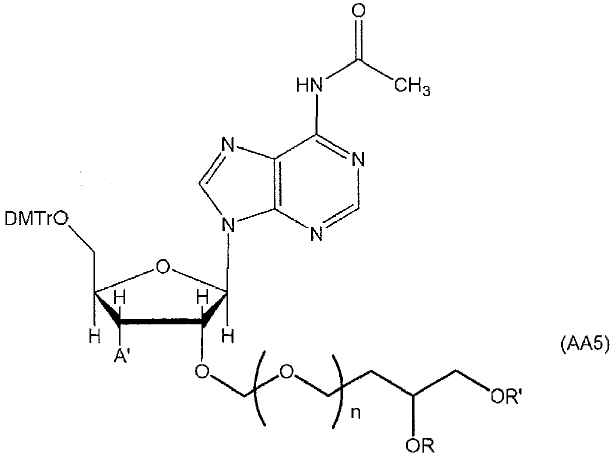

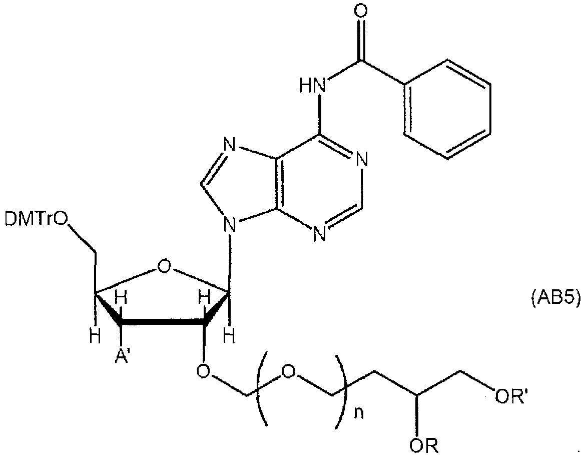

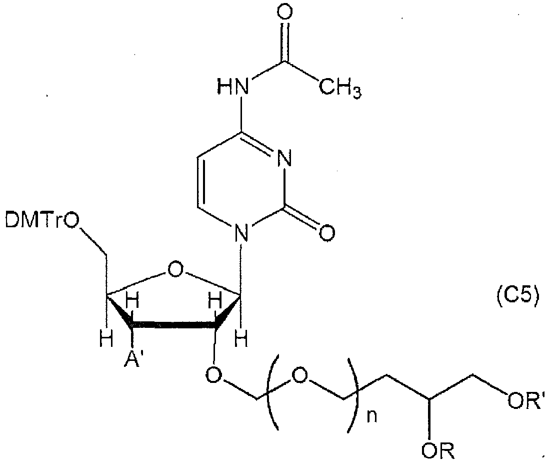

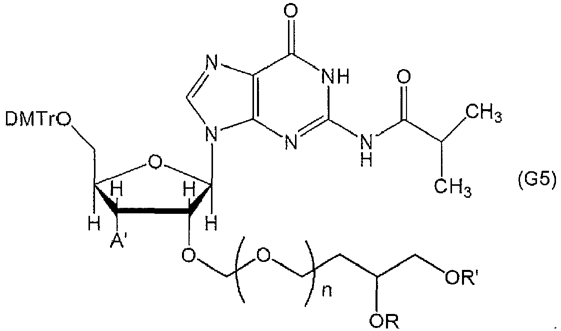

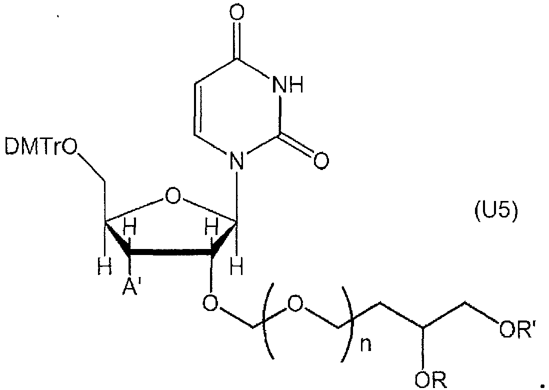

In the aforementioned chemical formulas (AA5), (AB5), (C5), (G5) and (U5), R, R' and n are as defined for the aforementioned chemical formula (1), DMTr is a 4,4'-dimethoxy(triphenylmethyl) group, and A' is a group represented by the following chemical formula (3-1), (3-2-1) or (3-2-2):

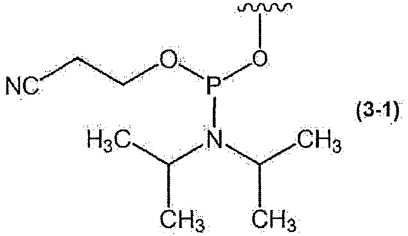

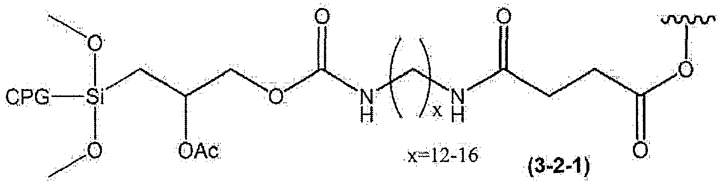

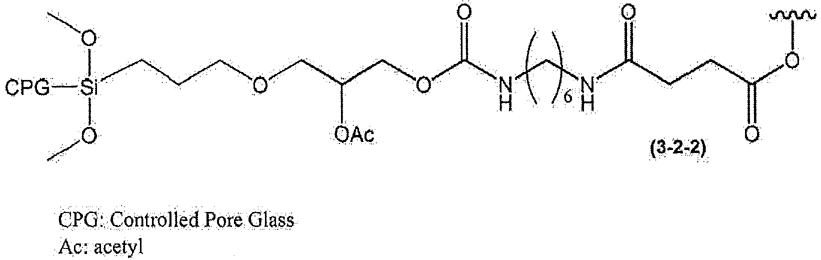

When A' is the aforementioned chemical formula (3-1), the compounds represented by the aforementioned chemical formulas (AA5), (AB5), (C5), (G5) and (U5) are respectively represented by the following chemical formula (AA5-1), (AB5-1), (C5-1), (G5-1) and (U5-1). When A' is the aforementioned chemical formula (3-2-1), the compounds represented by the aforementioned chemical formulas (AA5), (AB5), (C5), (G5) and (U5) are respectively represented by the following chemical formula (AA5-2-1), (AB5-2-1), (C5-2-1), (G5-2-1) and (U5-2-1). When A' is the aforementioned chemical formula (3-2-2), the compounds represented by the aforementioned chemical formulas (AA5), (AB5), (C5), (G5) and (U5) are respectively represented by the following chemical formulas (AA5-2-2), (AB5-2-2), (C5-2-2), (G5-2-2) and (U5-2-2). In each formula, R, R' and n are as defined for the aforementioned chemical formula (1), DMTr is a 4,4'-dimethoxy(triphenylmethyl) group, and CPG shows Controlled Pore Glass.

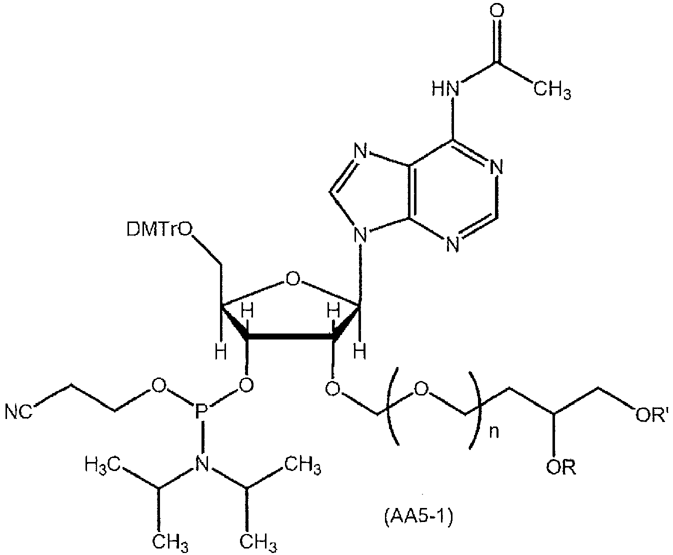

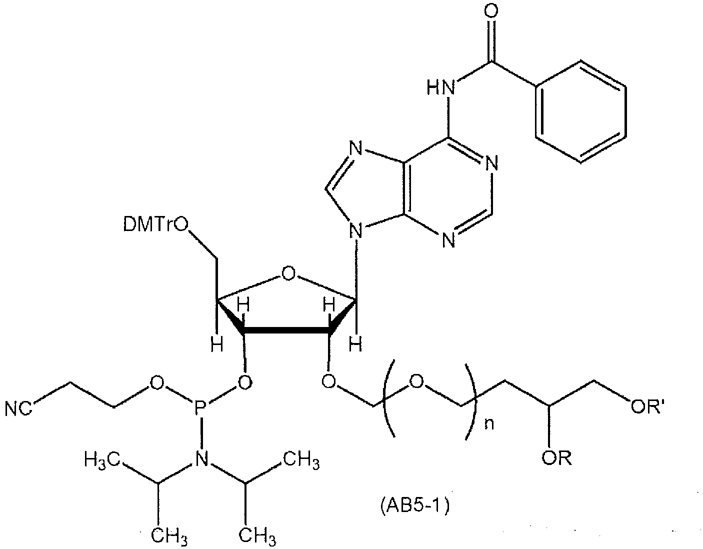

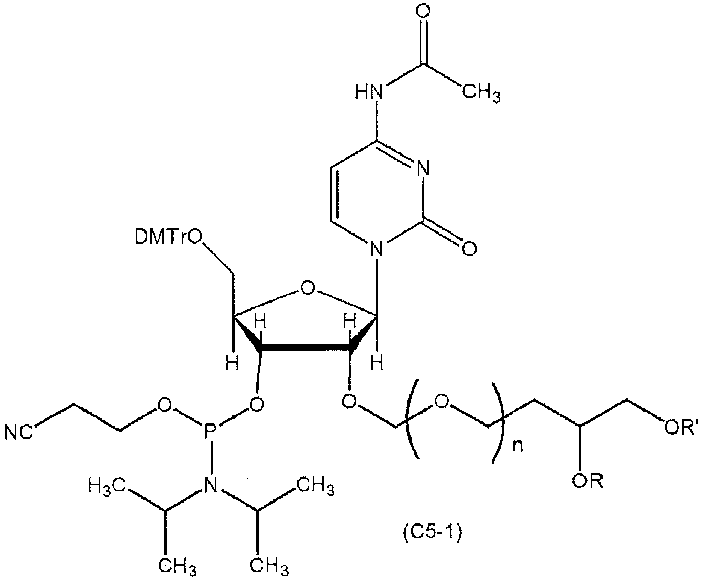

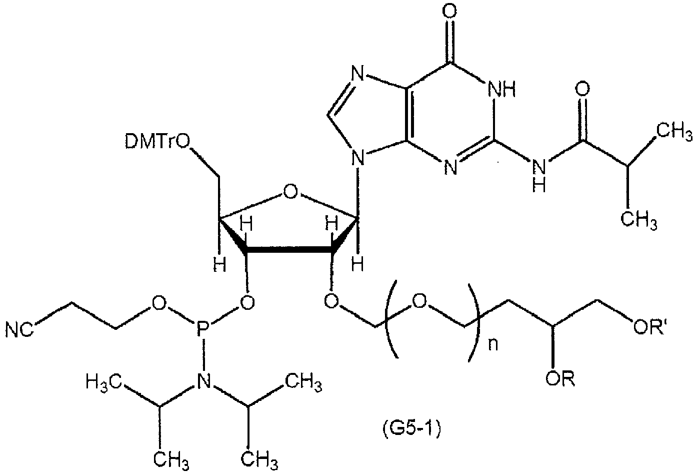

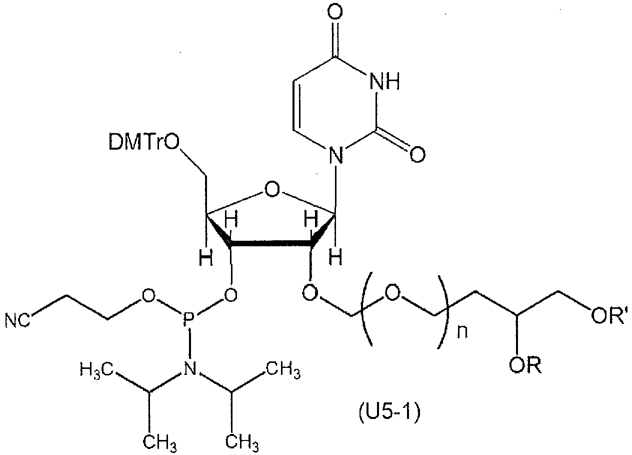

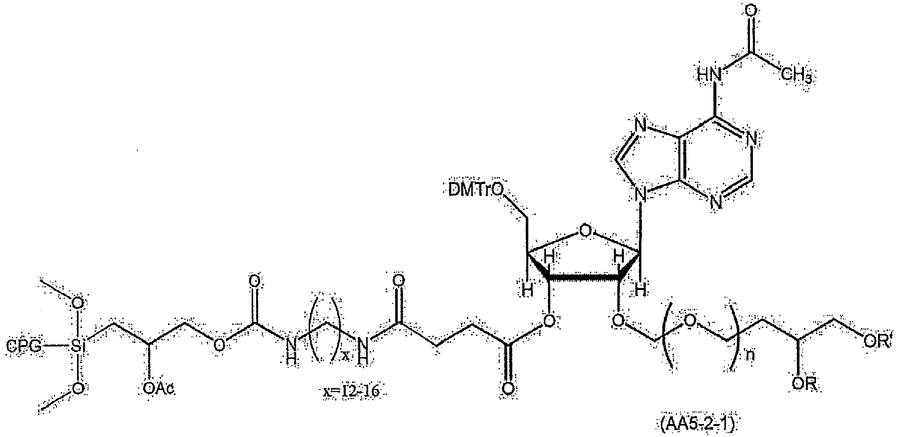

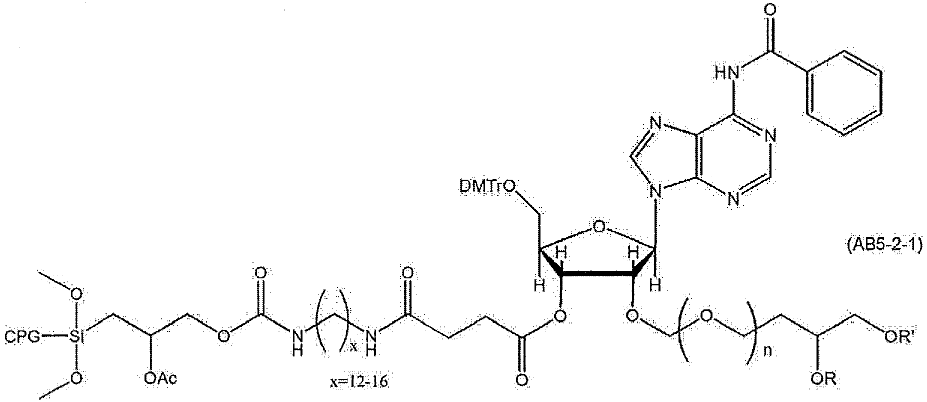

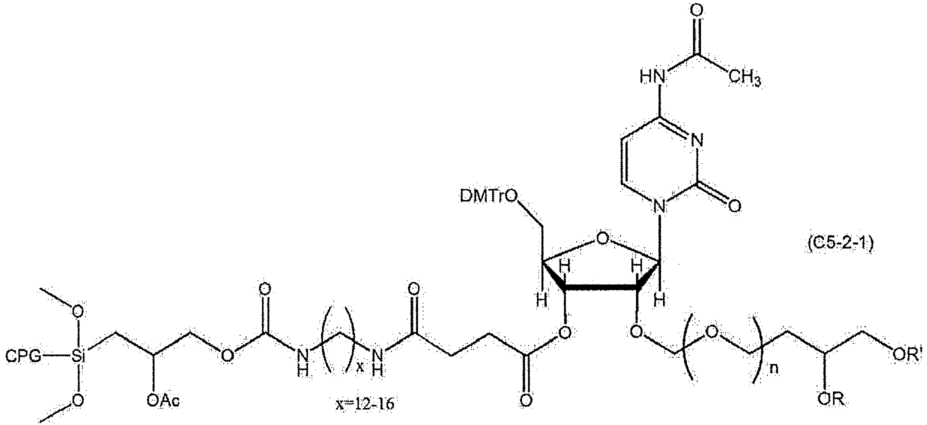

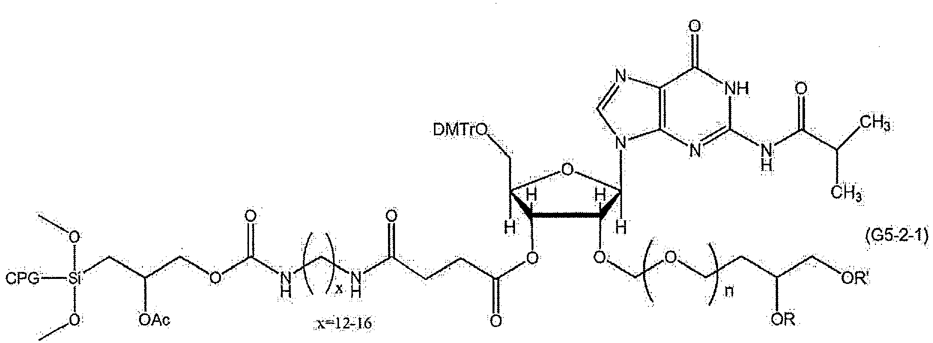

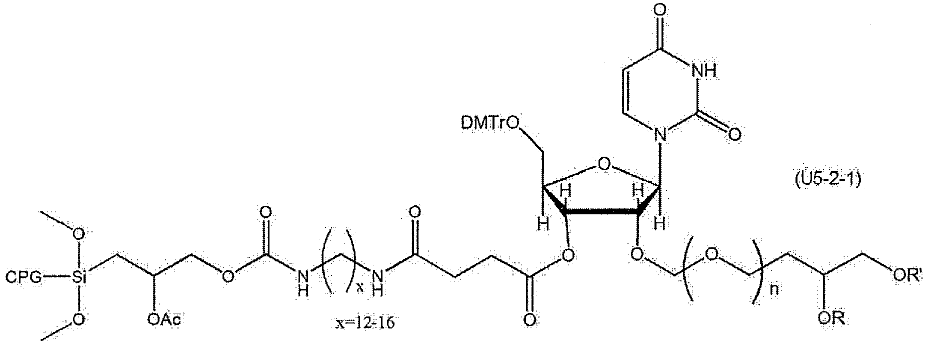

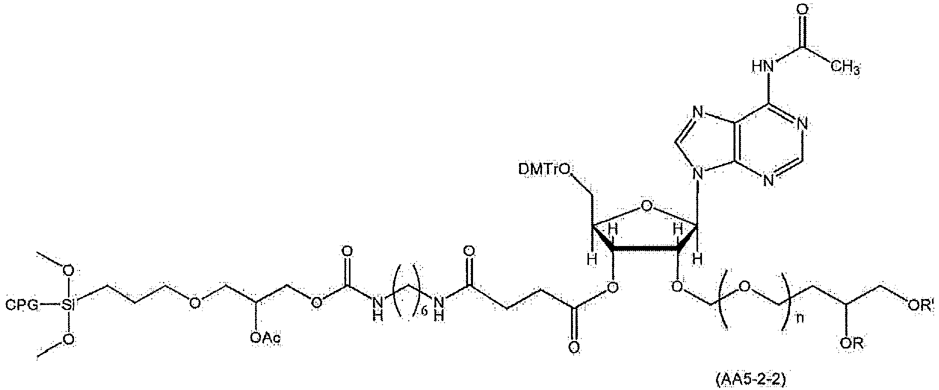

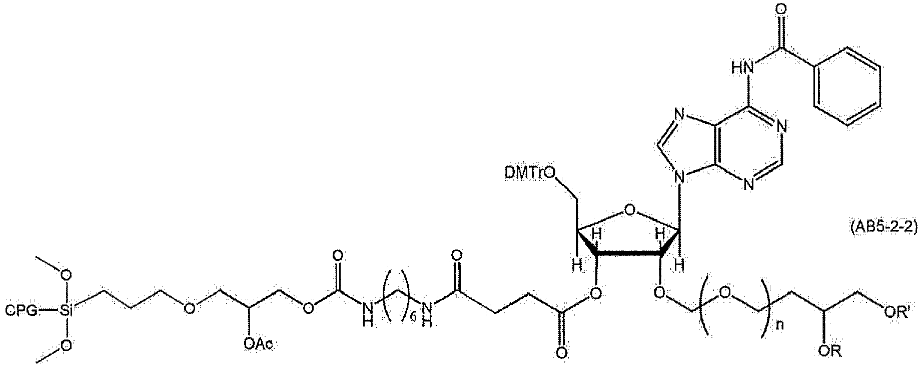

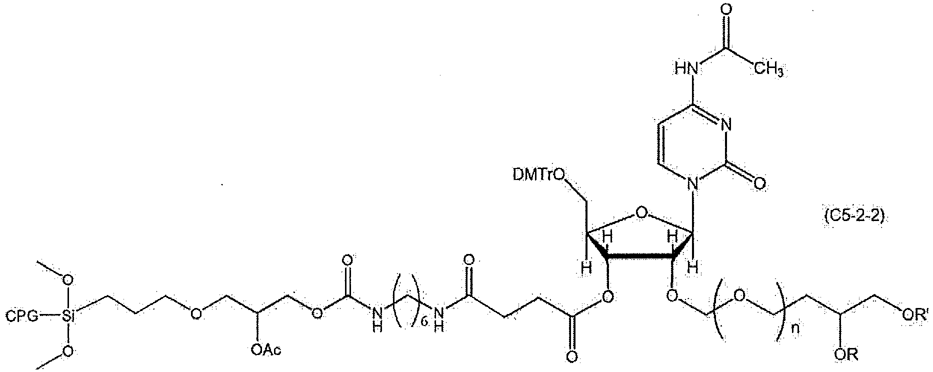

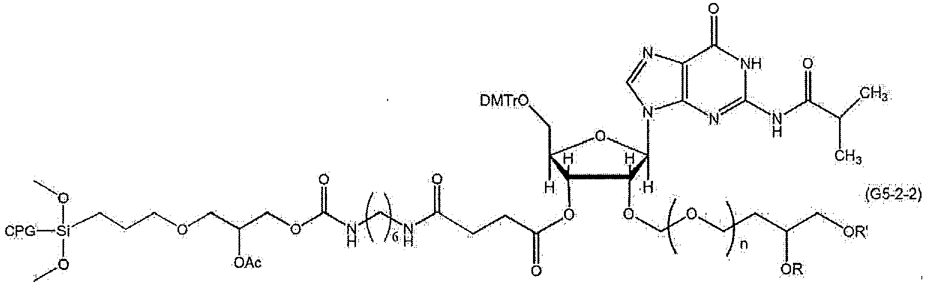

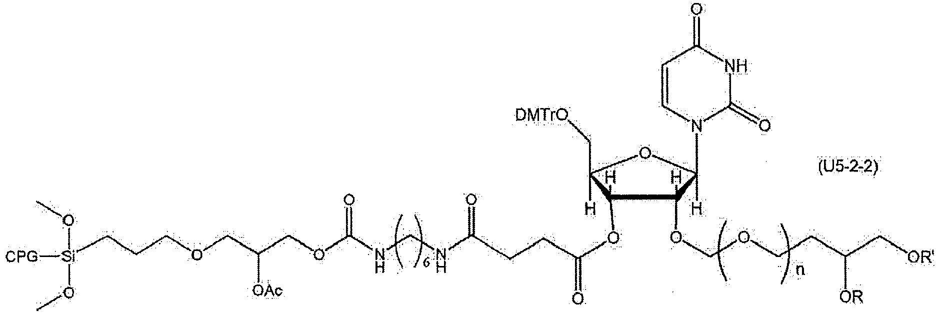

In the aforementioned chemical formulas, n=1 is particularly preferable from the aspects of easiness of synthesis and the like.

When an isomer such as enantiomer, tautomer or stereoisomer (e.g., geometric isomer, conformational isomer and optical isomer) and the like is present in the novel compounds provided by the present invention such as the glycoside compound, ether and the like of the present invention (hereinafter sometimes to be simply referred to as "the compound of the present invention"), all isomers are encompassed in the compound of the present invention. For example, while the chemical formulas showing the glycoside compounds of the present invention depicts as if the sugar skeleton of glycoside is D-ribose, it may be an enantiomer thereof, i.e., L-ribose. When the compound of the present invention can form a salt, such salt is also encompassed in the compound of the present invention. The aforementioned salt of the compound of the present invention may be an acid addition salt or a base addition salt. Furthermore, an acid that forms the aforementioned acid addition salt may be an inorganic acid or an organic acid, and a base that forms the aforementioned base addition salt may be an inorganic base or an organic base. While the aforementioned inorganic acid is not particularly limited, for example, sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluorine acid, chlorine acid, bromine acid, iodine acid, perfluoric acid, perchloric acid, perbromic acid, periodic acid and the like can be mentioned. While the aforementioned organic acid is not particularly limited, for example, p-toluenesulfonic acid, methanesulfonic acid, oxalic acid, p-bromobenzenesulfonic acid, carbonic acid, succinic acid, citric acid, benzoic acid, acetic acid and the like can be mentioned. While the aforementioned inorganic base is not particularly limited, for example, ammonium hydroxide, alkali metal hydroxide, alkaline earth metal hydroxide, carbonate and hydrogencarbonates and the like can be mentioned and, more specifically, for example, sodium hydroxide, potassium hydroxide, potassium carbonate, sodium carbonate, sodium hydrogen carbonate, potassium hydrogen carbonate, calcium hydroxide and calcium carbonate and the like can be mentioned. The aforementioned organic base is not particularly limited and, for example, ethanolamine, triethylamine and tris(hydroxymethyl)aminomethane and the like can be mentioned. The production method of these salts is not particularly limited, and they can be produced by, for example, a method including appropriately adding the aforementioned acid or base to the aforementioned electron donor acceptor connected molecule by a known method and the like. When an isomer is present in the substituent and the like, any isomer can be used. For example, the "naphthyl group" may be a 1-naphthyl group or a 2-naphthyl group, and the "propyl group" may be an n-propyl group or an isopropyl group.

### 2. Ether

Ether of the present invention is, as mentioned above, ether represented by the following chemical formula (105) (hereinafter to be also simply referred to as compound (105)), an enantiomer, tautomer or stereoisomer thereof or a salt thereof: 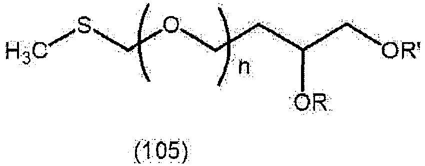 wherein,
n is a positive integer, and
R and R' are the same or different and each is a hydrogen atom or a hydroxyl-protecting group.

In the aforementioned chemical formula (105), the hydroxyl-protecting group is, for example, an acyl-protecting group such as acetyl, benzoyl and the like, an alkyl-protecting group such as trityl, 4-methoxytrityl, 4,4'-dimethoxytrityl, benzyl and the like, or a silyl-protecting group such as trimethylsilyl, triisopropylsilyl, tertbutyldimethylsilyl, tert-butyldiphenylsilyl and the like, preferably an acyl group, more preferably an acetyl group. In the aforementioned chemical formula (105), n is not particularly limited and, for example, it is within the range of 1 - 30, preferably 1 - 20, and n is particularly preferably 1.

The ether represented by the aforementioned chemical formula (105) of the present invention is preferable as a synthetic intermediate for the aforementioned glycoside compound of the present invention. However, the ether of the present invention is not limited thereto and may be used for any use.

### 3. Production method of ether

One embodiment of the production method of ether of the present invention includes the following steps (Production Method 1):
(1) methylthiomethylating a compound represented by the following chemical formula (102) (hereinafter to be also simply referred to as compound (102)) to produce a compound represented by the following chemical formula (103) (hereinafter to be also simply referred to as compound (103));
(2) subjecting the compound (103) to a ring opening reaction to produce a compound represented by the following chemical formula (104) (hereinafter to be also simply referred to as compound (104)); and
(3) protecting a hydroxyl group of the compound (104), and repeating, where necessary, a coupling reaction with methylthiomethanol in the presence of a halogenating agent and a Lewis acid to produce an ether represented by the following chemical formula (105) (hereinafter to be also simply referred to as compound (105)). 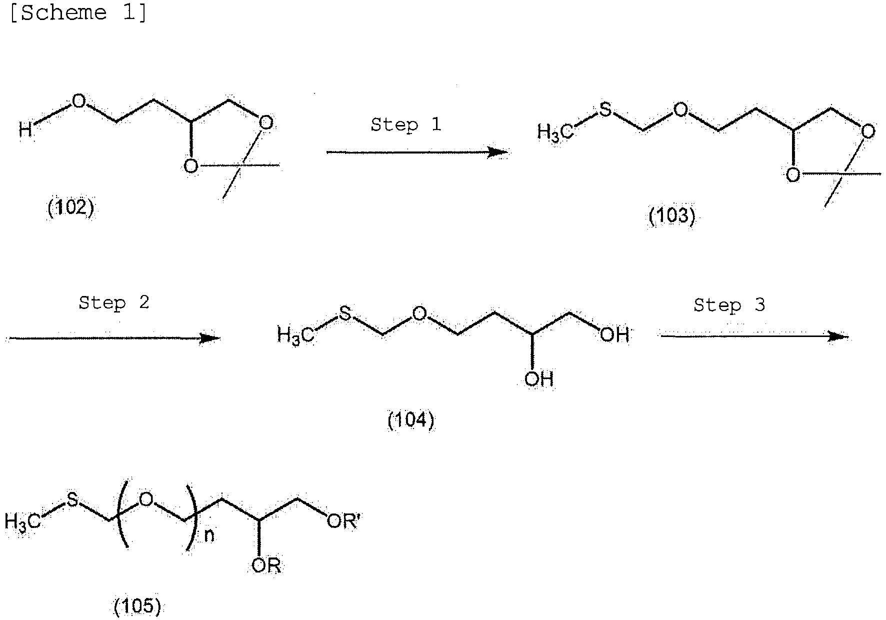
in each formula,
n is a positive integer, and
R and R' are the same or different and each is a hydrogen atom or a hydroxyl-protecting group.

Compound (102) can be produced by protecting diol represented by the following chemical formula (101) (hereinafter to be also simply referred to as compound (101)) by cyclization.

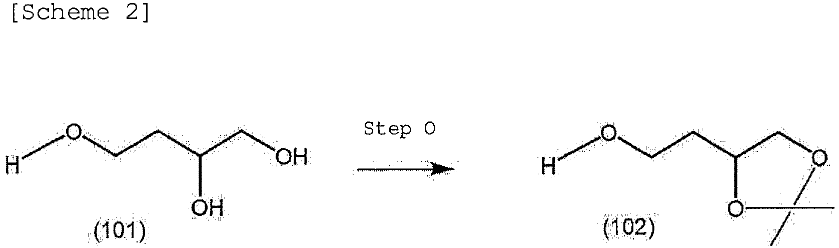

In Scheme 1, n is not particularly limited as long as it is a positive integer. For example, it is 1 - 30, preferably 1 - 20, more preferably 1.

In Scheme 1, the hydroxyl-protecting group for R or R' is not particularly limited as long as it can protect a hydroxyl group. It is preferably a general hydroxyl-protecting group known to those of ordinary skill in the art, more preferably an acyl-protecting group such as acetyl, benzoyl and the like, particularly preferably an acetyl group. R and R' may be the same or different, and preferably the same to be more convenient.
(1) The conditions of step 1 (methylthiomethylation reaction) in Scheme 1 are not particularly limited and, for example, step 1 can be performed by reacting compound (102) with a methylthiomethylating agent such as dimethyl sulfoxide and the like in the presence of an acid in a reaction solvent. The reaction solvent is not particularly limited as long as it is not involved in the reaction and conventionally-known solvents can be used. Carboxylic acid and carboxylate solvents such as acetic acid, methyl acetate and the like; alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; and a mixed solvent thereof can be used, and dimethyl sulfoxide is particularly preferable. As the acid, acetic acid is preferable. The reaction temperature is 0 - 80°C, preferably 20 - 60°C. While the reaction time is not particularly limited, it is, for example, 1 - 10 hr, preferably 2 - 8 hr, more preferably 3 - 6 hr. The concentrations of compound (102) and a methylthiomethylating agent are not particularly limited, and can be appropriately determined. The substance amount ratio of compound (102) and the methylthiomethylating agent is not particularly limited and may be, for example, a stoichiometric mixture ratio or any other ratio. Also, the amount of other reaction substance to be used is not particularly limited. The number of moles of the methylthiomethylating agent is, for example, 1- to 30-fold, preferably 3- to 20-fold, more preferably 5- to 15-fold, relative to that of compound (102). The reaction conditions of the reaction may be appropriately determined by referring to, for example, the below-mentioned Example 1.
(2) The conditions of step 2 (ring opening reaction) in Scheme 1 are not particularly limited and, for example, step 1 can be performed by reacting compound (103) with an acid in a reaction solvent. The reaction solvent is not particularly limited as long as it is not involved in the reaction and conventionally-known solvents can be used. Water; alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; and a mixed solvent thereof can be used, and water is particularly preferable. As the acid, acetic acid is preferable. Step 1 can be performed within a range of 0 - 40°C, and room temperature is particularly preferable. The reaction time is not particularly limited and is, for example, 30 min - 6 hr, preferably 30 min - 5 hr, more preferably 1 - 4 hr. The concentrations of compound (103) and the acid are not particularly limited, and can be appropriately determined. The substance amount ratio of compound (103) and the acid is not particularly limited and may be, for example, a stoichiometric mixture ratio or any other ratio. Also, the amount of other reaction substance to be used is not particularly limited. The number of moles of the acid is, for example, 1- to 40-fold, preferably 5- to 30-fold, more preferably 10- to 20-fold, relative to that of compound (103). The reaction conditions of the reaction may be appropriately determined by referring to, for example, the below-mentioned Example 1.
(3) The conditions of step 3 (reaction to protect hydroxyl group) in Scheme 1 are not particularly limited, and the conditions of the coupling reaction with methylthiomethanol in the presence of a halogenating agent and a Lewis acid, which is performed as necessary, are not particularly limited.

For protection of a hydroxyl group, a protecting group that can be deprotected under basic conditions, for example, an acyl-protecting group, is used. The acylating agent is not particularly limited and, for example, acetic anhydride, phenoxyacetic anhydride and the like can be mentioned. For example, the aforementioned acylating agent may be used as a solution. While the solvent of the aforementioned solution is not particularly limited, for example, pyridine, dichloromethane, acetonitrile, tetrahydrofuran, and a mixed solvent thereof and the like can be mentioned. Particularly preferred is pyridine. The concentration of the aforementioned solution is not particularly limited and it is, for example, 0.05 - 1 M. In addition, an appropriate reaction promoter such as 4-dimethylaminopyridine, N-methylimidazole and the like may be used in combination. While the reaction temperature is not particularly limited, it is 20 - 50°C, preferably room temperature. The reaction time is not particularly limited and varies depending on the kind of the acylating agent to be used, reaction temperature and the like. It is, for example, 2 - 8 hr, preferably 4 - 6 hr. The amount of the aforementioned acylating agent to be used is not particularly limited. It is, for example, 1- to 30-fold, preferably 2- to 20-fold, more preferably 5- to 10-fold, relative to that of compound (104).

Where necessary, the obtained compound is subjected to a coupling reaction with methylthiomethanol in the presence of a halogenating agent and Lewis acid to extend the carbon chain. While the reaction solvent for the coupling reaction is not particularly limited, for example, ketones such as acetone, methyl ethyl ketone and the like, ethers such as diethyl ether, THF (tetrahydrofuran), dioxane and the like, nitriles such as acetonitrile etc., and the like can be mentioned. The aforementioned halogenating agent is not particularly limited, but preferably at least one selected from the group consisting of N-chlorosuccinimide, N-bromosuccinimide, N-iodosuccinimide, iodine, bromine and chlorine. Also, the aforementioned Lewis acid is not particularly limited, but preferably at least one selected from the group consisting of perfluoroalkylcarboxylic acid, perfluoroalkylsulfonic acid, alkylsulfonic acid and a salt thereof. While the reaction time of the aforementioned coupling reaction is not particularly limited, it is, for example, 30 min - 4 hr, preferably 30 min - 2 hr, more preferably 30 min - 1 hr. While the reaction temperature of the aforementioned coupling reaction is not particularly limited, it is, for example, 15 - 40°C, preferably 15 - 37°C, more preferably 20 - 37°C. The concentration of the halide is not particularly limited, and can be appropriately determined.

Compound (102) can be produced by protecting compound (101) by cyclization (Scheme 2). The conditions of step 0 (cyclization reaction) in Scheme 2 are not particularly limited. The reaction solvent is not particularly limited as long as it is not involved in the reaction and conventionally-known solvents can be used. Ketone solvents such as acetone and the like; alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; and a mixed solvent thereof can be used. Acetone is particularly preferable. While the acid is not particularly limited, p-toluenesulfonic acid is preferable. Step 0 can be performed within the range of 0 - 40°C, and room temperature is particularly preferable. While the reaction time is not particularly limited, it is, for example, 10 - 24 hr, preferably 10 hr - overnight. The concentrations of compound (101), acid and base are not particularly limited, and can be appropriately determined. The substance amount ratio of compound (101), acid and base is not particularly limited and may be, for example, a stoichiometric mixture ratio or any other ratio. The amount of other reaction substance to be used is not particularly limited. The number of moles of the acid is, for example, 0.01- to 10-fold, preferably 0.01- to 0.1-fold, relative to that of compound (101). The reaction conditions of the reaction may be appropriately determined by referring to, for example, the below-mentioned Example 1.

Compound (102) can also be produced by the following steps:
(1) alkaline hydrolyzing a compound represented by the following chemical formula (101-1) (hereinafter to be also simply referred to as compound (101-1)) to produce a compound represented by the following chemical formula (101-2) (hereinafter to be also simply referred to as compound (101-2));
(2) producing a compound represented by the following chemical formula (101-3) (hereinafter to be also simply referred to as compound (101-3)) by protecting a hydroxyl group of compound (101-2) ;
(3) oxidizing compound (101-3) to produce a diol represented by the following chemical formula (101-4) (hereinafter to be also simply referred to as compound (101-4));
(4) isopropylidenating compound (101-4) to produce a compound represented by the following chemical formula (101-5) (hereinafter to be also simply referred to as compound (101-5)); and
(5) deprotecting a hydroxyl group of compound (101-5) to produce compound (102). 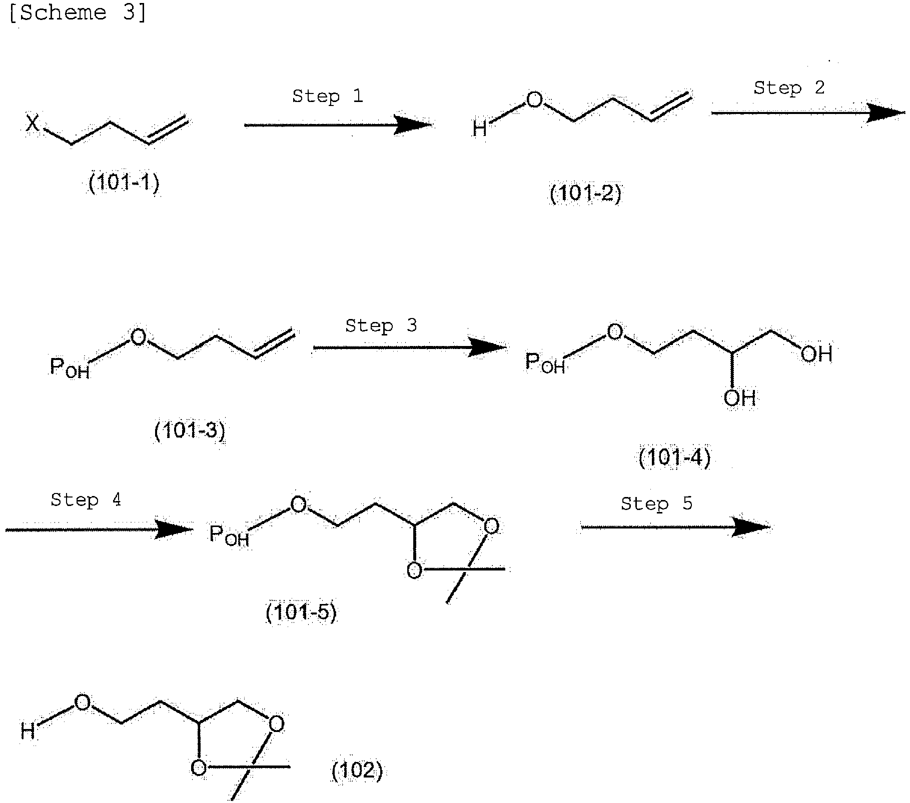
in each formula,
P_{OH} is a hydroxyl-protecting group, and
X is a halogen atom, an acetyloxy group, an optionally substituted carboxyl group or an optionally substituted carbamoyl group.

The hydroxyl-protecting group for P_{OH} is not particularly limited as long as it can protect a hydroxyl group. It is preferably a general hydroxyl-protecting group known to those of ordinary skill in the art, more preferably a silyl-protecting group, an acyl-protecting group such as acetyl, benzoyl and the like, particularly preferably a triisopropylsilyl group.

X is preferably a halogen atom.
(1) The conditions of step 1 (alkaline hydrolysis) in Scheme 3 are not particularly limited and, for example, step 1 can be performed by reacting compound (101-1) with an alkali in a reaction solvent. The reaction solvent is not particularly limited as long as it is not involved in the reaction and conventionally-known solvents can be used. Alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; basic solvent such as pyridine, triethylamine and the like; and a mixed solvent thereof can be used, and tetrahydrofuran is particularly preferable. Examples of the alkali include alkali metal hydroxides such as lithium hydroxide, sodium hydroxide, potassium hydroxide and the like, ammonia, amines and the like. From the aspect of handling property, alkali metal hydroxide is preferable and sodium hydroxide is particularly preferable. The reaction temperature and reaction time can be appropriately determined according to a conventional method. The concentration and the amount of use of each reaction substance are not particularly limited, and can be appropriately determined within the range where the reaction proceeds.
(2) The conditions of step 2 (hydroxyl group protection reaction) in Scheme 3 are not particularly limited and appropriately determined according to the protecting group to be used. When an acyl-protecting group such as acetyl group and the like is used as the protecting group, a method of treating with acetyl chloride or acetic anhydride (preferably acetic anhydride) in the presence of a base such as pyridine, triethylamine and the like (preferably pyridine) can be mentioned. When a silyl-protecting group such as triisopropylsilyl group and the like is used as the protecting group, a method of reacting various silylchlorides (preferably triisopropylsilyl chloride) in the presence of a base such as imidazole and the like in a reaction solvent (preferably dimethylformamide) can be mentioned. The reaction temperature and reaction time can be appropriately determined according to a conventional method. The concentration and the amount of use of each reaction substance are not particularly limited, and can be appropriately determined within the range where the reaction proceeds.
(3) The conditions of step 3 (oxidation reaction) in Scheme 3 are not particularly limited and, for example, step 3 can be performed by reacting compound (101-3) with an oxidant in a reaction solvent. The reaction solvent is not particularly limited as long as it is not involved in the reaction and conventionally-known solvents can be used. Ketone solvents such as acetone and the like; alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; basic solvent such as pyridine, triethylamine and the like; and a mixed solvent thereof can be used. Examples of the oxidant include hydrogen peroxide, potassium permanganate, potassium chlorate, potassium dichromate, sodium bromate, potassium bromate, halogen, concentrated sulfuric acid, nitric acid, sodium hypochlorite, chlorine dioxide,-chloramine, osmium tetroxide, dimethyl sulfoxide, metachloroperbenzoic acid and the like, preferably osmium tetroxide. The reaction temperature and reaction time can be appropriately determined according to a conventional method. The concentration and the amount of use of each reaction substance are not particularly limited, and can be appropriately determined within the range where the reaction proceeds.
(4) The conditions of step 4 (protection reaction by isopropylidenation) in Scheme 3 are not particularly limited and, for example, step 4 can be performed by reacting compound (101-4) with a para-toluenesulfonic acid in acetone. The reaction temperature and reaction time can be appropriately determined according to a conventional method. The concentration and the amount of use of each reaction substance are not particularly limited, and can be appropriately determined within the range where the reaction proceeds.
(5) The conditions of step 5 (deprotection reaction) in Scheme 3 are not particularly limited and appropriately determined by the protecting group (protecting group to be eliminated) used in step 2. When an acyl-protecting group such as acetyl group and the like is used as the protecting group, alkali water such as sodium methoxide, aqueous sodium hydroxide solution, aqueous ammonia and the like can be used. When a silyl-protecting group such as triisopropylsilyl group and the like is used as the protecting group, deprotecting agents such as hydrogen fluoride pyridine, hydrogen fluoride triethylamine, ammonium fluoride, hydrofluoric acid, tetrabutyl ammonium fluoride and the like are used in a solvent. The reaction solvent for the aforementioned deprotection reaction is not particularly limited, and examples thereof include ketones such as acetone, ethers such as diethyl ether and THF (tetrahydrofuran), alcohols such as methanol and ethanol, nitriles such as acetonitrile and the like. The reaction temperature and reaction time can be appropriately determined according to a conventional method. The concentration and the amount of use of each reaction substance are not particularly limited, and can be appropriately determined within the range where the reaction proceeds.

Compound (102) can also be produced by the following steps:
(1) protecting a polyol represented by the following chemical formula (101-5) (hereinafter to be also simply referred to as compound (101-5)) by cyclization to produce a compound represented by the following chemical formula (101-6) (hereinafter to be also simply referred to as compound (101-6));
(2) periodically oxidizing compound (101-6) to produce a compound represented by the following chemical formula (101-7) (hereinafter to be also referred to as compound (101-7)); and (3) carbon-elongating compound (101-7) to produce compound (102) .

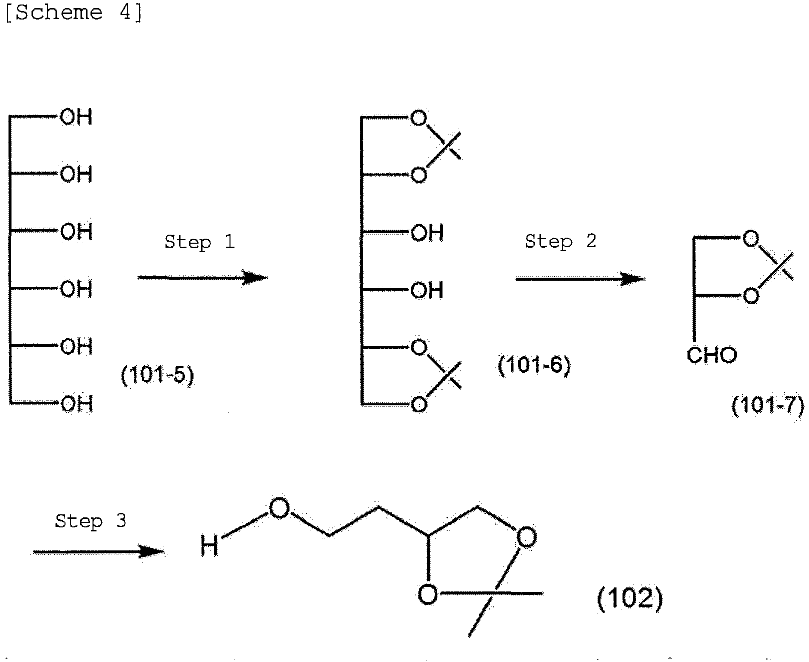

Steps 1 - 2 can be performed, for example, according to the method described in a document (Organic Syntheses, Coll. Vol.9, p450 (1998); Vol.72, p16 (1995)), and step 3 can be performed, for example, according to the method described in a document (European Journal of Organic Chemistry 2015(32), 7009-7019) .

Another embodiment of the production method of ether of the present invention includes the following steps (Production Method 2):
(1) protecting the 4-position hydroxyl group of a compound represented by the following chemical formula (104-1) (hereinafter to be also simply referred to as compound (104-1)) to produce hydroxycarboxylic acid represented by the following chemical formula (104-2) (hereinafter to be also simply referred to as compound (104-2));
(2) reducing a carbonyl group of compound (104-2) and acylating same to protect the hydroxyl group to produce a compound represented by the following chemical formula (104-3) (hereinafter to be also simply referred to as compound (104-3));
(3) deprotecting the 4-position of compound (104-3) to produce a compound represented by the following chemical formula (104-4) (hereinafter to be also simply referred to as compound (104-4)); and
(4) methylthiomethyling compound (104-4), and repeating, where necessary, a coupling reaction with methylthiomethanol in the presence of a halogenating agent and a Lewis acid to produce compound (105). 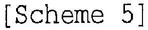 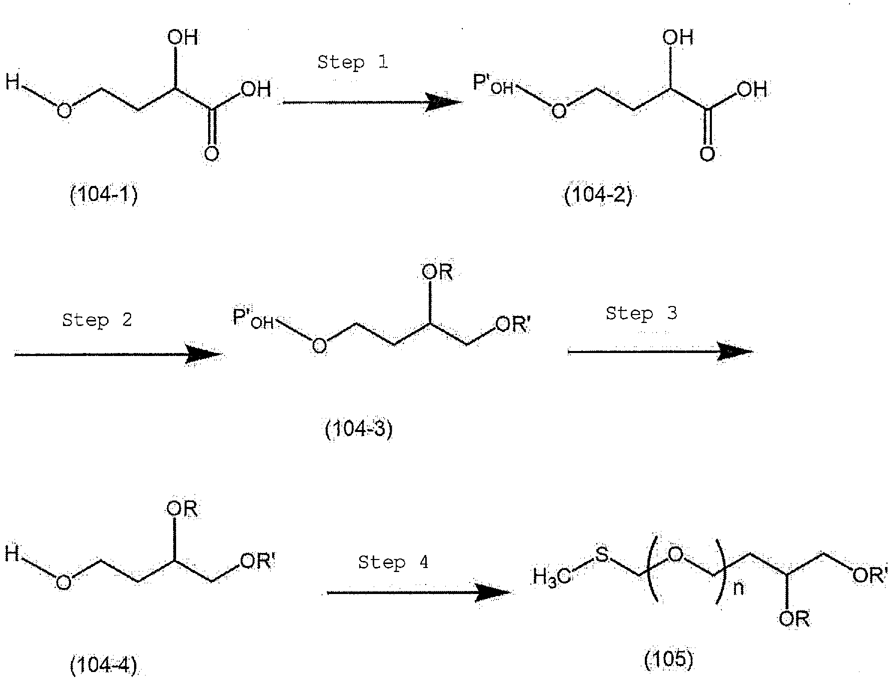
in each formula,
n is a positive integer,
P'_{OH} is a hydroxyl-protecting group,
R and R' are the same or different and each is a hydrogen atom or a hydroxyl-protecting group.

In Scheme 5, n is not particularly limited as long as it is a positive integer and is, for example, 1 - 30, preferably 1 - 20, more preferably 1.

In Scheme 5, the hydroxyl-protecting group for R or R' is not particularly limited as long as it can protect a hydroxyl group. It is preferably a general hydroxyl-protecting group known to those of ordinary skill in the art, more preferably an acyl-protecting group such as acetyl, benzoyl and the like, particularly preferably an acetyl group. R and R' may be the same or different, and preferably the same to be more convenient.

The hydroxyl-protecting group for P'_{OH} is not particularly limited as long as it can protect a hydroxyl group. It is preferably a general hydroxyl-protecting group known to those of ordinary skill in the art, more preferably a protecting group removable with a weak acid or an alkyl-protecting group such as trityl, 4-methoxytrityl, 4,4'-dimethoxytrityl, benzyl and the like, particularly preferably a trityl group.
(1) The conditions of step 1 (hydroxyl group protection reaction) in Scheme 5 are not particularly limited and appropriately determined by the protecting group to be used. When a trityl group is used as the protecting group, a method of treating with a tritylating agent such as trityl chloride and the like in the presence of an organic base (DMAP, collidine, DBU etc.) can be mentioned. The reaction temperature and reaction time can be appropriately determined according to a conventional method. The concentration and the amount of use of each reaction substance are not particularly limited, and can be appropriately determined within the range where the reaction proceeds.
(2) The conditions of step 2 (reduction reaction and acylation reaction) in Scheme 5 are not particularly limited. For example, compound (104-2) is reacted with a hydride reducing agent such as sodium borohydride (NaBH₄), lithium aluminum hydride (LiAlH₄) and the like in a reaction solvent to reduce the carbonyl group, and then an acylation reaction can be performed according to step 3 in Scheme 1. The reaction solvent is not particularly limited as long as it is not involved in the reaction and conventionally-known solvents can be used. Alcohol solvents such as methanol, ethanol and the like; ether solvents such as diethyl ether, 1,4-dioxane, tetrahydrofuran and the like; non-aqueous polar solvents such as N,N-dimethylformamide, dimethyl sulfoxide and the like; basic solvent such as pyridine, triethylamine and the like; and a mixed solvent thereof can be used.
(3) The conditions of step 3 (deprotection reaction) in Scheme 5 are not particularly limited and appropriately determined by the protecting group used in step 1 (i.e., protecting group to be eliminated). When a trityl group is used as the protecting group, bronsted acid such as trifluoroacetic acid and the like, and Lewis acid are used. Deprotection by hydrogenolysis is also performed. The reaction temperature and reaction time can be appropriately determined according to a conventional method. The concentration and the amount of use of each reaction substance are not particularly limited, and can be appropriately determined within the range where the reaction proceeds.
(4) The conditions of step 4 in Scheme 5 are not particularly limited. Methylthiomethylation is performed according to step 1 of Scheme 1 and, where necessary, a coupling reaction with methylthiomethanol is further repeated according to step 3 of Scheme 1 to elongate the carbon chain. The reaction temperature and reaction time can be appropriately determined according to a conventional method. The concentration and the amount of use of each reaction substance are not particularly limited, and can be appropriately determined within the range where the reaction proceeds.

### 4. Production method of glycoside compound

The production method of the glycoside compound of the present invention is not particularly limited and can be appropriately performed by referring to, for example, a known production method of glycoside (ACE amidite etc.). For example, the production method described in Current Protocols in Nucleic Acid Chemistry, unit 2.16.1-2.16.31 (2009) may be referred to.

The glycoside of the present invention is preferably produced by, for example, the aforementioned production method of the present invention (production method of glycoside compound). The aforementioned production method of the present invention (production method of the glycoside compound) includes, as mentioned above, a coupling step including a coupling reaction of a glycoside compound represented by the following chemical formula (106) (hereinafter to be also simply referred to as glycoside compound (106)) and compound (105) in the presence of a halogenating agent and a Lewis acid to give a glycoside compound represented by the following chemical formula (1a) (hereinafter to be also simply referred to as glycoside compound (1a)). The glycoside compound (1a) is a glycoside compound wherein R¹ and R² in the aforementioned chemical formula (1) in conjunction form an atomic group represented by the aforementioned chemical formula (R¹R²A) or (R¹R²B) .

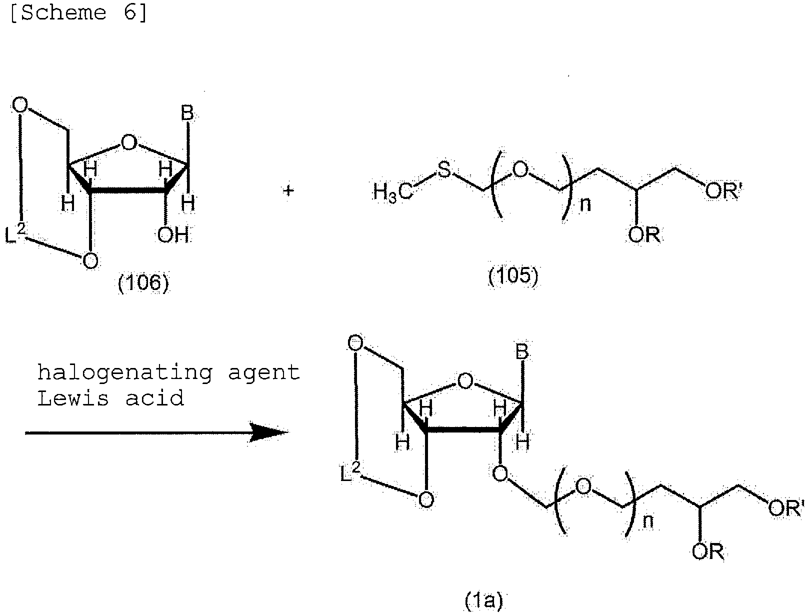

In the aforementioned chemical formulas (106) and (1a),
L² is an atomic group represented by the aforementioned chemical formula (R¹R²A) or (R¹R²B),
in the aforementioned chemical formulas (105), (106) and (1a),
B, n, R and R' are as defined for the aforementioned chemical formula (1).

The method of obtaining the glycoside compound (106) is not particularly limited and, for example, it may be obtained as a commercially available product or may be produced by a known method. In the aforementioned coupling reaction (the aforementioned Scheme 6), the aforementioned halogenating agent is not particularly limited, but preferably at least one selected from the group consisting of N-chlorosuccinimide, N-bromosuccinimide, N-iodosuccinimide, iodine, bromine and chlorine. Also, the aforementioned Lewis acid is not particularly limited, but preferably at least one selected from the group consisting of perfluoroalkylcarboxylic acid, perfluoroalkylsulfonic acid, alkylsulfonic acid and a salt thereof. The aforementioned Lewis acid is particularly preferably trifluoromethanesulfonic acid or methanesulfonic acid.

In the production method of the glycoside compound of the present invention, the conditions of the coupling reaction of the glycoside compound (106) and compound (105) are not particularly limited. While the reaction solvent for the aforementioned coupling reaction is not particularly limited, for example, ketones such as acetone, methyl ethyl ketone, acetophenone and the like, ethers such as diethyl ether, THF (tetrahydrofuran), dioxane and the like, nitriles such as acetonitrile etc., and the like can be mentioned. While the reaction time of the aforementioned coupling reaction is not particularly limited, it is, for example, 30 min - 12 hr, preferably 30 min - 8 hr, more preferably 30 min - 4 hr. While the reaction temperature of the aforementioned coupling reaction is not particularly limited, it is, for example, -75 to 30°C, preferably -60 to 30°C, more preferably -50 to 30°C. The concentration of the glycoside compound (106) and compound (105) is not particularly limited, and can be appropriately determined. The substance amount ratio of the glycoside compound (106) and compound (105) is not particularly limited and may be, for example, a stoichiometric mixture ratio or any other ratio. Also, the amount of other reaction substance to be used is not particularly limited. The number of moles of compound (105) is, for example, 1- to 5-fold, preferably 1- to 3-fold, more preferably 1.2- to 2-fold, relative to that of the glycoside compound (106). The number of moles of the aforementioned halogenating agent is, for example, 1- to 10-fold, preferably 1- to 8-fold, more preferably 1.2- to 6-fold, relative to that of the glycoside compound (106). The number of moles of the aforementioned Lewis acid is, for example, 0.05- to 5-fold, preferably 0.1- to 2-fold, more preferably 0.1 to 1.5-fold, relative to that of the glycoside compound (106). The reaction conditions of the aforementioned coupling reaction may be appropriately determined by referring to, for example, as mentioned above, the conditions of a known amidite synthesis of the glycoside compound, and the like, or by reference to any of the below-mentioned Examples 2 to 5.

The production method of the glycoside compound of the present invention preferably further includes a deprotection step for removing the aforementioned atomic group L² from the glycoside compound (1a) to produce glycoside compound represented by the following chemical formula (1b) (hereinafter to be also simply referred to as glycoside compound (1b)). In this case, the glycoside compound (1b) is a glycoside compound of the aforementioned chemical formula (1) wherein R¹ and R² are hydrogen atoms.

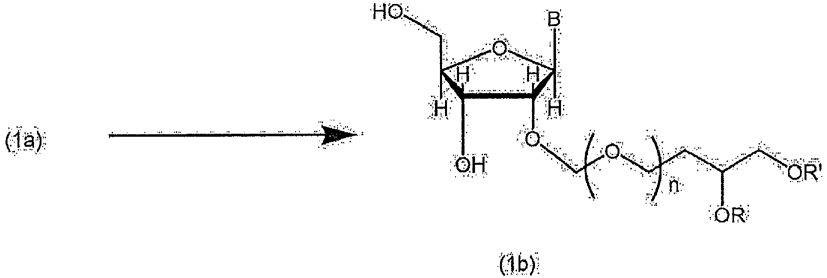

In the aforementioned chemical formula (1b),
B, n, R and R' are as defined for the aforementioned chemical formula (1).

In the aforementioned deprotection step, while the conditions of the deprotection reaction are not particularly limited, for example, a known deprotecting agent can be used. While the aforementioned deprotecting agent is not particularly limited, for example, hydrogen fluoride pyridine, hydrogen fluoride triethylamine, ammonium fluoride, hydrofluoric acid, tetrabutylammoniumfluoride and the like can be mentioned. While the reaction solvent for the aforementioned deprotection reaction is not particularly limited, for example, ketones such as acetone and the like, ethers such as diethyl ether, THF (tetrahydrofuran) and the like, alcohols such as methanol, ethanol and the like, nitriles such as acetonitrile etc., and the like can be mentioned. While the reaction time of the aforementioned deprotection reaction is not particularly limited, it is, for example, 30 min - 24 hr, preferably 2 - 12 hr, more preferably 2 - 4 hr. While the reaction temperature of the aforementioned deprotection reaction is not particularly limited, it is, for example, 0 to 100°C, preferably 20 to 60°C, more preferably 20 to 50°C. The concentration of the glycoside compound (1a) and the aforementioned deprotecting agent is not particularly limited, and can be appropriately determined. The substance amount ratio of the glycoside compound (1a) and the aforementioned deprotecting agent is not particularly limited and may be, for example, a stoichiometric mixture ratio or any other ratio. Also, the amount of other reaction substance to be used is not particularly limited. The number of moles of the aforementioned deprotecting agent is, for example, 1- to 10-fold, preferably 1- to 5-fold, more preferably 1- to 3-fold, relative to that of the glycoside compound (1a). The reaction conditions of the aforementioned deprotection reaction may be appropriately determined by referring to, for example, the conditions of a similar deprotection reaction in a known glycoside compound, and the like, or by reference to any of the below-mentioned Examples 2 to 5.

The production method of the glycoside compound of the present invention preferably further includes an introduction step of a protecting group for introducing protecting groups R¹ and R² into the aforementioned chemical formula (1b) to produce glycoside compound represented by the following chemical formula (1c) (hereinafter sometimes to be simply referred to as glycoside compound (1c)). In this case, the glycoside compound (1c) is a glycoside compound of the aforementioned chemical formula (1) wherein R¹ and R² are other than a hydrogen atom and the aforementioned chemical formulas (R¹R²A) and (R¹R²B).

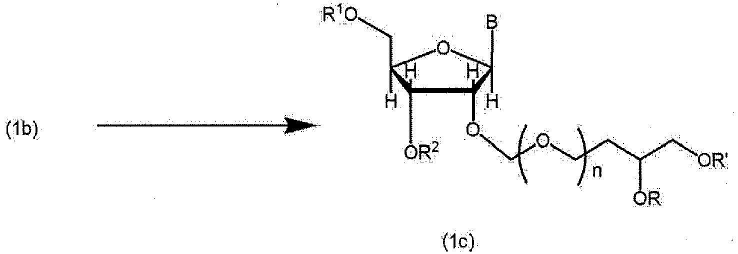

In the aforementioned chemical formula (1c),
R¹ and R² are R¹ and R² in the aforementioned chemical formula (1) and other than a hydrogen atom and the aforementioned chemical formulas (R¹R²A) and (R¹R²B), and
B, n, R and R' are as defined for the aforementioned chemical formula (1).

The reaction conditions of the aforementioned protecting group introduction step are not particularly limited and may be appropriately determined, for example, by referring to a similar reaction in a known glycoside compound and the like. In the aforementioned protecting group introduction step, for example, the aforementioned R¹ and R² may be simultaneously (in one step) introduced, or R² may be added after introduction of R¹, or R¹ may be introduced after introduction of R². For example, it is preferable to introduce R² after introduction of R¹. While the protecting groups R¹ and R² are not particularly limited, for example, they are as mentioned above.

In an introduction reaction of the protecting group R¹, a protecting group-introducing agent may be appropriately selected according to R¹. While the reaction solvent is not particularly limited, for example, polar solvents such as pyridine and the like, nitriles such as acetonitrile and the like, ethers such as tetrahydrofuran etc., and the like can be mentioned. While the reaction time is not particularly limited, it is, for example, 30 min - 24 hr, preferably 2 - 12 hr, more preferably 2 - 4 hr. While the reaction temperature is not particularly limited, it is, for example, 0 to 100°C, preferably 10 to 60°C, more preferably 20 to 30°C. The concentration of the glycoside compound to be used and the protecting group-introducing agent is not particularly limited, and can be appropriately determined. The substance amount ratio of the aforementioned glycoside compound and the aforementioned protecting group-introducing agent is not particularly limited and may be, for example, a stoichiometric mixture ratio or any other ratio. Also, the amount of other reaction substance to be used is not particularly limited. The number of moles of the aforementioned protecting group-introducing agent is, for example, 1- to 100-fold, preferably 1- to 20-fold, more preferably 1- to 5-fold, relative to that of the aforementioned glycoside compound. The reaction conditions of the introduction reaction of a protecting group R¹ may be appropriately determined by referring to, for example, the conditions of a similar reaction in a known glycoside compound, and the like, or by reference to any of the below-mentioned Examples 2 to 5.

In the introduction reaction of the protecting group R², the protecting group-introducing agent may be appropriately selected according to R². While the reaction solvent is not particularly limited, for example, nitriles such as acetonitrile and the like, ethers such as tetrahydrofuran, halogenated solvents such as dichloromethane etc., and the like can be mentioned. While the reaction time is not particularly limited, it is, for example, 30 min - 24 hr, preferably 1 - 12 hr, more preferably 2 - 12 hr. While the reaction temperature is not particularly limited, it is, for example, 0 to 100°C, preferably 10 to 60°C, more preferably 20 to 40°C. The concentration of the glycoside compound to be used and the protecting group-introducing agent is not particularly limited, and can be appropriately determined. The substance amount ratio of the aforementioned glycoside compound and the aforementioned protecting group-introducing agent is not particularly limited and may be, for example, a stoichiometric mixture ratio or any other ratio. Also, the amount of other reaction substance to be used is not particularly limited. The number of moles of the aforementioned protecting group-introducing agent is, for example, 1- to 20-fold, preferably 1- to 5-fold, more preferably 1- to 2-fold, relative to that of the aforementioned glycoside compound.

A reaction to bind a glycoside compound to a solid phase carrier (e.g., CPG) can be performed, for example, as follows.

Anhydrous succinic acid is reacted with controlled pore glass (CPG) having a long chain alkylamino (LCAA) group in a pyridine solution in the presence of dimethylaminopyridine to give succinylated CPG-LCAA. 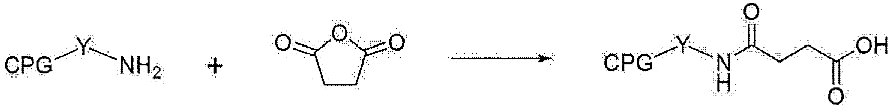 wherein Y is a group represented by the following chemical formula (4): 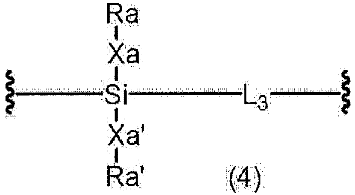 wherein Xa and Xa' are the same or different (preferably same) and each is a single bond or -O-, Ra and Ra' are the same or different (preferably same) and each is alkyl (preferably methyl) or CPG, and L₃ is a linker. As the linker, a divalent hydrocarbon group optionally having an ester bond (-COO-), amide bond (-CONH-), carbonate bond (-OCOO-), ether bond (-O-) and/or thioether bond (-S-) can be mentioned. In the present specification, a divalent hydrocarbon group means a group in which two hydrogen atoms are removing from the carbon - atom of hydrocarbon. Examples of the divalent hydrocarbon group include alkylene, alkenylene, alkynylene, arylene and the like. As "alkylene", linear or branched chain ones having 1 - 8 carbon atoms, for example, methylene, ethylene, 1-methylethylene, 2-methylethylene, trimethylene, tetramethylene, pentamethylene, hexamethylene, heptamethylene and octamethylene can be mentioned. As "alkenylene", linear or branched chain ones having 2 - 8 carbon atoms, for example, ethenylene, 1-propenylene, 2-propenylene, 1-butenylene, 2-butenylene, 3-butenylene, 1-pentenylene, 2-pentenylene, 3-pentenylene and 4-pentenylene can be mentioned. As "alkynylene", those having 2 - 6 carbon atoms, for example, ethynylene, provinylene, butenylene, bentynylene and hexenylene can be mentioned. As "arylene", those having 6 - 14 carbon atoms, for example, phenylene and naphthylene can be mentioned.

The obtained succinylated CPG-LCAA is reacted with a glycoside compound wherein, in the chemical formula (1c), R¹ is a 4,4'-dimethoxy(triphenylmethyl) group and R² is a hydrogen atom in the presence of 1-(3-dimethylaminopropyl)-3-ethylcarbodiimide and dimethylaminopyridine, whereby the object solid phase carrier-bonded glycoside compound can be synthesized. 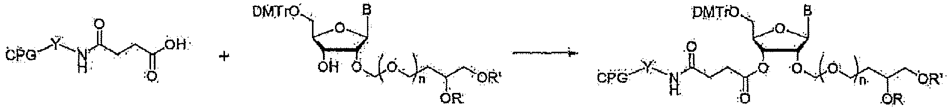 wherein
Y is the same as in the aforementioned chemical formula (4), B, n, R and R' are the same as in the aforementioned chemical formula (1), and DMTr is a 4,4'-dimethoxy(triphenylmethyl) group, and CPG shows Controlled Pore Glass.

As preferable solid phase carrier-bonded glycoside compound, the above-mentioned glycoside compounds (AA5-2-1), (AB5-2-1), (C5-2-1), (G5-2-1), (U5-2-1), (AA5-2-2), (AB5-2-2), (C5-2-2), (G5-2-2) and (U5-2-2) can be mentioned.

Also, in each of the aforementioned reaction steps, the purification method of the reaction product is not particularly limited and the method can be appropriately performed by reference to a known method and the like.

In the production method of the glycoside compound of the present invention, compound (105) is more preferably produced by the aforementioned production method of ether in the present invention. In this way, the glycoside compound of the present invention can be obtained in a still higher yield.

### 5. Production method of nucleic acid

The production method of a nucleic acid of the present invention is, as mentioned above, a production method of a nucleic acid having the structure represented by the following chemical formula (I), and characteristically includes a condensation step for a condensation reaction of the glycoside compound represented by the aforementioned chemical formula (1) and a glycoside compound of the present invention represented by the following chemical formula (2).

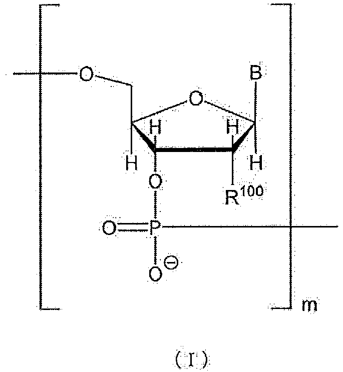

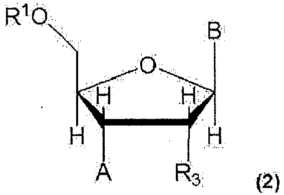

In the aforementioned chemical formula (I), B is as defined for the aforementioned chemical formula (1), (2) or (3),

R¹⁰⁰ is a hydrogen atom or a hydroxyl group, or a group represented by the following chemical formula (R³) or (R⁴): 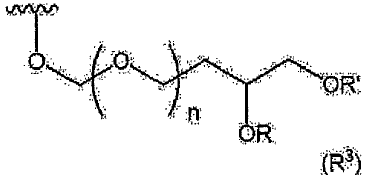 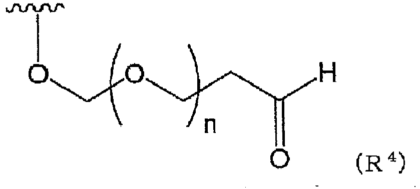 in the aforementioned chemical formula,
n is a positive integer,
R and R' are the same or different and each is a hydrogen atom or a hydroxyl-protecting group,
   each B may be the same or different, and each R¹⁰⁰ may be the same or different, and
   m is a positive integer.

In the chemical formula (I), a nucleic acid containing a structure represented by the following chemical formula (I') wherein R¹⁰⁰ is a group represented by the chemical formula (R³) or (R⁴) is a novel compound:

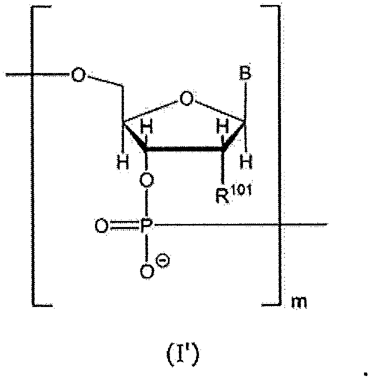

In the aforementioned chemical formula (I'), R¹⁰¹ is a group represented by the following chemical formula (R³) or (R⁴) and each of the other symbols is as defined for the chemical formula (I). 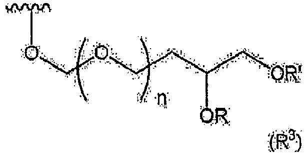 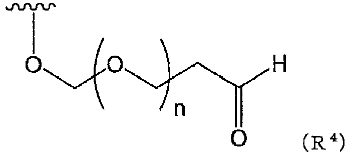 wherein each symbol is as defined for chemical formula (I).

The reaction conditions of the production method of a nucleic acid of the present invention are not particularly limited and, for example, the method can be performed in the same manner as in general phosphoramidite method and the like. For example, the production method of a nucleic acid of the present invention may include production (synthesis) by a general automatic synthesizer of nucleic acid and the like. That is, the glycoside compound represented by the aforementioned chemical formula (2) of the present invention can be used as an amidite for an automatic nucleic acid synthesizer. Using the glycoside compound represented by the aforementioned chemical formula (2) of the present invention, the production method of a nucleic acid of the present invention can produce a nucleic acid with high purity and in a high yield.

In the production method of a nucleic acid of the present invention, for example, the nucleic acid having the structure represented by the aforementioned chemical formula (I) may be a nucleic acid represented by the following chemical formula (II) : 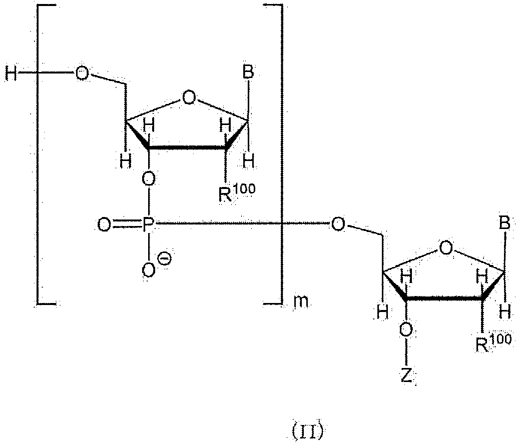 in the aforementioned chemical formula (II),
B, R¹⁰⁰ and m are as defined for the aforementioned chemical formula (I), each B may be the same or different, each R¹⁰⁰ may be the same or different, and
Z is a hydrogen atom or a phosphate group,
   and
the production method may contain the following steps A1 - A6.

### [Step A1]

A step of producing the glycoside compound represented by the following chemical formula (202) (hereinafter to be also simply referred to as glycoside compound (202)) by reacting an acid with the glycoside compound represented by the following chemical formula (201) (hereinafter to be also simply referred to as glycoside compound (201)), and deprotecting the hydroxyl group of the 5' position.

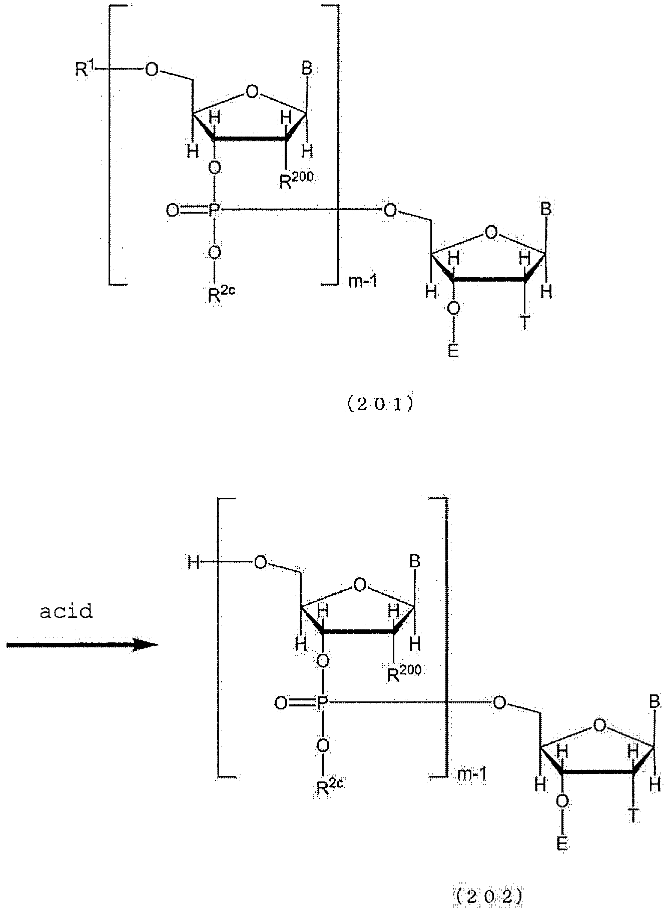

In the aforementioned chemical formulas (201) and (202),
m and B are as defined for the aforementioned chemical formula (II),
R¹ and R^{2c} are as defined for the aforementioned chemical formula (2),
each R²⁰⁰ may be the same or different and each is a hydrogen atom, an acyloxy group or a substituent represented by the following chemical formula (203),
T is a hydrogen atom, an acyloxy group, or a substituent represented by the following chemical formula (203) or (204),
E is an acyl group or a substituent represented by the following chemical formula (204),
at least one of E and T is a substituent represented by the following chemical formula (204), 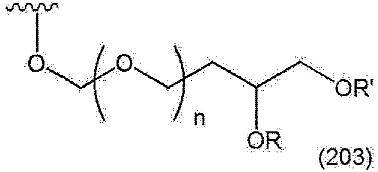 in the aforementioned chemical formula (203),
R, R' and n are as defined for the aforementioned chemical formula (1), 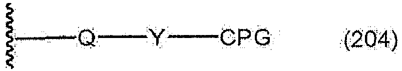 in the aforementioned chemical formula (204),
Y is the same as in the aforementioned chemical formula (4),
Q is a substituent represented by the following chemical formula (205), 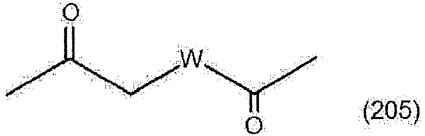
in the aforementioned chemical formula (205),
W is 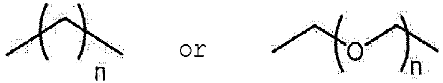 wherein n is as defined for the aforementioned chemical formula (1).

In the aforementioned chemical formulas (201) and (202), examples of the aforementioned acyl group for E include an acetyl group, a propionyl group, a butyryl group, an isobutyryl group, a benzoyl group, a 4-methoxybenzoyl group, a phenylacetyl group, a phenoxyacetyl group, a 4-tert-butylphenoxyacetyl group, a 4-isopropylphenoxyacetyl group and the like. The acyl group in the aforementioned acyloxy group for T and R²⁰⁰ is also the same.

While the aforementioned acid to be used for step A1 is not particularly limited, for example, halogenated carboxylic acid and the like can be mentioned. Examples of the aforementioned halogenated carboxylic acid include trifluoroacetic acid, dichloroacetic acid, trichloroacetic acid and the like. The aforementioned acid may be used, for example, after dissolving in a suitable solvent. While the concentration of the solution is not particularly limited, it is, for example, 1 - 5 wt%. While the aforementioned solvent is not particularly limited, for example, halogenated solvents such as dichloromethane and the like, nitriles such as acetonitrile and the like, water and the like can be mentioned. These may be used alone or plural kinds thereof may be used in combination. While the reaction temperature in step A1 is not particularly limited, 20°C to 50°C is preferable. While the reaction time is not particularly limited and varies depending on the kind of the acid to be used, reaction temperature and the like, it is, for example, 1 min - 1 hr. Also, while the amount of the aforementioned acid to be used (number of moles) is not particularly limited, it is, for example, 1- to 100-fold, preferably 1- to 10-fold, relative to the number of moles of the sugar (or base) in the aforementioned glycoside compound (201).

### [Step A2]

A step of producing the glycoside compound represented by the following chemical formula (206) (hereinafter to be also simply referred to as glycoside compound (206)) by condensing the glycoside compound (202) produced in the aforementioned step A1 with a nucleic acid monomer compound in the presence of an activator.

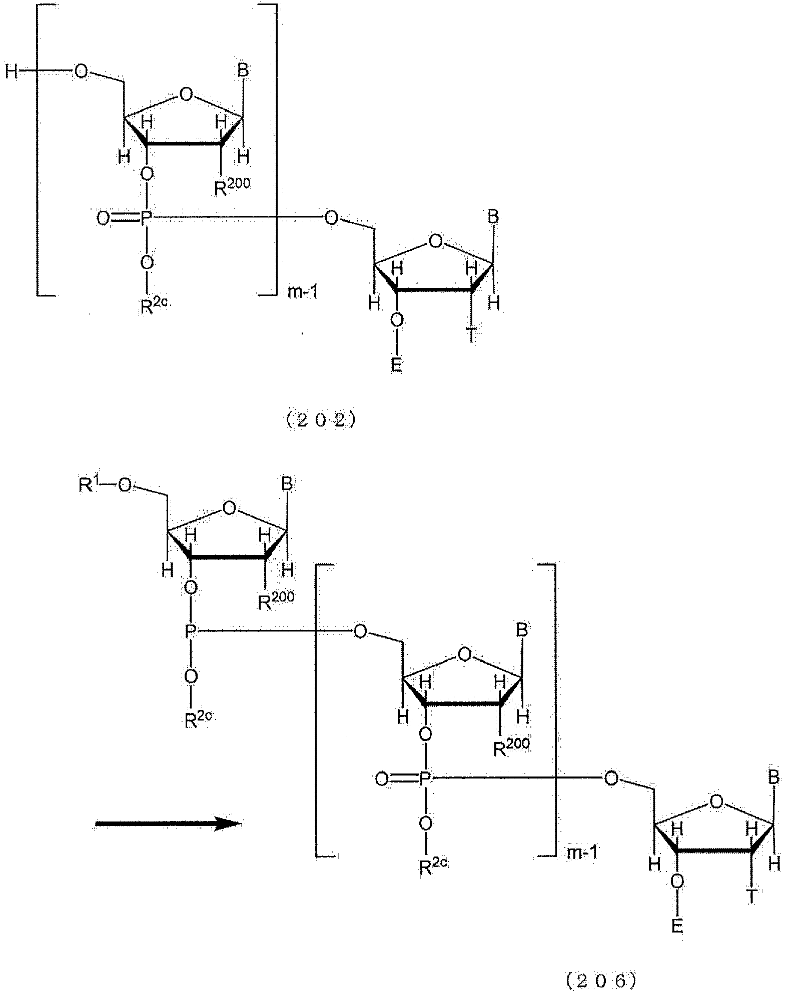

In the aforementioned chemical formula (206),
B, E, m, R¹, R²⁰⁰, T and R^{2c} are as defined for the aforementioned chemical formula (201), each B, each R²⁰⁰ and each R^{2c} may be the same or different.

Examples of the aforementioned "nucleic acid monomer compound" in step A2 include the glycoside compound represented by the aforementioned chemical formula (2) of the present invention. While it is possible to use other glycoside compounds as the aforementioned "nucleic acid monomer compound", the glycoside compound represented by the aforementioned chemical formula (2) is preferably used from the aspects of reaction efficiency, yield of the object resultant product, purity of the object resultant product and the like. Moreover, the glycoside compound represented by the aforementioned chemical formula (2) may be used along with other glycoside compound. Examples of the aforementioned "other glycoside compound" include a glycoside compound of the aforementioned chemical formula (2) wherein R³ is changed to H (hydrogen atom) or OH (hydroxyl group). In the production method of a nucleic acid of the present invention, 1 molecule or more at minimum of the glycoside compound represented by the aforementioned chemical formula (2) of the present invention is used to produce a nucleic acid. As mentioned below, moreover, the condensation reaction may be repeated plural times in Step A2 by repeating steps A1 - A4 appropriate times. In this way, the chain length of the object nucleic acid (glycoside compound (I) or (II)) can be a desired (given) chain length. In the production method of a nucleic acid of the present invention, the glycoside compound represented by the aforementioned chemical formula (2) of the present invention is preferably subjected to plural molecule polymerization (condensation polymerization). In this way, for example, RNA (i.e., nucleic acid of the aforementioned chemical formulas (I) or (II), wherein each R¹⁰⁰ is a hydrogen atom) can be synthesized (produced). Alternatively, DNA (nucleic acid of the aforementioned chemical formulas (I) or (II), wherein each R¹⁰⁰ is a hydroxyl group) can be synthesized by, for example, reverse transcription of RNA synthesized by plural molecule polymerization (condensation polymerization) of the glycoside compound represented by the aforementioned chemical formula (2) of the present invention. The nucleic acid of the aforementioned chemical formulas (I) or (II), which includes R¹⁰⁰ as a hydrogen and R¹⁰⁰ as a hydroxyl group may be synthesized by, for example, a condensation reaction of the glycoside compound represented by the aforementioned chemical formula (2) and the glycoside compound of the aforementioned chemical formula (2) wherein R³ is changed to H (hydrogen atom).

In step A2, the aforementioned activator is not particularly limited and, for example, may be an activator similar to that used for known nucleic acid synthesis. Examples of the aforementioned activator include 1H-tetrazole, 5-ethylthiotetrazole, 4,5-dichloroimidazole, 4,5-dicyanoimidazole, benzotriazole triflate, imidazole triflate, pyridinium triflate, N,N-diisopropylethylamine, 2,4,6-collidine/N-methylimidazole and the like.

In step A2, while the reaction solvent is not particularly limited, for example, nitriles such as acetonitrile and the like, ethers such as tetrahydrofuran, dioxane etc., and the like can be mentioned. These solvents may be used alone or plural kinds thereof may be used in combination. While the reaction temperature is not particularly limited, 20°C to 50°C is preferable. Also, while the reaction time is not particularly limited and varies depending on the kind of the activator to be used, reaction temperature and the like, it is, for example, 1 min - 1 hr. While the amount of the aforementioned nucleic acid monomer compound to be used (number of moles) is not particularly limited, it is, for example, 1- to 100-fold, preferably 1- to 10-fold, relative to the number of moles of the sugar (or base) in the aforementioned glycoside compound (202). The amount of the aforementioned activator to be used is also the same.

### [Step A3]

A step of capping the hydroxyl group at the 5'-position of the aforementioned glycoside compound (202), which was unreacted in the aforementioned step A2.

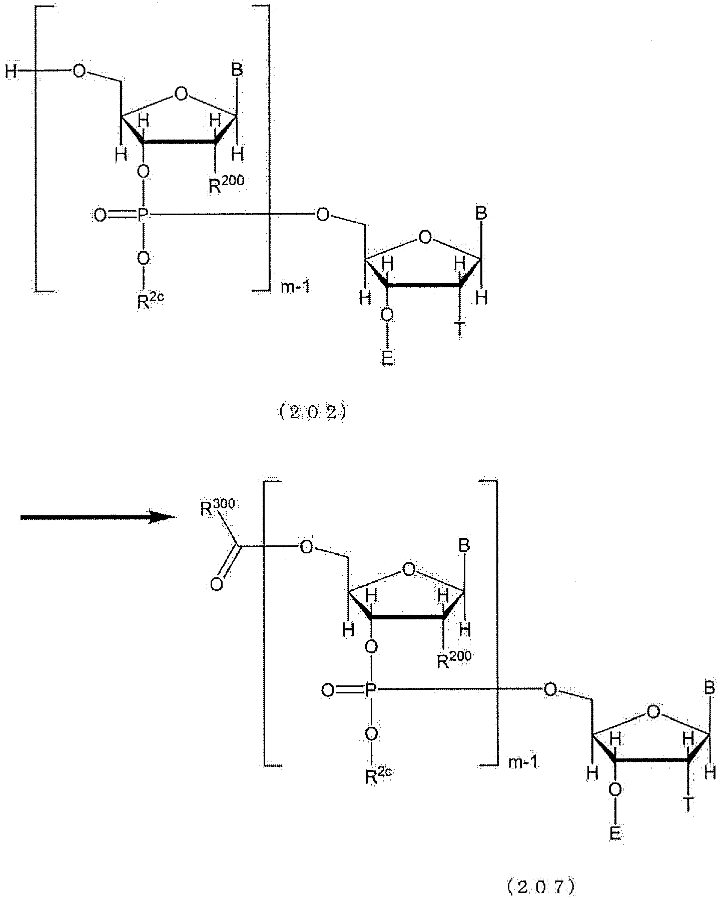

In the aforementioned chemical formula (207),
R³⁰⁰ is a methyl group or a phenoxymethyl group, and
B, E, m, R²⁰⁰, T and R^{2c} are as defined for the aforementioned chemical formula (201).

In step A3, the hydroxyl group at the 5'-position, which was unreacted on completion of the aforementioned step A2, is protected by reacting with a capping agent. While the aforementioned capping agent is not particularly limited, for example, acetic anhydride, phenoxyacetic anhydride and the like can be mentioned. For example, the aforementioned capping agent may be used in the form of a solution. While the solvent of the aforementioned solution is not particularly limited, for example, pyridine, dichloromethane, acetonitrile, tetrahydrofuran, a mixed solvent thereof and the like can be mentioned. While the concentration of the aforementioned solution is not particularly limited, it is, for example, 0.05 - 1 M. In step A3, for example, an appropriate reaction accelerator such as 4-dimethylaminopyridine, N-methylimidazole and the like may also be used in combination. While the reaction temperature is not particularly limited, 20°C to 50°C is preferable. Also, while the reaction time is not particularly limited and varies depending on the kind of the capping agent to be used, reaction temperature and the like, it is, for example, 1 - 30 min. While the amount of the aforementioned capping agent to be used (number of moles) is not particularly limited, it is, for example, 1- to 100-fold, preferably 1- to 10-fold, relative to the number of moles of the sugar (or base) in the aforementioned glycoside compound (202). The amount of the aforementioned activator to be used is also the same. The amount of the aforementioned reaction accelerator to be used is also the same.

### [Step A4]

A step of converting a phosphorous acid group in the aforementioned chemical formula (206) into a phosphate group by reacting the glycoside compound (206) produced in the aforementioned step A2 with an oxidant.

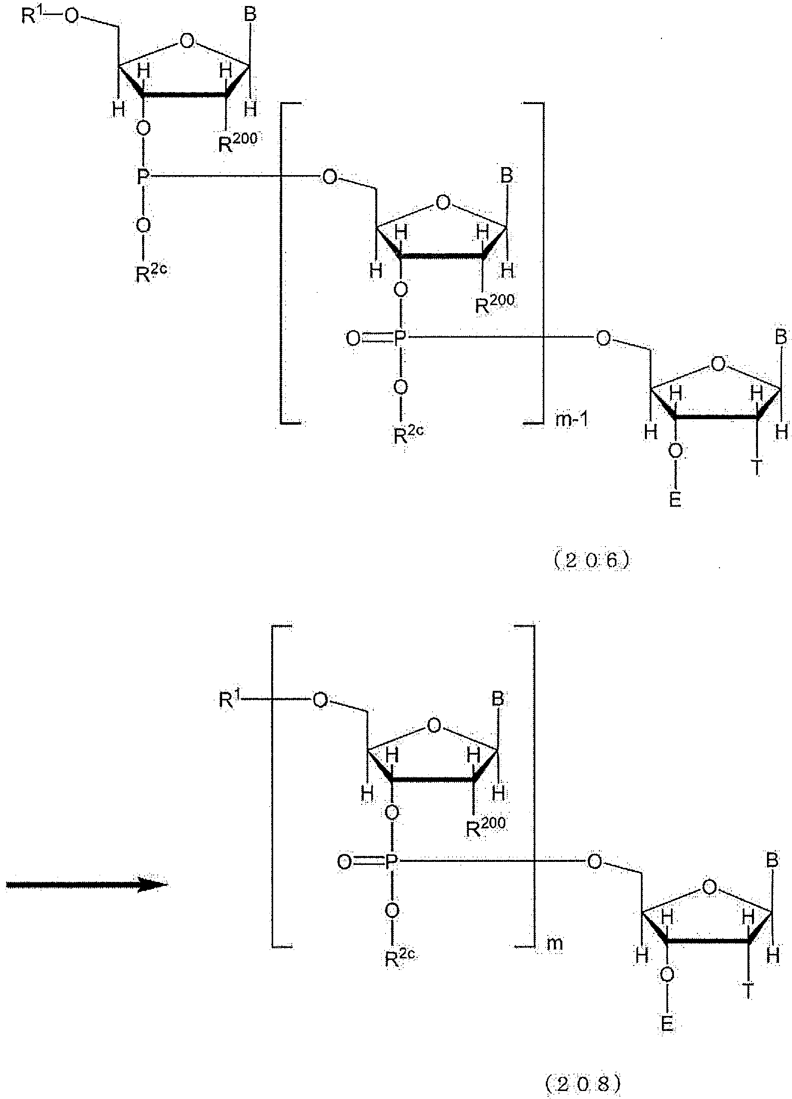

In the aforementioned chemical formula (208),
B, E, m, R¹, R²⁰⁰, T and R^{2c} are as defined for the aforementioned chemical formula (206).

While the aforementioned oxidant in step A4 is not particularly limited, for example, iodine, peroxide (e.g., tert-butyl hydroperoxide) and the like can be mentioned. The aforementioned oxidant may be used in the form of a solution. While the solvent for the aforementioned solution is not particularly limited, for example, pyridine, tetrahydrofuran, water, acetic acid, methylene chloride, a mixed solvent thereof and the like can be mentioned. As the aforementioned solution, a solution obtained by dissolving iodine in a mixed solvent of water, pyridine and tetrahydrofuran, a solution obtained by dissolving iodine in a mixed solvent of pyridine and acetic acid, a solution obtained by dissolving peroxide in methylene chloride, and the like can be used. While the concentration of the aforementioned solvent is not particularly limited, it is, for example, 0.05 - 2 M. While the reaction temperature is not particularly limited, 20°C to 50°C is preferable. Also, while the reaction time is not particularly limited and varies depending on the kind of the oxidant to be used, reaction temperature and the like, it is, for example, 1 - 30 min. While the amount of the aforementioned oxidant to be used (number of moles) is not particularly limited, it is, for example, 1- to 100-fold, preferably 1- to 10-fold, relative to the number of moles of the sugar (or base) in the aforementioned glycoside compound (206) .

After step A4 and before performing the next step A5, the operation may return to step A1. By repeating steps A1 - A4 an appropriate number of times in this way, the chain length of the object nucleic acid (glycoside compound (208)) can become a desired (given) chain length.

### [Step A5]

A step of cleaving the glycoside compound (208) produced in the aforementioned step A4 from the aforementioned solid phase carrier, and deprotecting each nucleic acid base moiety and the hydroxyl group at each 2'-position.

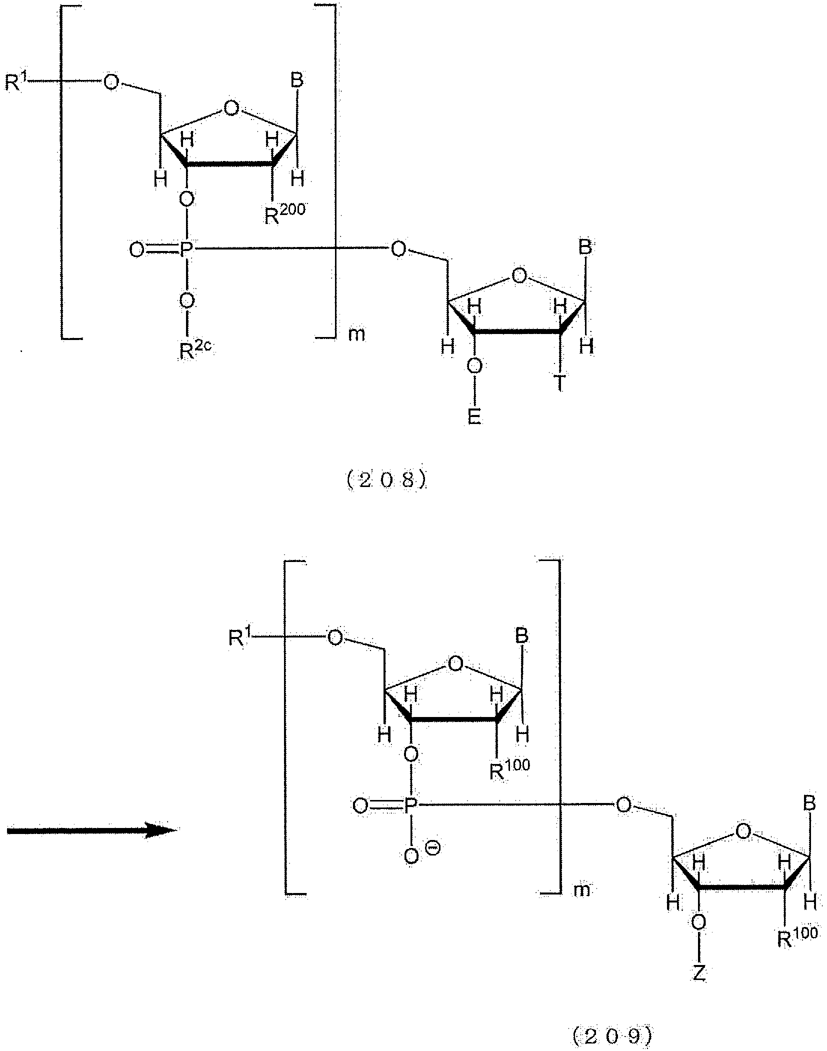

In the aforementioned chemical formula (209),
B, m, R¹ and R²⁰⁰ are as defined for the aforementioned chemical formula (208), and
R¹⁰⁰ and Z are as defined for the aforementioned chemical formula (II).

In step A5, a step for cleaving the aforementioned glycoside compound (208), namely, a nucleic acid with a given chain length, from a solid phase carrier (cleaving step) can be performed by adding a cleaving agent to a solid carrier carrying the aforementioned nucleic acid (208). While the aforementioned cleaving agent is not particularly limited, for example, conc. aqueous ammonia, methylamine and the like can be mentioned. One kind or two or more kinds may be used in combination. The aforementioned cleaving agent may be used by, for example, dissolving in a solvent. While the aforementioned solvent is not particularly limited, for example, water, methanol, ethanol, isopropyl alcohol, acetonitrile, tetrahydrofuran, a mixed solvent thereof and the like can be mentioned, with particular preference given to ethanol. While the concentration of the aforementioned solution is not particularly limited, for example, the concentration of ammonium hydroxide in the aforementioned solution is set to 20 - 30 wt%. The concentration of the aforementioned ammonium hydroxide is preferably 25 - 30 wt%, more preferably 28 - 30 wt%. While the amount of the aforementioned cleaving agent to be used (number of moles) is not particularly limited, it is, for example, 1- to 100-fold, preferably 1- to 10-fold, relative to the number of moles of the sugar (or base) in the aforementioned glycoside compound (208). While the reaction temperature of the aforementioned cleaving step is not particularly limited, it is, for example, 20°C to 90°C. While the reaction time is not particularly limited and varies depending on the kind of the cleaving agent to be used, reaction temperature and the like, it is, for example, 1 - 24 hr, preferably 3 - 12 hr.

The reaction of the ammonia treatment step when R¹⁰⁰ is the chemical formula (R³) is shown below. 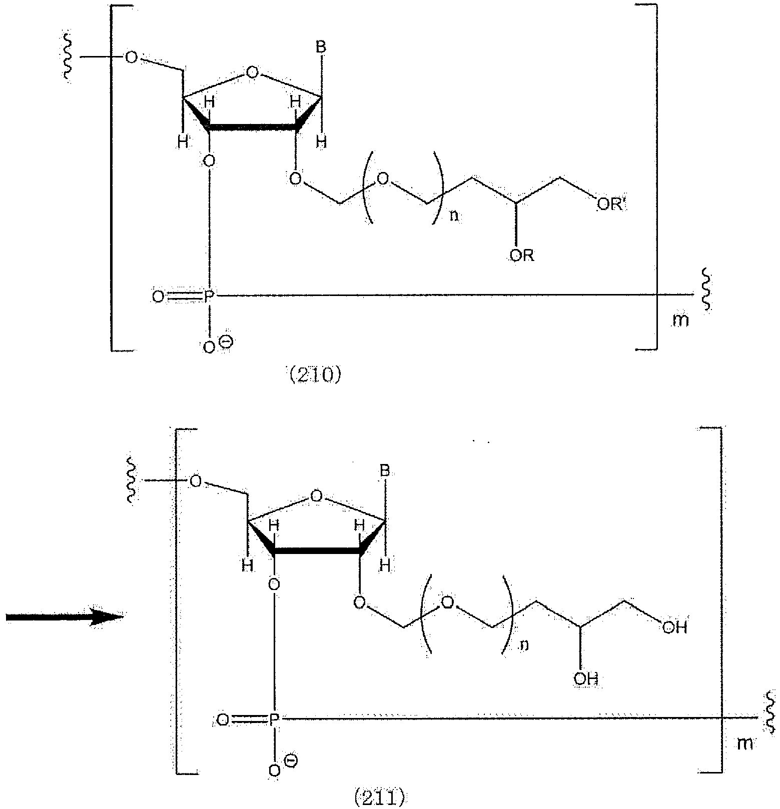 wherein at least one of R and R' is not a hydrogen atom and each of other symbols is as defined above.

In the deprotection step of the aforementioned 2'-position hydroxyl group in step A5, using an oxidant under weak acidity, the 1,2-diol group is oxidized and converted to a formyl group to obtain an aldehyde derivative. The aforementioned oxidant is not particularly limited and, for example, sodium periodate and potassium periodate can be mentioned, and sodium periodate is particularly preferable. A weak acidic reaction solution is preferable, and pH3 - pH5 is more preferable. While the buffer to be used is not particularly limited, for example, 0.4 M acetate buffer with pH 4.2 is used. The reaction temperature of the aforementioned oxidation step is not particularly limited, and it is, for example, 10°C to 60°C, preferably 20°C to 50°C. While the reaction time is not particularly limited and varies depending on the kind of the oxidant, reaction temperature and the like, it is, for example, 10 min - 3 hr, preferably 30 min - 1 hr. After completion of the reaction, glycerol or ethylene glycol is added to discontinue the reaction, and the object resultant product is separated and purified from the reaction solution. The method for separating and purifying is not particularly limited, and a conventional purification method can be used. Examples of the aforementioned purification method include filtration, elution, concentration, neutralization, centrifugation, chromatography (silica gel column, thin layer, reversed-phase ODS, ion exchange, gel filtration), dialysis, ultrafiltration and the like. These may be used alone or plural kinds thereof may be used in combination.

The reaction of the step, particularly the periodic acid treatment step, is shown below. 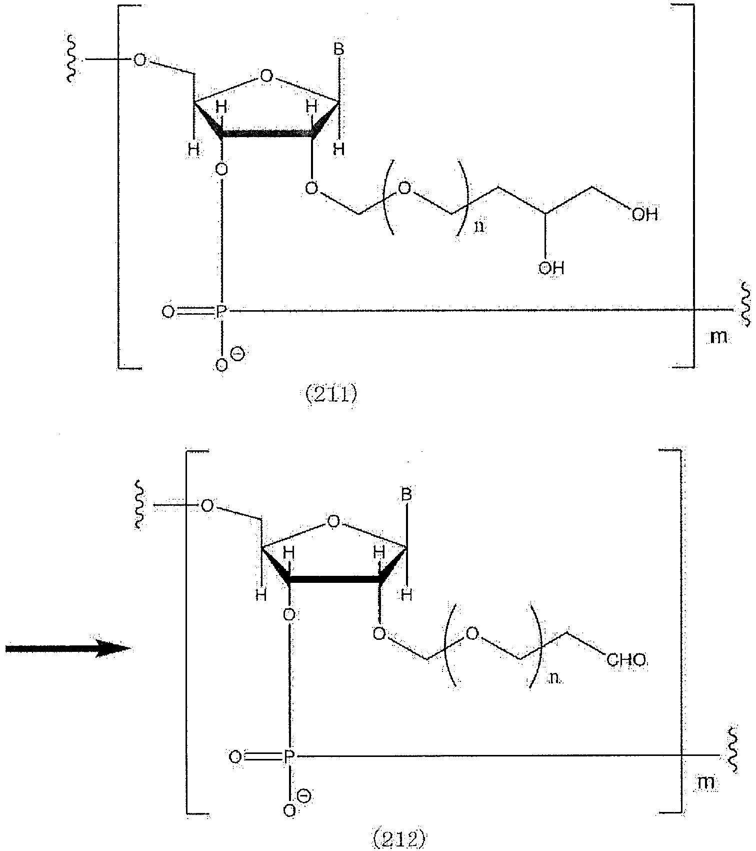 wherein each symbol is as defined above.

The deprotecting agent to be successively used is not particularly limited as long as acrolein is produced from the 2'-position β-oxyaldehyde moiety by β elimination, as a result of which the 2'-protecting group is removed and the 2'-position is converted to a 2'-hydroxyl group.

For example, tetraalkylammoniumhalide can be mentioned. More specifically, for example, tetrabutylammoniumfluoride (TBAF) can be mentioned. While the solvent to be used for the aforementioned deprotection step is not particularly limited, for example, tetrahydrofuran, N-methylpyrrolidone, N,N-dimethylformamide, dimethyl sulfoxide, a mixed solvent thereof and the like can be mentioned. In addition, the byproducts such as acrolein and the like to be developed in the aforementioned deprotection step may be trapped with, for example, alkylamine, thiol, nitromethane or a mixture thereof. Examples of the aforementioned alkylamine include alkylamine having a linear alkyl group having 1 - 10 carbon atoms. Examples of the aforementioned thiol include alkylthiol having a linear alkyl group having 1 - 10 carbon atoms. While the reaction time and reaction temperature of the aforementioned deprotection step are not particularly limited, 30 min - 50 hr, preferably 40 - 10 hr, and 10 - 70°C, preferably 20 - 50°C, are preferable. The amount of the aforementioned deprotecting agent to be used is, for example, 10- to 1000-fold, preferably 50- to 200-fold, relative to the number of moles of the sugar (or base) in the aforementioned glycoside compound (202). The amount of the aforementioned trapping agents to be used is also the same. In addition, the method for separating and purifying the glycoside compound (209), which is the object product, from the reaction mixture of the aforementioned deprotection step is not particularly limited, and a conventional purification method can be used. Examples of the aforementioned purification method include filtration, elution, concentration, neutralization, centrifugation, chromatography (silica gel column, thin layer, reversed-phase ODS, ion exchange, gel filtration), dialysis, ultrafiltration and the like. These may be used alone or plural kinds thereof may be used in combination.

The reaction of the step, particularly the tetraalkylammonium halide treatment step, is shown below. 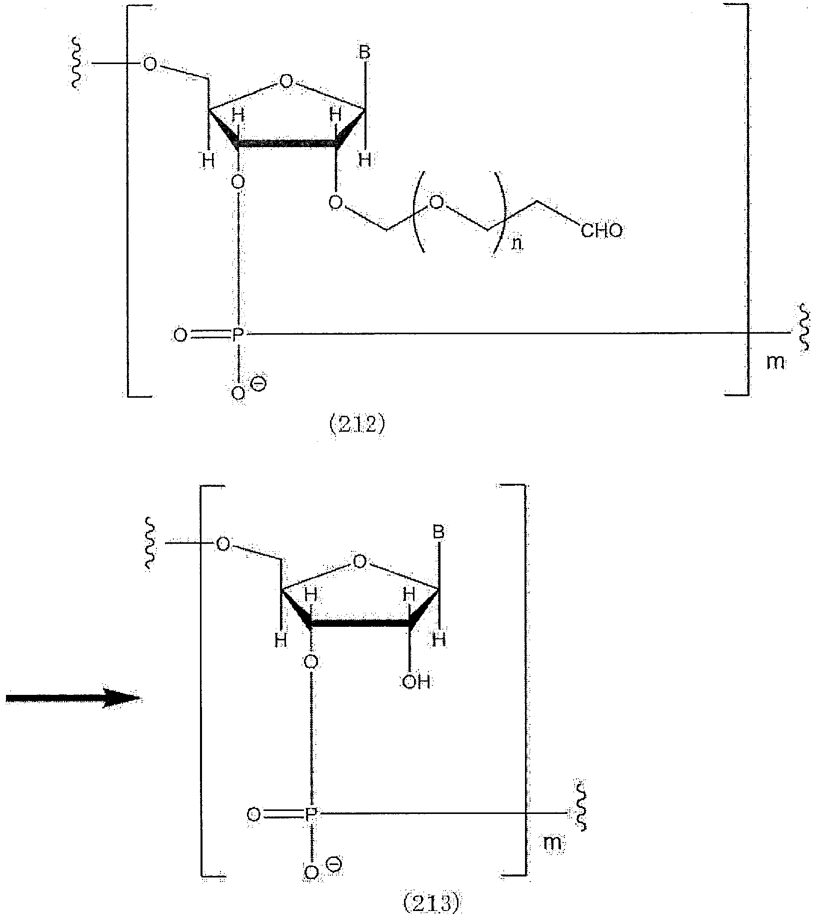 wherein each symbol is as defined above.

In the deprotection step of the 2'-position hydroxyl group in the aforementioned step A5, a step of protecting the 2'-position formyl group of the aldehyde derivative obtained in the oxidation step can be performed between a step of obtaining an aldehyde derivative by an oxidation reaction using an oxidant (e.g., sodium periodate and potassium periodate, preferably sodium periodate) and a deprotection reaction with a deprotecting agent (e.g., tetraalkylammoniumhalide, preferably TBAF) (step A5').

In the protection step of the aforementioned 2'-position formyl group in step A5', an amine compound (benzylamine having an electron-withdrawing group at ortho and para-positions (e.g., orthonitrobenzylamine)) is reacted with the aldehyde derivative obtained by the periodization reaction (periodic acid treatment) in step A4 to give an imino derivative. The solvent to be used in the aforementioned protection step is not particularly limited and, for example, tetrahydrofuran, N-methylpyrrolidone, N,N-dimethylformamide, dimethyl sulfoxide and a mixed solvent thereof and the like can be mentioned. The reaction temperature of the aforementioned protection step is not particularly limited and is, for example, 10°C to 60°C, preferably about room temperature. The reaction time is not particularly limited and varies depending on the kind of the amine compound to be used, reaction temperature and the like, and is, for example, 1 - 10 hr, preferably a few hours.

The imino compound is subjected to the next step A6.

### [step A6]

A step of removing the hydroxyl-protecting group at the 5'-position of the compound (209) produced in the aforementioned step A5 (or step A5').

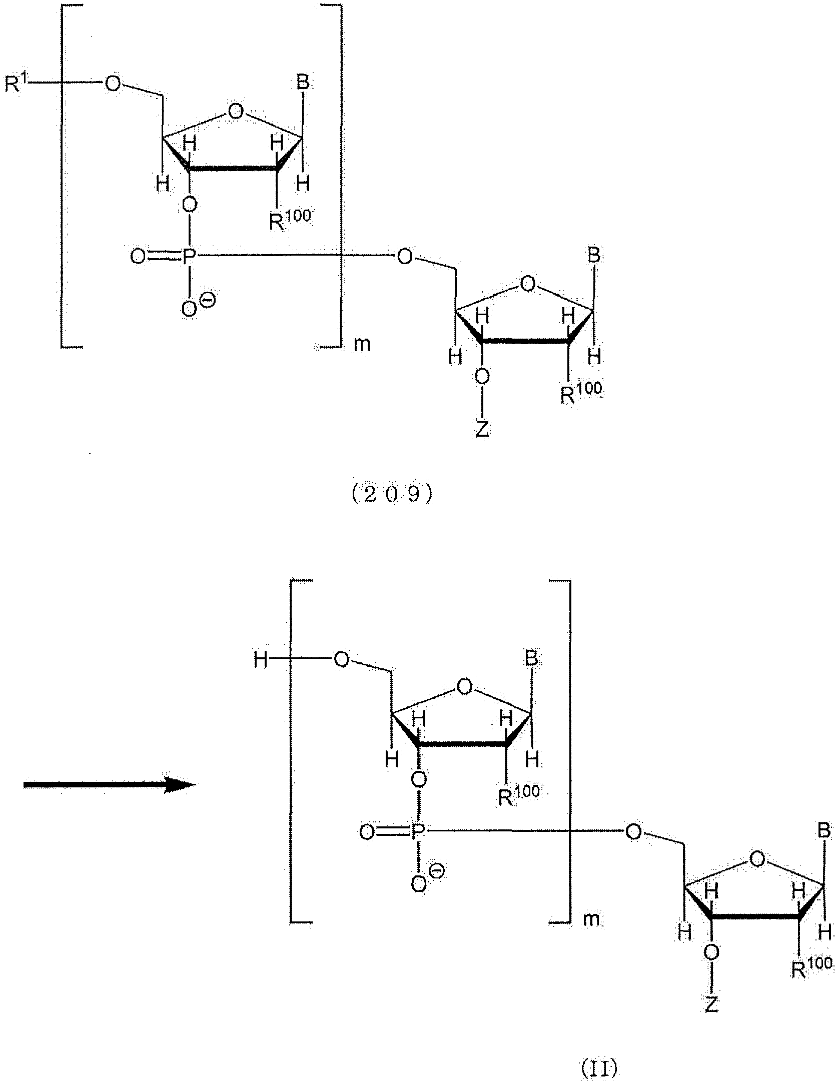

While the aforementioned acid to be used for step A6 is not particularly limited, for example, halogenated carboxylic acid, carboxylic acid and the like can be mentioned. Examples of the aforementioned halogenated carboxylic acid or carboxylic acid include trifluoroacetic acid, dichloroacetic acid, trichloroacetic acid, acetic acid and the like. The aforementioned acid may be used, for example, after dissolving in a suitable solvent. While the concentration of the solution is not particularly limited, it is, for example, 10 - 70 wt%. While the aforementioned solvent is not particularly limited, for example, dichloromethane, acetonitrile, chloroform, ethanol, water, buffer having pH 2 - 5, a mixed solvent thereof and the like can be mentioned. Examples of the aforementioned buffer include acetate buffer. While the reaction temperature in step A6 is not particularly limited, 10°C to 60°C is preferable. While the reaction time is not particularly limited and varies depending on the kind of the acid to be used, reaction temperature and the like, it is, for example, 1 min - 30 min. While the amount of the aforementioned acid to be used (number of moles) is not particularly limited, it is, for example, 1- to 200-fold, preferably 1- to 20-fold, relative to the number of moles of the sugar (or base) in the aforementioned glycoside compound (209) .

,

The deprotecting agent to be successively used is not particularly limited as long as the 2'-position can be converted to a 2'-hydroxyl group from the imino derivative moiety of (II) by β elimination.

For example, tetraalkylammoniumhalide can be mentioned. More specifically, for example, tetrabutylammoniumfluoride (TBAF) can be mentioned. While the solvent to be used for the aforementioned deprotection step is not particularly limited, for example, tetrahydrofuran, N-methylpyrrolidone, N,N-dimethylformamide, dimethyl sulfoxide, a mixed solvent thereof and the like can be mentioned. In addition, the byproducts such as acrolein and the like to be developed in the aforementioned deprotection step may be trapped with, for example, alkylamine, thiol, nitromethane or a mixture thereof. Examples of the aforementioned alkylamine include alkylamine having a linear alkyl group having 1 - 10 carbon atoms. Examples of the aforementioned thiol include alkylthiol having a linear alkyl group having 1 - 10 carbon atoms. While the reaction time and reaction temperature of the aforementioned deprotection step are not particularly limited, 30 min - 50 hr, preferably 40 - 10 hr, and 10 - 70°C, preferably 20 - 50°C, are preferable. The amount of the aforementioned deprotecting agent to be used is, for example, 10- to 1000-fold, preferably 50- to 200-fold, relative to the number of moles of the sugar (or base) in the aforementioned glycoside compound (202). The amount of the aforementioned trapping agents to be used is also the same. In addition, the method for separating and purifying the glycoside compound (209), which is the object product, from the reaction mixture of the aforementioned deprotection step is not particularly limited, and a conventional purification method can be used. Examples of the aforementioned purification method include filtration, elution, concentration, neutralization, centrifugation, chromatography (silica gel column, thin layer, reversed-phase ODS, ion exchange, gel filtration), dialysis, ultrafiltration and the like. These may be used alone or plural kinds thereof may be used in combination.

The aforementioned compound (II), which is the object product of the aforementioned step A6, may be separated and purified as necessary. The "separation" includes, for example, isolation. The separation and purification method is not particularly limited and, for example, extraction, concentration, neutralization, filtration, centrifugation, reversed-phase column chromatography, ion exchange column chromatography, gel filtration column chromatography, high performance liquid chromatography, dialysis, ultrafiltration and the like can be mentioned, which may be used alone or plural kinds thereof may be used in combination.

The order of step A5 (or step A5') and the aforementioned step A6 may be reversed. That is, the aforementioned step A6 may be performed after the aforementioned step A4 and before the aforementioned step A5 (or step A5'), after which the aforementioned step A5 may be performed.

Use of the nucleic acid produced by the production method of a nucleic acid of the present invention is not particularly limited and, for example, it is similar to that of known nucleic acids. Since the aforementioned nucleic acid can be produced at a low cost and with high purity when produced by the production method of a nucleic acid of the present invention, use thereof is broad and, for example, it is suitable for use in the production of a medicament and the like.

While the present invention is explained in detail in the following by referring to Examples and the like, the present invention is not limited by them.

### [Examples]

### [Example 1: Synthesis of DBM-forming reagent]

According to the following Scheme, a DBM-forming reagent was synthesized. "DBM" is an abbreviation of "3,4-diacetoxybutoxymethyl" (hereinafter the same).

The following reagents were used.
1,2,4-butanetriol (>97.0%, reagent 1st grade, Tokyo Chemical Industry Co., Ltd.)
acetone (99.5+%, reagent special grade, Wako Pure Chemical Industries, Ltd.)
p-toluenesulfonic acid monohydrate (99.0+%, reagent special grade, Wako Pure Chemical Industries, Ltd.)
acetic acid (99.7+%, reagent special grade, Wako Pure Chemical Industries, Ltd.)
acetic anhydride (97.0+%, reagent special grade, Wako Pure Chemical Industries, Ltd.)
dimethyl sulfoxide (99.0+%, dehydrated, Wako Pure Chemical Industries, Ltd.)
pyridine (dehydrated) (99.5+%, for organic synthesis, Wako Pure Chemical Industries, Ltd.)

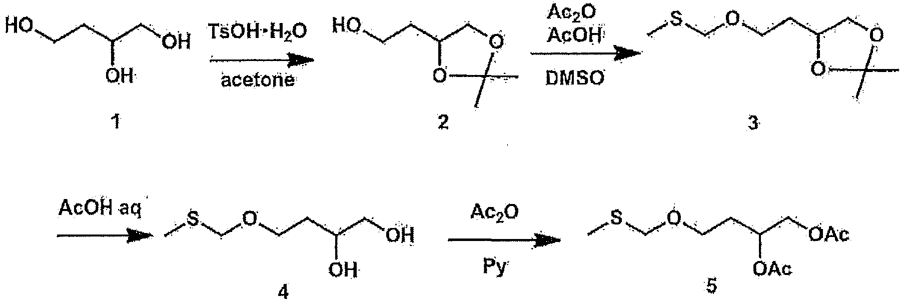

### Synthesis of compound 2

Compound 1 (30 g; 283 mmol) was added to acetone (400 mL) and the mixture was stirred. p-Toluenesulfonic acid monohydrate (1.4 g; 0.025 eq.) was added, and the mixture was stirred at room temperature overnight. Triethylamine (5 mL) was added and the mixture was stirred and confirmed with pH test paper to be pH 8 or more. The mixture was concentrate under reduced pressure with an evaporator to give a crude product. The mixture was evaporated under reduced pressure at 5.0 mmHg to give the object compound 2 (35 g, yield 85%, boiling point 98 - 99°C) as main distillate.
¹H-NMR (500 MHz, CDCl₃) δ: 4.29-4.25 (m, 1H), 4.10 (m, 1H), 3.80 (s, 2H), 3.59 (t, J= 8.0 Hz, 1H), 2.42 (s, 1H), 1.92-1.81 (m, 2H), 1.43 (s, 3H), 1.37 (s, 3H).

### Synthesis of compound 3

Compound 2 (15 g, 103 mmol) was dissolved in dimethyl sulfoxide (110 mL), acetic anhydride (150 mL) and acetic acid (60 mL) were added and the mixture was stirred at 40°C for 8 hr. The disappearance of compound 2 was confirmed by TLC, the reaction solution was added to a saturated aqueous sodium hydrogen carbonate solution (1.6 L), and the mixture was stirred for 1 hr. The mixture was extracted with ethyl acetate, washed with water and saturated brine, and dried over sodium sulfate. The mixture was concentrated under reduced pressure with an evaporator to give the object compound 3 as a crude product (39 g).
¹H-NMR (500 MHz, CDCl₃) δ: 4.62 (s, 2H) 4.22-4.17 (m, 1H), 4.07 (dd, J = 8.0, 6.1 Hz, 1H), 3.67-3.60 (m, 2H), 3.57 (t, J= 8.0 Hz, 1H), 2.15 (s,3H), 1.96-1.89 (m, 1H), 1.86-1.79 (m, 1H), 1.41 (s, 3H), 1.36 (s, 3H).

### Synthesis of compound 4

The crude product (39 g) of compound 3 was added to an 80% acetic acid aqueous solution (150 mL), and the mixture was stirred at room temperature for 3 hr. The solvent was evaporated with a vacuum pump and further azeotropically distilled with pyridine, whereby a crude product (22 g) of the object compound 4 was obtained.

### Synthesis of compound 5

The crude product (22 g) of compound 4 was dissolved in pyridine (150 mL). Acetic anhydride (100 mL) was added, and the mixture was stirred at room temperature for 5 hr. The disappearance of compound 4 was confirmed by TLC, the reaction solution was added to a saturated aqueous sodium hydrogen carbonate solution (500 mL) at 0°C, and the mixture was stirred for 30 min. The mixture was extracted with ethyl acetate, washed with saturated aqueous sodium hydrogen carbonate solution, water and saturated brine, and dried over sodium -sulfate. The mixture was concentrate under reduced pressure and the obtained crude product was purified by silica gel column chromatography (hexane/ethyl acetate=4/1) to give the object compound 5 (13 g, yield 50%/3 steps).
¹H-NMR (500 MHz, CDCl₃) δ: 5.24-5.19 (m, 1H), 4.60 (dd, J = 13.4, 11.5 Hz, 2H), 4.29 (dd, J = 12.0, 3.2 Hz, 1H), 4.10-4.07 (m, 1H), 3.57 (t, J= 8.0 Hz, 2H), 2.14 (s, 3H), 2.06(d, 6Hz, 6H), 1.89 (q, J = 6.2 Hz, 2H).

According to the following Scheme, compound 2 was synthesized.

The following reagents were used.
3-buten-1-ol (>98.0%, reagent special grade, Tokyo Chemical Industry Co., Ltd.)
N,N-dimethylformamide (super dehydrated) (99.5+%, for organic synthesis, Wako Pure Chemical Industries, Ltd.)
imidazole (98.0+%, reagent special grade, Wako Pure Chemical Industries, Ltd.)
triisopropylsilyl chloride (97.0+%, for organic synthesis, Wako Pure Chemical Industries, Ltd.)
acetone (super dehydrated) (99.5+%, for organic synthesis, Wako Pure Chemical Industries, Ltd.)
acetonitrile (99.8+%, Wako Pure Chemical Industries, Ltd.) 4-methylmorpholine N-oxide (>98.0%, Tokyo Chemical Industry Co., Ltd.)
microencapsulated osmium tetraoxide (10%, for organic synthesis, Wako Pure Chemical Industries, Ltd.)
2,2-dimethoxypropane (95.0%, reagent primary, Wako Pure Chemical Industries, Ltd.)
pyridinium p-toluenesulfonate (>98.0%, reagent 1st grade, Tokyo Chemical Industry Co., Ltd.)
tetrabutylammonium fluoride trihydrate (98.0+%, SynQuest Laboratories)
tetrahydrofuran (super dehydrated) (99.5+%, for organic synthesis, Wako Pure Chemical Industries; Ltd.)

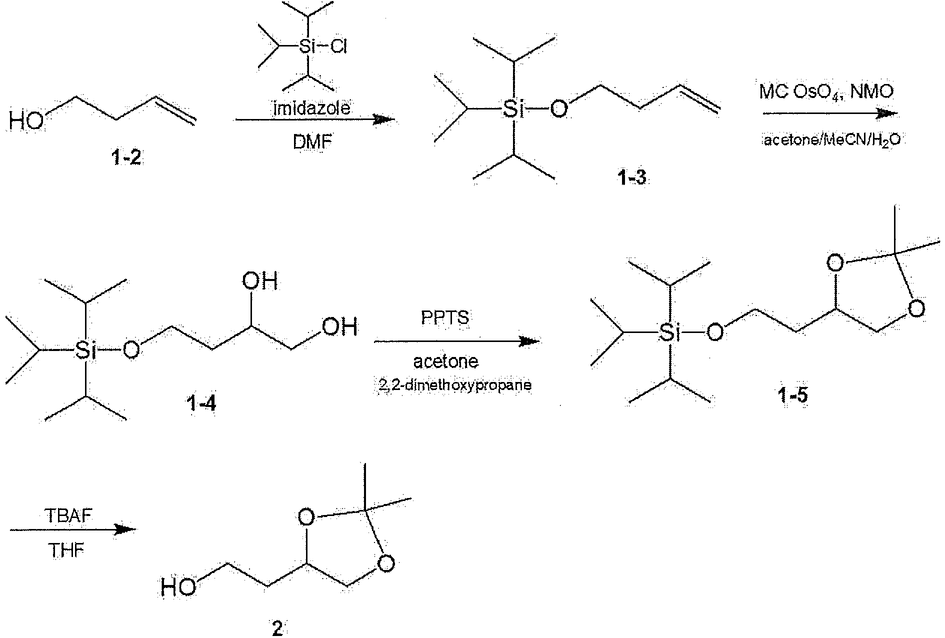

### Synthesis of compound 1-3

Compound 1-2 (7.2 g, 0.1 mol) was dissolved in N,N-dimethylformamide (50 mL), imidazole (13.6 g, 2.0eq.) and triisopropylsilylchloride (23.1 g, 1.2eq.) were added, and the mixture was stirred at room temperature overnight. The mixture was extracted with dichloromethane, the organic layer was washed with water and saturated brine and dried over sodium sulfate. The mixture was concentrated under reduced pressure, and the impurity was removed by silica gel to give the object compound 1-3 as a crude product (29.7 g).
¹H-NMR (500 MHz, CDCl₃) δ: 5.90-5.81 (m, 1H), 5.10-5.01 (m, 2H), 3.73 (t, J=6.8 Hz, 2H), 2.33-2.29 (m, 2H), 1.13-1.02 (m, 21H).

### Synthesis of compound 1-4

The crude product (29.7 g) of compound 1-3 was dissolved in a mixed solvent (120 mL) of acetone/acetonitrile/water (1:1:1). 4-Methylmorpholine N-oxide (15.2 g, 1.3eq.) and microencapsulated osmium tetraoxide (1 g, 0.004eq.) were added, and the mixture was stirred at room temperature overnight. After completion of the reaction, microencapsulated osmium tetraoxide was taken out from the reaction system, water was added, and the mixture was extracted with dichloromethane. The organic layer was washed with water and saturated brine and dried over sodium sulfate. The mixture was concentrated under reduced pressure, and the impurity was removed by silica gel to give the object compound 1-4 as a crude product (31.3 g).

### Synthesis of compound 1-5

The crude product (31.3 g) of compound 1-4 was dissolved in acetone (75 mL). Thereto were added 2,2-dimethoxypropane (7.5 mL) and pyridinium p-toluenesulfonate (0.50 g, 0.02eq.) were added, and the mixture was stirred at room temperature overnight. After completion of the reaction, the solvent was evaporated under reduced pressure, and saturated aqueous sodium hydrogen carbonate solution was added. The mixture was extracted with dichloromethane, and the organic layer was washed with water and saturated brine, and dried over sodium sulfate. The mixture was concentrated under reduced pressure to give the object compound 1-5 as a crude product (51.8 g). ¹H-NMR (500 MHz, CDCl₃) δ: 4.24-4.21 (m, 1H), 4.11-4.08 (m, 1H), 3.83-3.75 (m, 2H), 3.59 (t, J=7.8 Hz, 1H), 1.90-1.85 (m, 1H), 1.81-1.75 (m, 1H), 1.40 (s, 3H), 1.36 (s, 3H), 1.13-1.03 (m, 21H).

### Synthesis of compound 2

To the crude product (51.8 g) of compound 1-5 was added tetrabutylammoniumfluoride trihydrate (30 g)/tetrahydrofuran (130 mL), and the mixture was stirred at room temperature overnight. The solvent was evaporated under reduced pressure, water was added, and the mixture was extracted with ethyl acetate. The organic layer was dried over sodium sulfate. To the aqueous layer (350 mL) was added saturated aqueous sodium chloride solution (110 mL), and the mixture was further extracted with dichloromethane, and the dichloromethane layer was dried over sodium sulfate. The dried organic layers were combined and concentrated under reduced pressure. The obtained crude product was purified by silica gel column chromatography (hexane/ethyl acetate=8/1 - 1/1) to give the object compound 2 (13.4 g).
¹H-NMR (500 MHz, CDCl₃) δ: 4.31-4.25 (m, 1H), 4.11-4.08 (m, 1H), 3.83-3.80 (m, 2H), 3.60 (t, J=7.8 Hz, 1H), 2.25 (brs, 1H), 1.85-1.81 (m, 2H), 1.43 (s, 3H), 1.37 (s, 3H).

Various DBM amidites were synthesized in Examples 2 - 6.

The following reagents were used.
acetone (99.0%, Wako Pure Chemical Industries, Ltd.) acetonitrile (super dehydrated) (99.0%, Wako Pure Chemical Industries, Ltd.)
ammonium fluoride (97.0%, Wako Pure Chemical Industries, Ltd.) 2-cyanoethyl N,N,N',N'-tetraisopropylphosphorodiamidite (97.0%, Wako Pure Chemical Industries, Ltd.), (98.9%, ChemGenes Corporation)
dichloromethane (99.0%, Wako Pure Chemical Industries, Ltd.) diisopropylammoniumtetrazolide (prepared by Current Protocols in Nucleic Acid Chemistry, unit 2.15.16)
diisopropyl ether (EP 98.0%, Wako Pure Chemical Industries, Ltd.)
4,4'-dimethoxytrityl chloride (DMTr-Cl, 95.0%, Sigma-Aldrich) ethyl acetate (99.5%, Wako Pure Chemical Industries, Ltd.) hexane (95.0%, Wako Pure Chemical Industries, Ltd.)
iodine (GR 99.8%, Kanto Chemical Industry Co., Ltd.) N-iodosuccinimide (EP 98.0%, Tokyo Chemical Industry Co., Ltd.)
methanesulfonic acid (GR 98.0%, Wako Pure Chemical Industries, Ltd.)
methanol (99.8%, Wako Pure Chemical Industries, Ltd.) molecular sieves 4A 1/16 (Wako Pure Chemical Industries, Ltd.) dehydrated pyridine (99.5%, Wako Pure Chemical Industries, Ltd.)
silica gel (Wakogel C-200, Wako Pure Chemical Industries, Ltd.)
tetrahydrofuran (super dehydrated, not containing stabilizer) (99.5%, Wako Pure Chemical Industries, Ltd.)
toluene (super dehydrated) (99.5%, Wako Pure Chemical Industries, Ltd.)
triethylamine (GR99.0%, Wako Pure Chemical Industries, Ltd.) triethylamine trihydrofluoride (98.0%, Sigma-Aldrich)
trifluoromethanesulfonic acid (98.0%, Tokyo Chemical Industry Co., Ltd.)

### [Example 2: Synthesis of uridine DBM amidite (U5)]

Uridine DBM amidite (U5) was synthesized according to the following Scheme.

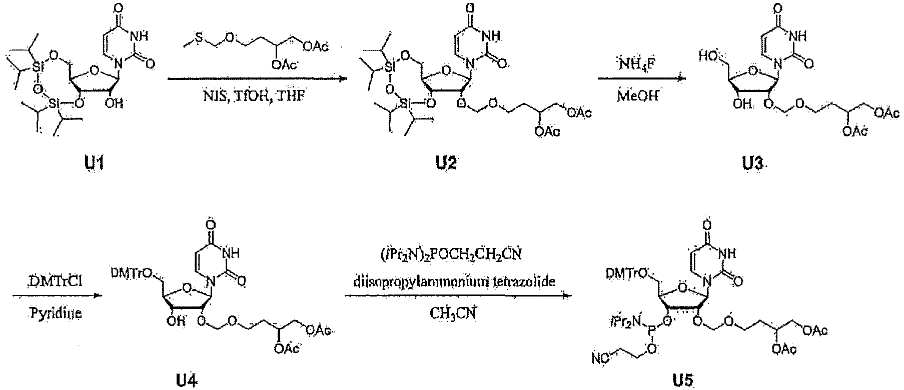

### (1) Synthesis of 3',5'-O-(tetraisopropyldisiloxane-1,3-diyl)-2'-O-(3,4-diacetoxybutoxymethyl)uridine(U2)

Compound U1 (5.0 g, 10.0 mmol) was azeotropically distilled twice with a mixed solvent of toluene and THF and once with THF, and dried in vacuo for 1 hr. This was dissolved in dehydrated THF (30 mL) under an argon atmosphere, molecular sieves 4A (5.0 g) and N-iodosuccinimide (3.5 g, 1.5eq.) were added and the mixture was stirred. The mixture was cooled to - 45°C. After stirring for 5 min, trifluoromethanesulfonic acid (1.4 mL, 1.5eq.) was added dropwise. After stirring for 5 min, a DBM-forming reagent (3.9 g, 1.5eq.) was added and the mixture was stirred at -45°C for 30 min. After completion of the reaction, a cooled mixed solution of a saturated aqueous sodium thiosulfate solution and a saturated aqueous sodium hydrogen carbonate solution were added to the reaction solution, and the mixture was vigorously stirred at room temperature until the brown color disappeared. The mixture was extracted with ethyl acetate, and the organic layer was washed once with a saturated aqueous sodium thiosulfate solution, once with a saturated aqueous sodium hydrogen carbonate solution, and once with a saturated aqueous sodium chloride solution. The washed organic layer was dried over sodium sulfate, and concentrated under reduced pressure to give the object compound U2 as a crude product (9.5 g, purity 94.8%). HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 80% isocratic; Flow rate, 1 mL/min; Temperature, 35°C; Detection, UV 260 nm.

### (2) Synthesis of 2'-O-(3,4-diacetoxybutoxymethyl)uridine (U3)

To a solution of compound U2 (9.5 g, 13.8 mmol) in dehydrated methanol (36 mL) was added ammonium fluoride (1.5 g, 3.0eq.) under an argon atmosphere and the mixture was stirred at 50°C for 4 hr. After completion of the reaction, the solvent was evaporated under reduced pressure. To the residue was added acetonitrile and the resulting precipitate was filtered off. The filtrate was evaporated under reduced pressure. The viscous residue was triturated with diisopropyl ether and the solvent was removed by decantation. This operation (trituration and decantation) was performed 3 times. After drying in vacuo, the object compound U3 was obtained as a crude product (8.5 g, purity 77.1%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; B conc. 0-10%/15 min, 10-100%/20 min; Flow rate, 1 mL/min;
Temperature, 35°C; Detection, UV 260 nm.

### (3) Synthesis of 5'-O-(4,4'-dimethoxytrityl)-2'-O-(3,4-diacetoxybutoxymethyl)uridine (U4)

Compound U3 (8.5 g, 19.1 mmol) was azeotropically distilled with pyridine and dried in vacuo for 1 hr. Thereto was added dehydrated pyridine (75 mL) under an argon atmosphere, DMTr-Cl (9.0 g, 1.4eq.) was added at 0°C, and the mixture was stirred at room temperature overnight. After completion of the reaction, methanol (10 mL) was added and the mixture was stirred for 5 min. The solvent was evaporated under reduced pressure, dichloromethane was added to the residue, and the organic layer was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (n-hexane/ethyl acetate=1:2, 0.05% pyridine-containing) to give the object compound U4 as a pale-yellow foamy substance (5.6 g, purity 99.3%) .
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 50% isocratic; Flow rate, 1 mL/min; Temperature, 35°C; Detection, UV 260 nm.

### ESI-Mass:771.29 [M+Na]⁺

### (4) Synthesis of 5'-O-(4,4'-dimethoxytrityl)-2'-O-(3,4-diacetoxybutoxymethyl)uridine 3'-O-(2-cyanoethyl N,N-diisopropylphosphoramidite) (U5)

Compound U4 (7.8 g, 10.4 mmol) was azeotropically distilled with acetonitrile and dried in vacuo. This was dissolved in dehydrated acetonitrile (40 mL) under an argon atmosphere, diisopropylammoniumtetrazolide (2.1 g, 1.2eq.) and 2-cyanoethyl N,N,N',N'-tetraisopropylphosphorodiamidite (3.8 mL, 1.2eq.) were added, and the mixture was stirred at 40°C for 2 hr. The solvent was evaporated under reduced pressure, ethyl acetate was added and the mixture was washed once with a saturated aqueous sodium hydrogen carbonate solution, and once with a saturated aqueous sodium chloride solution. The washed organic layer was dried over sodium sulfate and concentrated under reduced pressure. The obtained crude product was purified by silica gel column chromatography (n-hexane/ethyl acetate=1:2, 0.1% triethylamine-containing) to give the object compound U5 as a white foamy substance (4.4 g, purity 98.3%). HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 75% isocratic; Flow rate, 1 mL/min; Temperature, 35°C; Detection, UV 260 nm.
³¹P-NMR (202 MHz, CDCl₃) δ: 150.51, 150.53, 151.45 ESI-Mass:971.39 [M+Na]⁺

### [Example 3: Synthesis of cytosine DBM amidite (C5)]

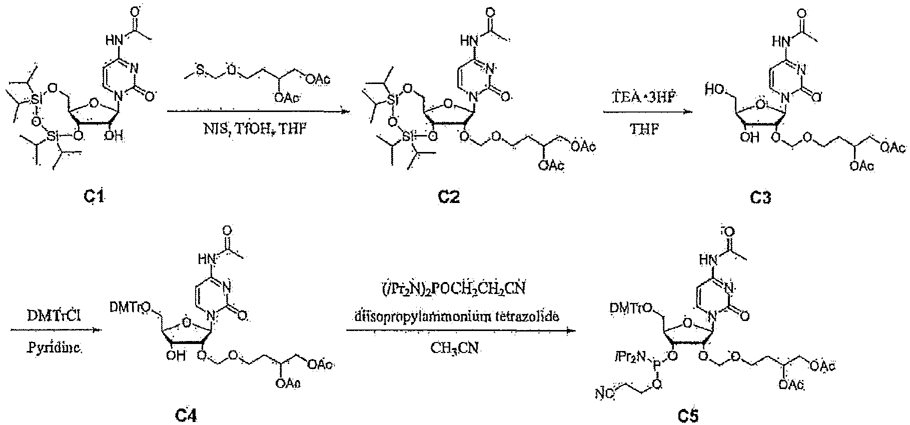

### (1) Synthesis of N⁴-acetyl-3',5'-O-(tetraisopropyldisiloxane-1,3-diyl)-2'-O-(3,4-diacetoxybutoxymethyl)cytidine (C2)

Compound C1 (2.0 g, 3.9 mmol) was azeotropically distilled twice with a mixed solvent of toluene and THF and once more with THF, and dried in vacuo for 1 hr. Dehydrated THF (12 mL), molecular sieves 4A (2.0 g), and N-iodosuccinimide (1.3 g, 1.5eq.) were added thereto under an argon atmosphere, and the mixture was stirred. The mixture was cooled to -45°C. After stirring for 5 min, trifluoromethanesulfonic acid (0.9 mL, 1.5eq.) was added dropwise. After stirring for 5 min, a DBM-forming reagent (1.5 g, 1.5eq.) was added and the mixture was stirred at -45°C for 30 min. After completion of the reaction, a cooled mixed solution of a saturated aqueous sodium thiosulfate solution and a saturated aqueous sodium hydrogen carbonate solution were added to the reaction solution, and the mixture was vigorously stirred at room temperature until the brown color disappeared. The mixture was extracted with ethyl acetate, and the organic layer was washed once with saturated aqueous sodium thiosulfate solution, once with saturated aqueous sodium hydrogen carbonate solution, and once with a saturated aqueous sodium chloride solution. The washed organic layer was dried over sodium sulfate and concentrated under reduced pressure. The obtained crude product was purified by silica gel column chromatography (n-hexane/ethyl acetate=1:2) to give the object compound C2 (2.3 g, purity 98.4%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 80% isocratic; Flow rate, 1 mL/min; Temperature, 35°C; Detection, UV 260 nm.

### (2) Synthesis of N⁴-acetyl-2'-O-(3,4-diacetoxybutoxymethyl)cytidine (C3)

To a solution of compound C2 (2.0 g) in dehydrated THF (12 mL) was added TEA/3HF (0.54 mL, 1.2eq.) under an argon atmosphere, and the mixture was stirred at room temperature for 3 hr. When n-hexane was added to the reaction solution, the solution became viscous. The supernatant was removed by decantation. The residue was triturated with n-hexane and the solvent was removed by decantation. This operation ((1) addition of n-hexane, (2) decantation of supernatant) was performed one more time. The solid was collected by filtration and dried in vacuo to give the object compound C3 as an amorphous solid (1.2 g, purity 98.0%).
HPLC conditions: Column, Inertsil ODS-3 reverse-phase (5.0 µm, 4.6 x 150 mm; GL Sciences); Buffer A, H₂O; Buffer B, MeOH; B conc. 0-100%/10 min, 100%/10-25 min; Flow rate, 1 mL/min; Temperature, 35°C; Detection, UV 260 nm.

### (3) Synthesis of N⁴-acetyl-5'-O-(4,4'-dimethoxytrityl)-2'-O-(3,4-diacetoxybutoxymethyl)cytidine (C4)

Compound C3 (1.7 g, 3.5 mmol) was azeotropically distilled with pyridine and dried in vacuo for 1 hr. Thereto was added dehydrated pyridine (20 mL) under an argon atmosphere, DMTr-Cl (1.8 g, 1.5eq.) was added at 0°C, and the mixture was stirred at room temperature overnight. After completion of the reaction, methanol (6 mL) was added and the mixture was stirred for 5 min. The solvent was evaporated under reduced pressure, dichloromethane was added to the residue, and the organic layer was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (hexane/ethyl acetate/acetone=1:1:1, 0.05% pyridine-containing) to give the object compound C4 as a white foamy substance (1.8 g, purity 98.6%) .
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 50% isocratic; Flow rate, 1 mL/min; Temperature, 35°C; Detection, UV 260 nm.
ESI-Mass: 790.31 [M+H]⁺

### (4) Synthesis of N⁴-acetyl-5'-O-(4,4'-dimethoxytrityl)-2'-O-(3,4-diacetoxybutoxymethyl)cytidine 3'-O-(2-cyanoethyl N,N-diisopropylphosphoramidite) (C5)

Compound C4 (2.0 g, 2.5 mmol) was azeotropically distilled with acetonitrile and dried in vacuo for 1 hr. Thereto was added dehydrated acetonitrile (8 mL) under an argon atmosphere, and the mixture was further suspended in diisopropylammonium tetrazolide (0.92 g, 1.2eq.). A solution of 2-cyanoethyl N,N,N',N'-tetraisopropylphosphorodiamidite (0.52 g, 1.2eq.) in dehydrated acetonitrile (2 mL) was added, and the mixture was stirred at 40°C for 3 hr. After completion of the reaction, the solvent was evaporated under reduced pressure, dichloromethane was added to the residue, and the organic layer was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (hexane/acetone=4:3, 0.1% triethylamine-containing) to give the object compound C5 as a white foamy substance (2.0 g, purity 97.5%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 70% isocratic; Flow rate, 1 mL/min; Temperature, 35°C; Detection, UV 260 nm.
³¹P-NMR (202 MHz, CDCl₃) δ: 151.50, 150.39
ESI-Mass: 990.43 [M+H]⁺

### [Example 4: Synthesis of guanosine DBM amidite (G5)]

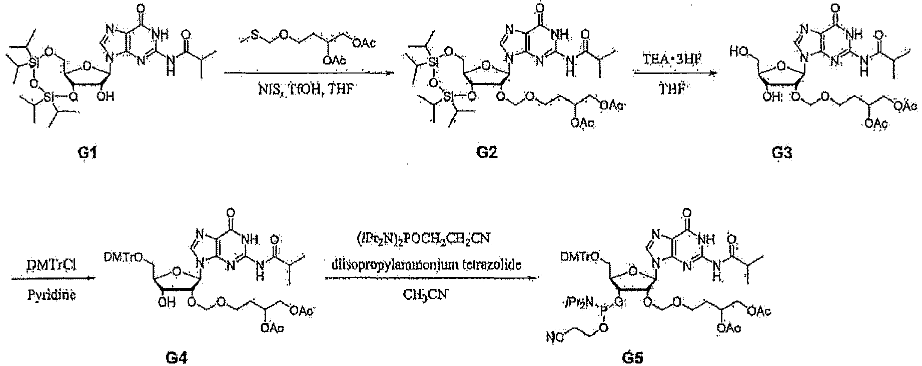

### (1) Synthesis of N²-isobutyryl-3',5'-O-(tetraisopropyldisiloxane-1,3-diyl)-2'-O-(3,4-diacetoxybutoxymethyl)-guanosine (G2)

Compound G1 (1.0 g, 1.7 mmol) was azeotropically distilled twice with a mixed solvent of toluene and THF and once with THF, and dried in vacuo for 1 hr. Dehydrated THF (6 mL), molecular sieves 4A (1.0 g), and N-iodosuccinimide (0.6 g, 1.5eq.) were added thereto under an argon atmosphere, and the mixture was stirred. The mixture was cooled to -45°C. After stirring for 5 min, trifluoromethanesulfonic acid (0.2 mL, 1.5eq.) was added dropwise. After stirring for 5 min, a DBM-forming reagent (0.6 g, 1.5eq.) was added and the mixture was stirred at -45°C for 30 min. After completion of the reaction, a cooled mixed solution of a saturated aqueous sodium thiosulfate solution and a saturated aqueous sodium hydrogen carbonate solution were added to the reaction solution, and the mixture was vigorously stirred at room temperature until the brown color disappeared. The mixture was extracted with ethyl acetate, and the organic layer was washed once with a saturated aqueous sodium thiosulfate solution, once with a saturated aqueous sodium hydrogen carbonate solution, and once with a saturated aqueous sodium chloride solution. The washed organic layer was dried over anhydrous sodium sulfate, and concentrated under reduced pressure to give the object compound G2 as a crude product (1.4 g, purity 96.5%). The obtained crude product was purified by silica gel column chromatography (n-hexane/ethyl acetate=1:2) to give the object compound G2 as a white foamy substance (1.2 g, purity 99.2%) .
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 80% isocratic; Flow rate, 1 mL/min; Temperature, 35°C; Detection, UV 260 nm.

### (2) Synthesis of N²-isobutyryl-2'-O-(3,4-diacetoxybutoxymethyl)guanosine (G3)

To a solution of compound G2 (1.0 g, 1.25 mmol) in dehydrated THF (3 mL) was added TEA/3HF (0.25 mL, 1.2eq.) under an argon atmosphere and the mixture was stirred at room temperature for 3 hr. After completion of the reaction, the solvent was evaporated under reduced pressure, and the residue was triturated with n-hexane (15 mL) and the solvent was removed by decantation. To the residue was added dichloromethane, and the mixture was washed with a saturated aqueous sodium chloride solution. The organic layer was dried over sodium sulfate and concentrated under reduced pressure. The obtained crude product was purified by silica gel column chromatography (dichloromethane/methanol=20:1) to give the object compound G3 as a white solid (0.7 g, purity 97.8%). HPLC conditions: Column, Inertsil ODS-3 reverse-phase (5.0 µm, 4.6 x 150 mm; GL Sciences); Buffer A, H₂O; Buffer B, MeOH; B conc. 0-100%/10 min, 100%/10-25 min; Flow rate, 1 mL/min; Temperature, 35°C; Detection, UV 260 nm.

### (3) Synthesis of N²-isobutyryl-5'-O-(4,4'-dimethoxytrityl)-2'-O-(3,4-diacetoxybutoxymethyl)guanosine (G4)

Compound G3 (2.0 g, 3.6 mmol) was azeotropically distilled with pyridine and dried in vacuo. Thereto was added pyridine (20 mL) under an argon atmosphere, DMTr-Cl (1.8 g, 1.5eq.) was added at 0°C, and the mixture was stirred at room temperature overnight. After completion of the reaction, methanol (5 mL) was added and the mixture was stirred for 30 min. The solvent was evaporated under reduced pressure, ethyl acetate was added to the residue, and the mixture was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (dichloromethane/ethyl acetate=2:3, 0.05% pyridine-containing) to give the object compound G4 as a white foamy substance (1.8 g, purity 98.3%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 50% isocratic; Flow rate, 1 mL/min; Temperature, 35°C; Detection, UV 260 nm.
ESI-Mass: 858.35 [M+H]⁺

### (4) Synthesis of N²-isobutyryl-5'-O-(4,4'-dimethoxytrityl)-2'-O-(3,4-diacetoxybutoxymethyl)guanosine 3'-O-(2-cyanoethyl N,N-diisopropylphosphoramidite) (G5)

Compound G4 (1.7 g, 2.0 mmol) was azeotropically distilled with acetonitrile and dried in vacuo. This was dissolved in acetonitrile (6 mL) under an argon atmosphere, diisopropylammoniumtetrazolide (0.4 g, 1.2eq.) and 2-cyanoethyl N,N,N',N'-tetraisopropylphosphorodiamidite (0.8 mL, 1.2eq.) were added, and the mixture was stirred at room temperature overnight. 2-Cyanoethyl N,N,N',N'-tetraisopropylphosphorodiamidite (0.5 mL, 0.8eq.) was added, and the mixture was stirred at room temperature for 3 hr more. The solvent was evaporated under reduced pressure, ethyl acetate was added and the mixture was washed once with a saturated aqueous sodium hydrogen carbonate solution, and once with a saturated aqueous sodium chloride solution. The washed organic layer was dried over sodium sulfate and concentrated under reduced pressure. The obtained crude product was purified by silica gel column chromatography (hexane/ethyl acetate=1:4, 0.1% triethylamine-containing) to give the object compound G5 as a white foamy substance (1.2 g, purity 98.0%). HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 75% isocratic; Flow rate, 1 mL/min; Temperature, 35°C; Detection, UV 260 nm.
³¹P-NMR (202 MHz, CDCl₃) δ: 150.86, 150.49
ESI-Mass: 1058.45 [M+H]⁺

### [Example 5: Synthesis of adenosine DBM amidite (AB5)]

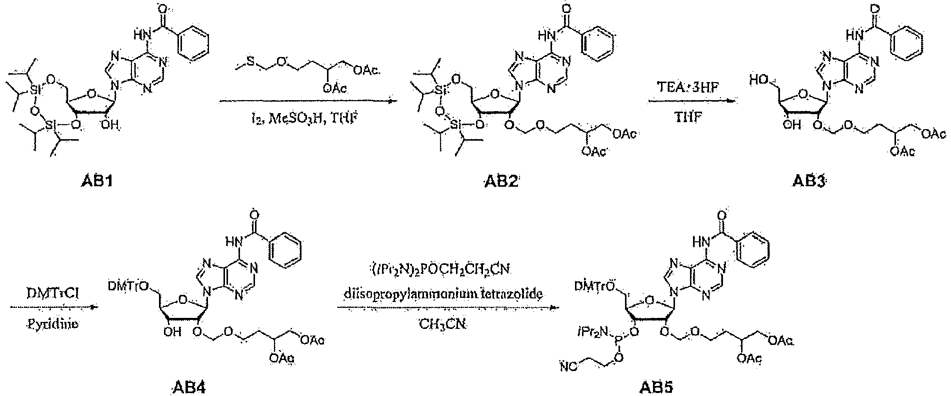

### (1) Synthesis of N⁶-benzoyl-3',5'-O-(tetraisopropyldisiloxane-1,3-diyl)-2'-O-(3,4-diacetoxybutoxymethyl)adenosine (AB2)

Compound AB1 (1.0 g, 1.6 mmol) was azeotropically distilled twice with a mixed solvent of toluene and THF and once with THF, and dried in vacuo for 1 hr. The residue was dissolved in dehydrated THF (3 mL) under an argon atmosphere, molecular sieves 4A (1.0 g) was added and the mixture was cooled to 0°C. Methanesulfonic acid (0.01 mL, 0.1eq.) was added dropwise to the mixture. After stirring for 10 min, iodine (2.5 g, 6.0eq.) was added, and the mixture was stirred for 10 min. A DBM-forming reagent (0.8 g, 2.0eq.) was added and the mixture was stirred at 0°C for 30 min. After completion of the reaction, a cooled mixed solution of a saturated aqueous sodium thiosulfate solution and a saturated aqueous sodium hydrogen carbonate solution were added to the reaction solution, and the mixture was vigorously stirred at room temperature until the brown color disappeared. The mixture was extracted with ethyl acetate, and the organic layer was washed once with saturated aqueous sodium thiosulfate solution, once with saturated aqueous sodium hydrogen carbonate solution, and once with a saturated aqueous sodium chloride solution. The washed organic layer was dried over sodium sulfate and concentrated under reduced pressure. The obtained crude product was purified by silica gel column chromatography (dichloromethane/methanol=40:1) to give the object compound AB2 (0.37 g, purity 98.4%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 80% isocratic; Flow rate, 1 mL/min; Temperature, 35°C; Detection, UV 260 nm.

### (2) Synthesis of N⁶-benzoyl-2'-O-(3,4-diacetoxybutoxymethyl)adenosine (AB3)

To a solution of compound AB2 (0.37 g, 0.45 mmol) in dehydrated THF (2 mL) was added TEA/3HF (0.22 mL, 3eq.) under an argon atmosphere, and the mixture was stirred at room temperature for 3.5 hr. The reaction solution was concentrated under reduced pressure to give an oily residue. When n-hexane was added to the residue, heavy syrup was produced. The supernatant was removed by decantation. The obtained crude product was purified by silica gel column chromatography (dichloromethane/methanol=20:1) to give the object compound AB3 (0.17 g, purity 91.3%).
HPLC conditions: Column, Inertsil ODS-3 reverse-phase (5.0 µm, 4.6 x 150 mm; GL Sciences); Buffer A, H₂O; Buffer B, MeOH; B conc. 0-100%/10 min, 100%/10-25 min; Flow rate, 1 mL/min; Temperature, 35°C; Detection, UV 260 nm.

### (3) Synthesis of N⁶-benzoyl-5'-O-(4,4'-dimethoxytrityl)-2'-O-(3,4-diacetoxybutoxymethyl)adenosine (AB4)

Compound AB3 (0.13 g, 0.23 mmol) was azeotropically distilled with pyridine and dried in vacuo. Dehydrated pyridine (2 mL) was added under an argon atmosphere, DMTr-Cl (0.12 g, 1.5eq.) was added at 0°C, and the mixture was stirred at room temperature overnight. DMTr-Cl (0.076 g, 1eq.) was further added and the mixture was stirred at room temperature for 3 hr. DMTr-Cl (0.19 g, 2.5eq.) was further added and the mixture was stirred at room temperature for 2 hr. The progress of the reaction was confirmed, methanol (0.25 mL) was added and the mixture was stirred for 10 min. The solvent was evaporated under reduced pressure, dichloromethane was added to the residue, and the mixture was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (dichloromethane/methanol=100:1 - 60:1, 0.05% pyridine-containing) to give the object compound AB4 (0.10 g, purity 96.7%) .
HPLC conditions: Column, XBridge Oligonucleotide BEH C18 (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 50% isocratic; Flow rate, 1 mL/min; Temperature, 35°C; Detection, UV 260 nm.
ESI-Mass:876.35 [M+H]⁺

### (4) Synthesis of N⁶-benzoyl-5'-O-(4,4'-dimethoxytrityl)-2'-O-(3,4-diacetoxybutoxymethyl)adenosine 3'-O-(2-cyanoethyl N,N-diisopropylphosphoramidite) (AB5)

Compound AB4 (0.09 g, 0.11 mmol) was azeotropically distilled with acetonitrile, and dried in vacuo. Thereto was added diisopropylammoniumtetrazolide (0.02 g, 1.1eq.) and the mixture was dried in vacuo. Thereto was added dehydrated acetonitrile (1.6 mL) under an argon atmosphere to give a suspension. 2-Cyanoethyl N,N,N',N'-tetraisopropylphosphorodiamidite (0.035 g, 1.1eq.) was added, and the mixture was stirred at 40°C for 2 hr. 2-Cyanoethyl N,N,N',N'-tetraisopropylphosphorodiamidite (0.04 g, 1.2 eq.) was further added and the mixture was stirred at 40°C for 1.5 hr. 2-Cyanoethyl N,N,N',N'-tetraisopropylphosphorodiamidite (0.08 g, 2.5eq.) was added and the mixture was stirred at 40°C for 1 hr. Then, diisopropylammoniumtetrazolide (0.02 g, 1.1eq.) was added and the mixture was stirred at 40°C for 1 hr. The solvent was evaporated under reduced pressure, dichloromethane was added to the residue, and the mixture was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (n-hexane/ethyl acetate=1:2, 0.1% triethylamine-containing) to give the object compound AB5 (0.024 g, purity 91.4%).
HPLC conditions: Column, XBridge Oligonucleotide BEH C18 (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 70% isocratic; Flow rate, 1 mL/min; Temperature, 35°C; Detection, UV 260 nm.
³¹P-NMR (202 MHz, CDCl₃) δ: 151.19, 151.10
ESI-Mass:1076.46 [M+H]⁺

### [Example 6: Synthesis of adenosine DBM amidite (AA5)]

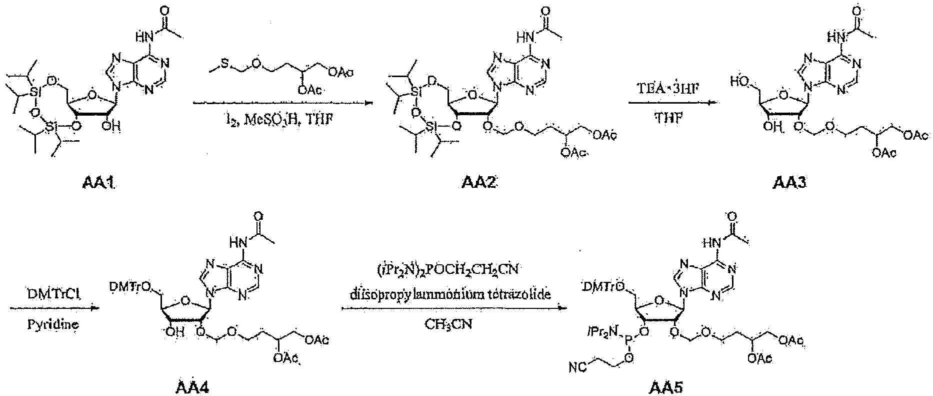

### (1) Synthesis of N⁶-acetyl-3',5'-O-(tetraisopropyldisiloxane-1,3-diyl)-2'-O-(3,4-diacetoxybutoxymethyl)adenosine (AA2)

Compound AA1 (1.0 g, 1.8 mmol) was azeotropically distilled twice with a mixed solvent of toluene and THF and once with THF, and dried in vacuo for 1 hr. This was dissolved in dehydrated THF (3 mL) under an argon atmosphere, molecular sieves 4A (1.0 g) was added and the mixture was cooled to 0°C. Methanesulfonic acid (0.01 mL, 0.1eq.) was added dropwise to the mixture. After stirring for 10 min, iodine (2.8 g, 6.0eq.) was added, and the mixture was stirred for 10 min. A DBM-forming reagent (0.7 g, 1.5eq.) was added and the mixture was stirred at 0°C for 30 min. After completion of the reaction, a cooled mixed solution of a saturated aqueous sodium thiosulfate solution and a saturated aqueous sodium hydrogen carbonate solution were added to the reaction solution, and the mixture was vigorously stirred at room temperature until the brown color disappeared. The mixture was extracted with ethyl acetate, and the organic layer was washed once with saturated aqueous sodium thiosulfate solution, once with saturated aqueous sodium hydrogen carbonate solution, and once with a saturated aqueous sodium chloride solution. The washed organic layer was dried over sodium sulfate and concentrated under reduced pressure. The obtained crude product was purified by silica gel column chromatography (hexane:ethyl acetate=1:1 - 1:3) to give the object compound AA2 (0.45 g, purity 99.3%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 80% isocratic; Flow rate, 1 mL/min; Temperature, 35°C; Detection, UV 260 nm.

### (2) Synthesis of N⁶-acetyl-2'-O-(3,4-diacetoxybutoxymethyl)adenosine (AA3)

To a solution of compound AA2 (0.45 g, 0.9 mmol) in dehydrated THF (2 mL) was added TEA/3HF (0.12 mL, 1.2eq.) under an argon atmosphere and the mixture was stirred at room temperature for 2 hr. After completion of the reaction, the solvent was evaporated under reduced pressure. The residue was triturated with n-hexane and the solvent was removed by decantation. This operation (trituration and decantation) was performed 3 times. After drying in vacuo, the object compound AA3 was obtained as a crude product (0.47 g, purity 87.2%). HPLC conditions: Column, Inertsil ODS-3 reverse-phase (5.0 µm, 4.6 x 150 mm; GL Sciences); Buffer A, H₂O; Buffer B, MeOH; B conc. 0-100%/10 min, 100%/10-25 min; Flow rate, 1 mL/min; Temperature, 35°C; Detection, UV 260 nm.

### (3) Synthesis of N⁶-acetyl-5'-O-(4,4'-dimethoxytrityl)-2'-O-(3,4-diacetoxybutoxymethyl)adenosine (AA4)

Compound AA3 (0.47 g, 0.9 mmol) was azeotropically distilled with pyridine and dried in vacuo for 1 hr. Thereto was added dehydrated pyridine (4 mL) under an argon atmosphere, DMTr-Cl (0.5 g, 1.5eq.) was added at 0°C, and the mixture was stirred at room temperature for 2 hr. DMTr-Cl (0.5 g, 1.5eq.) was further added and the mixture was stirred at room temperature for 1 hr. After completion of the reaction, methanol (1 mL) was added and the mixture was stirred for 30 min. The solvent was evaporated under reduced pressure, dichloromethane was added to the residue, and the organic layer was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (hexane:ethyl acetate=2:3 - 1:2, 0.05% pyridine-containing) to give the object compound AA4 (0.18 g, purity 100%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 50% isocratic; Flow rate, 1 mL/min; Temperature, 35°C; Detection, UV 260 nm.
ESI-Mass: 814.32 [M+H]⁺

### (4) Synthesis of N⁶-acetyl-5'-O-(4,4'-dimethoxytrityl)-2'-O-(3,4-diacetoxybutoxymethyl)adenosine 3'-O-(2-cyanoethyl N,N-diisopropylphosphoramidite) (AA5)

Compound AA4 (0.17 g, 0.2 mmol) was azeotropically distilled with acetonitrile, and dried in vacuo for 1 hr. Thereto was added dehydrated acetonitrile (1.4 mL) under an argon atmosphere, and diisopropylammoniumtetrazolide (0.04 g, 1.1eq.) was further added to give a suspension. 2-Cyanoethyl N,N,N',N'-tetraisopropylphosphorodiamidite (0.07 g, 1.1eq.) was added, and the mixture was stirred at 40°C for 2 hr. 2-Cyanoethyl N,N,N',N'-tetraisopropylphosphorodiamidite (0.07 g, 1.1 eq.) was further added and the mixture was stirred at 40°C for 1 hr. 2-Cyanoethyl N,N,N',N'-tetraisopropylphosphorodiamidite (0.14 g, 2.2 eq.) was further added and the mixture was stirred at 40°C for 1 hr. Diisopropylammoniumtetrazolide (0.04 g, 1.1eq.) was added and the mixture was stirred at 40°C for 1 hr. Progress of the reaction was confirmed and the solvent was evaporated under reduced pressure. Dichloromethane was added to the residue, and the organic layer was washed twice with a saturated aqueous sodium hydrogen carbonate solution and once with a saturated aqueous sodium chloride solution. The washed organic layer was dried over sodium sulfate and concentrated under reduced pressure. The obtained crude product was purified by silica gel column chromatography (hexane:acetone=3:1 - 2:1, 0.1% triethylamine-containing) to give the object compound AA5 (0.04 g, purity 98.6%).
HPLC conditions: Column, XBridge Oligonucleotide BEH C18 reverse-phase (2.5 µm, 4.6 x 50 mm; Waters); Buffer A, 5% CH₃CN/ 50 mM TEAA, pH 7.0; Buffer B, 90% CH₃CN/ 50 mM TEAA, pH 7.0; Buffer B 70% isocratic; Flow rate, 1 mL/min; Temperature, 35°C; Detection, UV 260 nm.
³¹P-NMR (202 MHz, CDCl₃) δ: 151.19, 151.08.
ESI-Mass: 1014.44 [M+H]⁺

### [Example 7: Synthesis of uridine 20-mer (dT+U20 mer) using uridine DBM amidite (U5)]

Using the uridine DBM amidite (U5) synthesized in Example 2 and a nucleic acid automatic synthesizer (ABI 3900: trade name of Applied Biosystems), the uridine 20-mer shown by the sequence of the following SEQ ID NO: 1 was synthesized. As the solid phase carrier of the 3'-terminal, deoxythymidine free of 2'-OH group was used.
5'-UUUUUUUUUUUUUUUUUUUUT-3' (SEQ ID NO: 1)

For the synthesis of the uridine 20-mer in this Example, a CPG solid phase carrier wherein 5'-O-(4,4'-dimethoxytrityl)thymidine is linked by a linker was used as a solid phase carrier. Furthermore, 5'-O-(4,4'-dimethoxytrityl)-2'-O-(3,4-diacetoxybutoxymethyl)uridine 3'-O-(2-cyanoethyl N,N-diisopropylphosphoramidite), i.e., uridine DBM amidite (U5), was used as a nucleic acid monomer compound, 5-benzylmercapto-1H-tetrazole was used as a condensing agent, an iodine solution was used as an oxidant, and an acetic anhydride/pyridine solution and an N-methylimidazole solution were used as capping solutions. Under these conditions, the aforementioned nucleic acid monomer compound was condensed 20 times, the 5' terminal hydroxyl group was deprotected on the solid phase, and cleavage from the CPG solid phase carrier and deprotection of each phosphoric acid site were performed using conc. aqueous ammonia-ethanol mixture (3:1) at 80°C for 6 hr. The reaction mixture thus obtained was subjected to oxidation of 1,2-cis-diol using sodium periodate in 0.2 M acetate buffer (pH 4.2), and after completion of the reaction, glycerol was added and the mixture was stirred. The solution thus obtained was precipitated by adding ethanol, the precipitate was dissolved in water for injection and reacted overnight at room temperature in a 1M solution of tetrabutylammonium fluoride in DMSO, whereby the 2'-position hydroxyl group was deprotected. Ethanol was added to the thus-obtained solution to allow precipitation, and the precipitate was dissolved in water for injection to give an aqueous solution containing the object compound (uridine 20-mer (dT+U20 mer)).

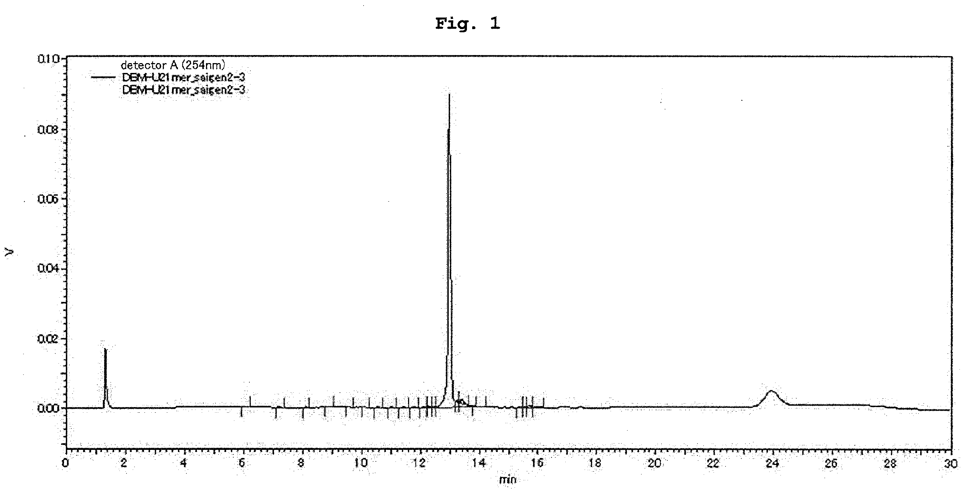

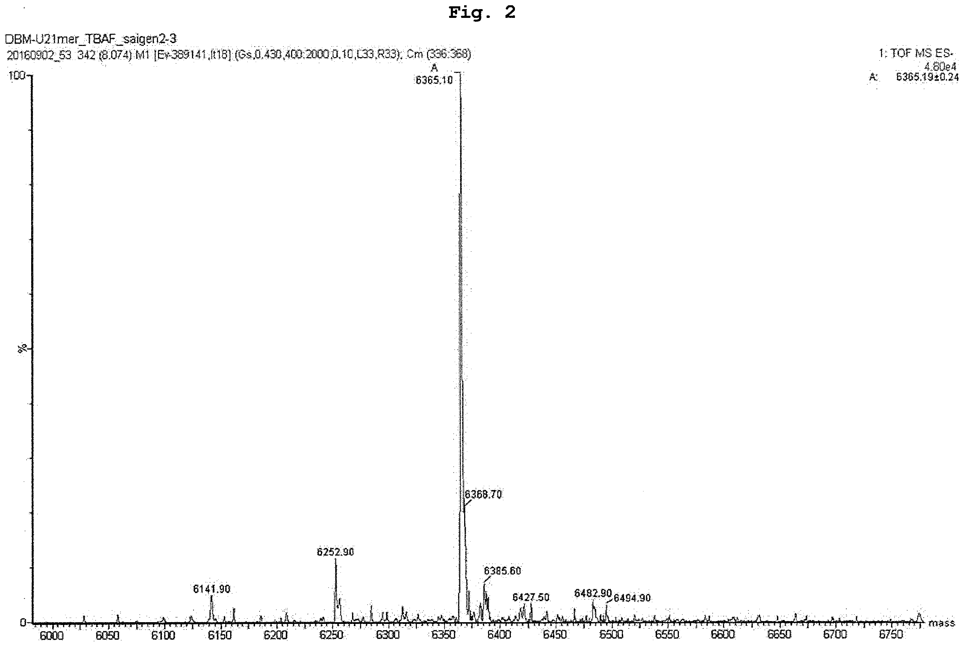

The analysis results of the uridine 20-mer (dT+U20 mer) obtained above by HPLC are shown in Fig. 1. As shown in the Figure, since an almost single sharp peak was obtained, it was suggested that the object uridine 20-mer (dT+U20 mer) was obtained with high purity. The purity of the uridine 20-mer (dT+U20 mer) calculated based on the peak intensity of Fig. 1 was 93.58%. The results of the mass spectrometry (mass spectrum chart) of this reaction mixture are shown in Fig. 2. As shown in the Figure, the molecular ion peak of molecular weight 6365.10 was observed. Since this molecular weight matched well with the calculated value (6365.57) of the molecular weight of the object uridine 20-mer (dT+U20 mer), it was confirmed that the object uridine 20-mer (dT+U20 mer) was obtained.

The HPLC analysis of the uridine 20-mer (dT+U20 mer) was performed using an instrument (HPLC system) of SHIMADZU CORPORATION, and the mass spectrometry was performed using an instrument of Waters (SYNAPT G2 (trade name)).

### [Example 8: Synthesis of uridine 49-mer (dT+U49 mer) using uridine DBM amidite (U5) - 1]

Using the uridine DBM amidite (U5) synthesized in Example 2 and a nucleic acid automatic synthesizer (ABI 3900: trade name of Applied Biosystems), the uridine 49-mer shown by the sequence of the following SEQ ID NO: 2 was synthesized. As the solid phase carrier of the 3'-terminal, deoxythymidine free of 2'-OH group was used.
5'-UUUUUUUUUUUUUUUUUUUUUUUUUUUUUUUUUUUUUUUUUUUUUUUUUT-3' (SEQ ID NO: 2)

For the synthesis of the uridine 49-mer in this Example, a CPG solid phase carrier wherein 5'-O-(4,4'-dimethoxytrityl)thymidine is linked by a linker was used as a solid phase carrier. Furthermore, 5'-O-(4,4'-dimethoxytrityl)-2'-O-(3,4-diacetoxybutoxymethyl)uridine 3'-O-(2-cyanoethyl N,N-diisopropylphosphoramidite), i.e., uridine DBM amidite (U5), was used as a nucleic acid monomer compound, 5-benzylmercapto-1H-tetrazole was used as a condensing agent, an iodine solution was used as an oxidant, and an acetic anhydride/pyridine solution and an N-methylimidazole solution were used as capping solutions. Under these conditions, the aforementioned nucleic acid monomer compound was condensed 49 times, the 5' terminal hydroxyl group was deprotected on the solid phase, and cleavage from the CPG solid phase carrier and deprotection of each phosphoric acid site were performed using conc. aqueous ammonia-ethanol mixture (3:1) at 80°C for 6 hr. The reaction mixture thus obtained was subjected to oxidation of 1,2-cis-diol using sodium periodate in 0.2 M acetate buffer (pH 4.2), and after completion of the reaction, glycerol was added and the mixture was stirred. The solution thus obtained was precipitated by adding ethanol, the precipitate was dissolved in water for injection, transferred to a reaction flask and dried to solidness under reduced pressure with a vaccum pump. To the residue was added a 1M solution of tetrabutylammonium fluoride in DMSO and the mixture was reacted overnight at room temperature, whereby the 2'-position hydroxyl group was deprotected. Ethanol was added to the thus-obtained solution to allow precipitation, and the precipitate was dissolved in water for injection to give an aqueous solution containing the object compound (uridine 49-mer (dT+U49 mer)).

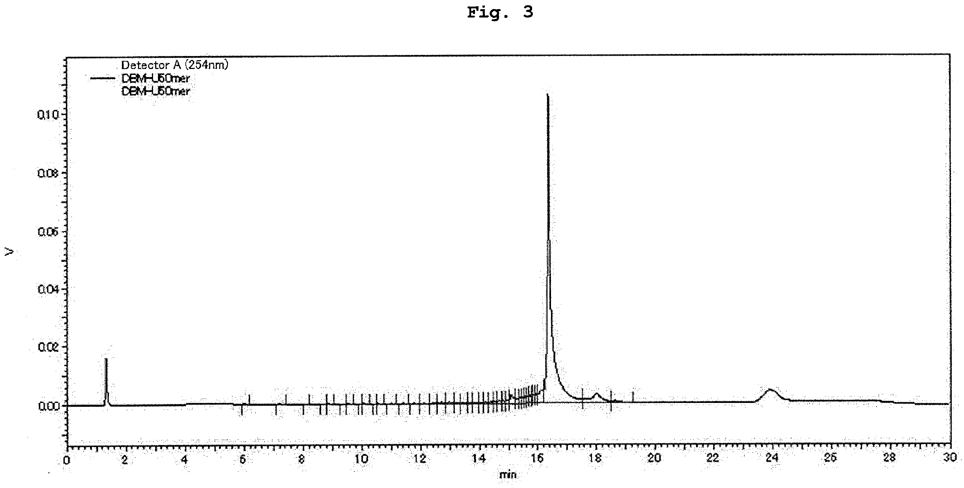

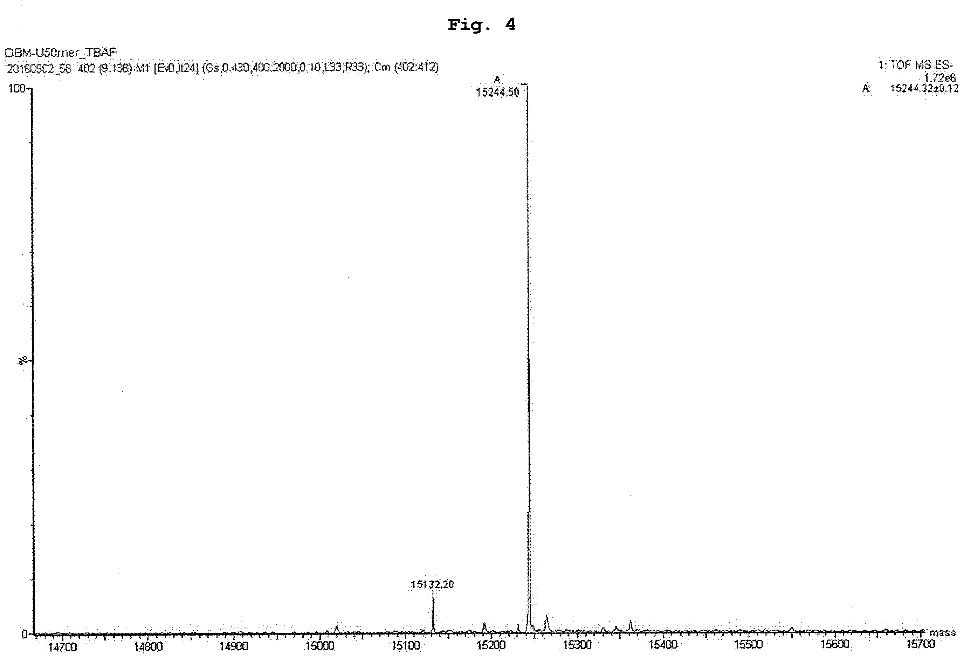

The analysis results of the uridine 49-mer (dT+U49 mer) obtained above by HPLC are shown in Fig. 3. As shown in the Figure, since an almost single sharp peak was obtained, it was suggested that the object uridine 49-mer (dT+U49 mer) was obtained with high purity. The purity of the uridine 49-mer (dT+U49 mer) calculated based on the peak intensity of Fig. 3 was 74.74%. The results of the mass spectrometry (mass spectrum chart) of this reaction mixture are shown in Fig. 4. As shown in the Figure, the molecular ion peak of molecular weight 15244.50 was observed. Since this molecular weight matched well with the calculated value (15244.37) of the molecular weight of the object uridine 49-mer (dT+U49 mer), it was confirmed that the object uridine 49-mer (dT+U49 mer) was obtained.

### [Example 9: Synthesis of uridine 49-mer (dT+U49 mer) using uridine DBM amidite (U5) - 2]

To the aldehyde derivative obtained by the periodization reaction of Example 8 is added a solution of orthonitrobenzylamine (2 - 5 equivalents relative to the aldehyde group) in DMSO, and the mixture is stirred at room temperature for several hours to give an imino compound. The solution thus obtained is precipitated by adding ethanol, the precipitate is dissolved in water for injection, transferred to a reaction flask and dried to solidness under reduced pressure with a vaccum pump. To the residue is added a 1M solution of tetrabutylammonium fluoride in DMSO and the mixture is reacted overnight at room temperature, whereby the 2'-position protecting group is deprotected. Ethanol is added to the thus-obtained solution to allow precipitation, and the precipitate is dissolved in water for injection to give an aqueous solution containing the object compound (uridine 49-mer (dT+U49 mer)).

### [Example 10: Synthesis of guanosine·uridine 20-mer (dT+GU20 mer) using guanosine DBM amidite (G5) and uridine DBM amidite (U5)]

In the same manner as in Example 8 and using uridine DBM amidite (U5) synthesized in Example 2, guanosine DBM amidite (G5) synthesized in Example 4, and a nucleic acid automatic synthesizer (ABI 3900: trade name of Applied Biosystems), a guanosine·uridine 20-mer shown by the sequence of the following SEQ ID NO: 3 was synthesized. As the solid phase carrier at the 3'-terminal, deoxythymidine free of a 2'-OH group was used.
5'-GUGUGUGUGUGUGUGUGUGUT-3' (SEQ ID NO: 3)

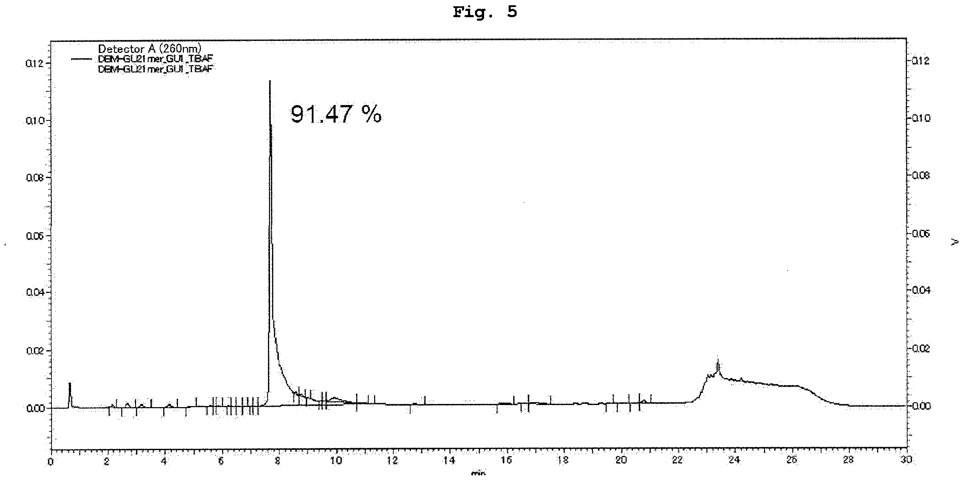

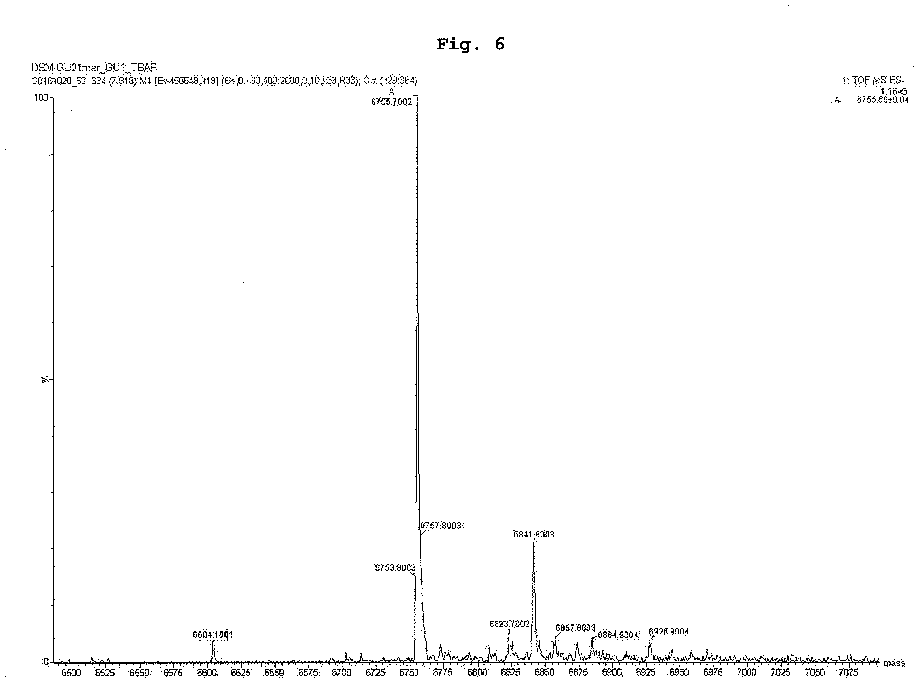

The analysis results of the guanosine·uridine 20-mer (dT+GU 20 mer) obtained above by HPLC are shown in Fig. 5. As shown in the Figure, since an almost single sharp peak was obtained, it was suggested that the object guanosine·uridine 20-mer (dT+GU 20 mer) was obtained with high purity. The purity of the guanosine·uridine 20-mer (dT+GU 20 mer) calculated based on the peak intensity of Fig. 5 was 91.47%. The results of the mass spectrometry (mass spectrum chart) of this reaction mixture are shown in Fig. 6. As shown in the Figure, the molecular ion peak of the molecular weight was observed. It was confirmed that the object guanosine·uridine 20-mer (dT+GU 20 mer) was obtained.

While the present invention has been explained by referring to the embodiments, the present invention is not limited by the above-mentioned embodiments. The constituent and detail of the present invention can be variously changed within the scope of the present invention as long as those of ordinary skill in the art can understand.

### [Industrial Applicability]

As explained above, according to the glycoside compound, ether, the production method of ether, and the production method of the glycoside compound of the present invention, a phosphoramidite, which can be produced at a low cost and can produce a nucleic acid in a high yield and with high purity can be provided. In addition, according to the production method of a nucleic acid of the present invention, a nucleic acid can be produced in a high yield and with high purity using the aforementioned phosphoramidite. The use of the aforementioned ether, glycoside compound, and nucleic acid produced by the present invention is not particularly limited, and they can be used for a wide range of use. According to the present invention, for example, they can be preferably used as pharmaceutical products or synthetic intermediate s therefor, since they can be obtained at a low cost, in a high yield, with high purity.

This application is based on a patent application Nos. 2016-202663 filed in Japan (filing date: October 14, 2016) and 2017-053633 filed in Japan (filing date: March 17, 2017), the contents of which are incorporated in full herein.

## Claims

1. A glycoside compound represented by the following chemical formula (1),
an enantiomer, tautomer or stereoisomer thereof or a salt thereof: 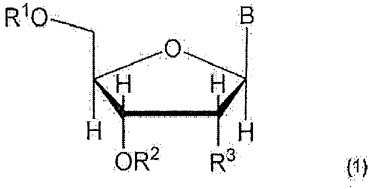 in said chemical formula (1),
B is an atomic group having a nucleic acid base skeleton, and optionally having a protecting group,
R¹ and R² are each a hydrogen atom or a protecting group,
or R¹ and R² in conjunction optionally form an atomic group represented by the following chemical formula (R¹R²A) or (R¹R²B) : 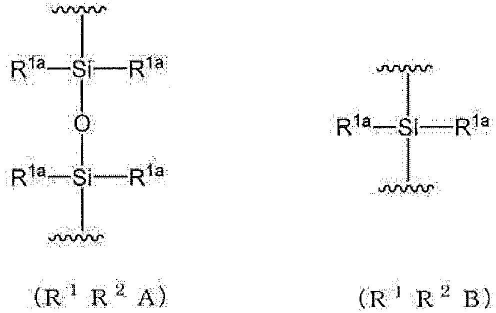
each R^{1a} is a hydrogen atom, a straight chain or branched alkyl group, or a straight chain or branched alkoxy group, which may be the same or different,
R³ is a group represented by the following chemical formula (R³) : 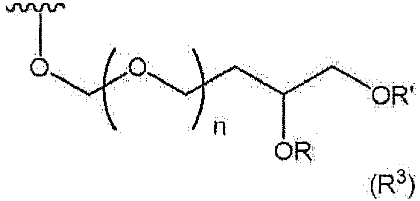
in said chemical formula (R³),
n is a positive integer,
R and R' are the same or different and each is a hydrogen atom or a hydroxyl-protecting group.

2. The glycoside compound according to claim 1, wherein R and R' are the same or different and each is an acyl group,
an enantiomer, tautomer or stereoisomer thereof or a salt thereof.

3. The glycoside compound according to claim 1, wherein R and R' are acetyl groups,
an enantiomer, tautomer or stereoisomer thereof or a salt thereof.

4. The glycoside compound according to any one of claims 1 to 3, wherein, in said chemical formula (1),
R¹ is a hydrogen atom, or a substituent represented by any of the following chemical formulas (R¹A), (R¹B), (R¹C) and (R¹D),
an enantiomer, tautomer or stereoisomer thereof or a salt thereof: 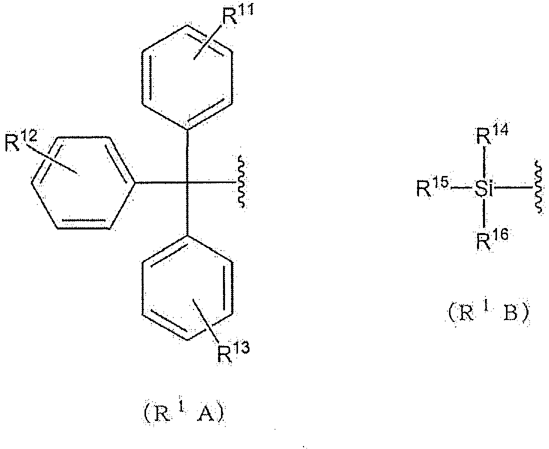 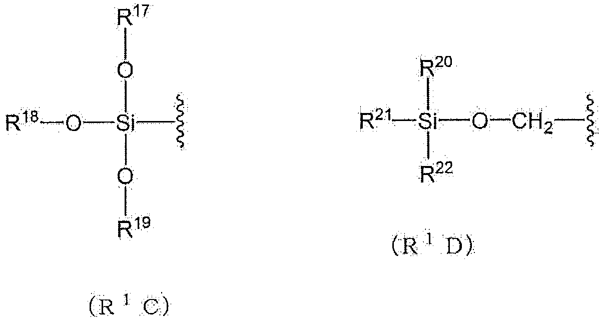 in said chemical formula (R¹A),
R¹¹ - R¹³ may be the same or different and each is a straight chain or branched alkoxy group, or a straight chain or branched alkyl group, or absent,
R¹¹ - R¹³ are, when they are present, respectively present singly or in plurality, and when present in plurality, they may be the same or different,
in said chemical formula (R¹B),
R¹⁴ - R¹⁶ may be the same or different and each is a hydrogen atom, a straight chain or branched alkyl group, or a straight chain or branched alkoxy group,
in said chemical formula (R¹C),
R¹⁷ - R¹⁹ are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which may be the same or different,
in said chemical formula (R¹D),
R²⁰ - R²² may be the same or different and each is a hydrogen atom, or a straight chain or branched alkyl group.

5. The glycoside compound according to any one of claims 1 to 3, wherein, in said chemical formula (1),
R¹ is a hydrogen atom, or a substituent represented by the following chemical formula (R¹Aa), (R¹Ba), (R¹Ca), (R¹Cb) or (R¹Da),
an enantiomer, tautomer or stereoisomer thereof or a salt thereof:

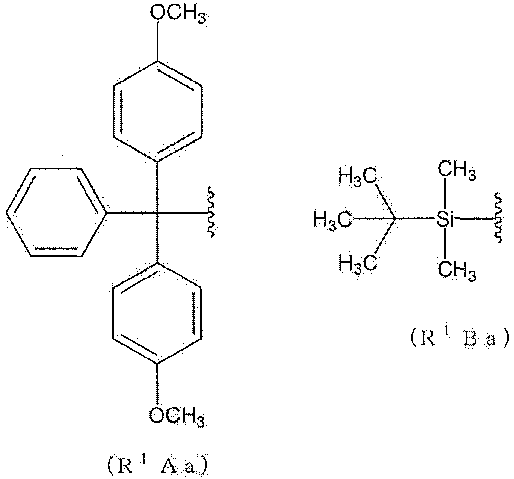

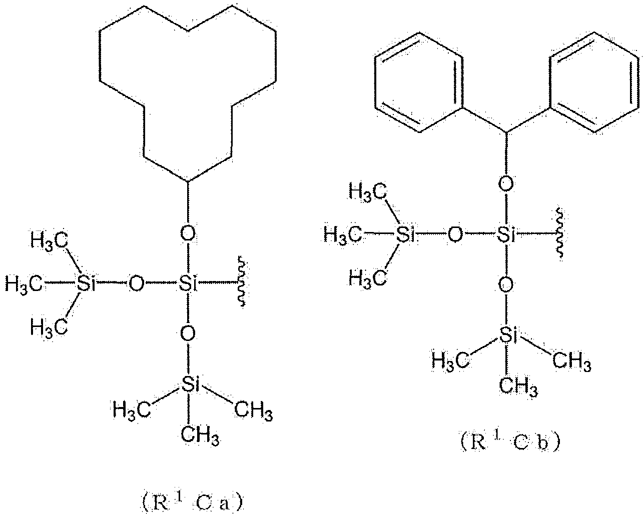

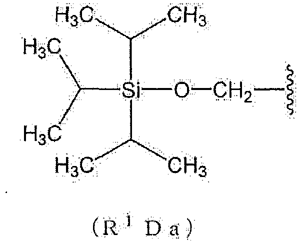

6. The glycoside compound according to any one of claims 1 to 3, wherein, in said chemical formula (1),
R¹ and R² in conjunction optionally form an atomic group represented by said chemical formula (R¹R²A) or (R¹R²B):
in said chemical formula (R¹R²A) and (R¹R²B),
each R^{1a} is a hydrogen atom, a straight chain or branched alkyl group, or a straight chain or branched alkoxy group, which may be the same or different,
an enantiomer, tautomer or stereoisomer thereof or a salt thereof.

7. The glycoside compound according to any one of claims 1 to 5, wherein the glycoside compound represented by said chemical formula (1) is a glycoside compound represented by the following chemical formula (2),
an enantiomer, tautomer or stereoisomer thereof or a salt thereof: 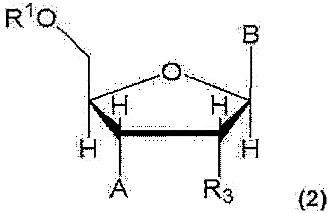 in said chemical formula (2),
B, R¹ and R³ are the same as those in said chemical formula (1),
R¹ is a protecting group,
A is a group represented by the following chemical formula (2-1), (2-2-1), (2-2-2) or (2-2-3): 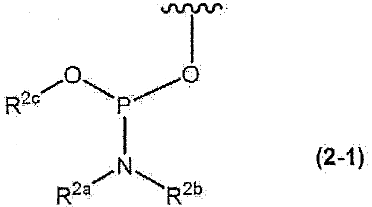 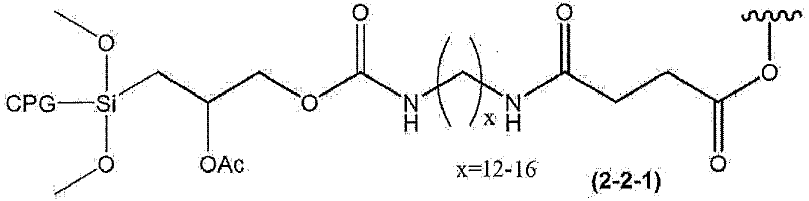 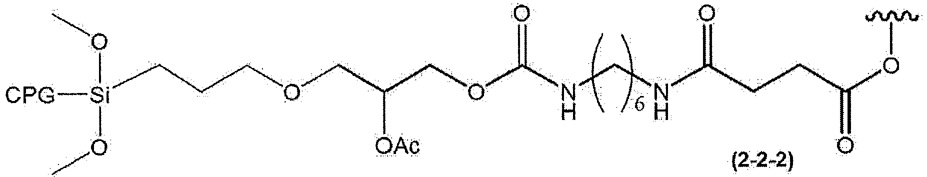 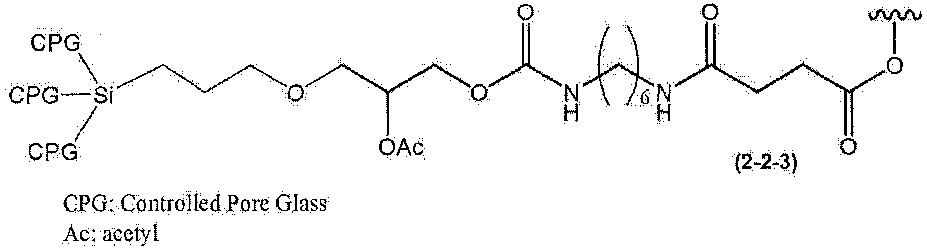
R^{2a} and R^{2b} may be the same or different and each is a hydrogen atom or any substituent,
alternatively, R^{2a} and R^{2b} may form, in conjunction with the nitrogen atom bonded thereto, a non-aromatic ring, wherein said non-aromatic ring may or may not have a nitrogen atom, an oxygen atom or a sulfur atom besides said nitrogen atom, and may or may not further have a substituent, and
R^{2c} is a hydrogen atom, an electron-withdrawing group or any substituent, which may be optionally substituted by an electron-withdrawing group [D²].

8. The glycoside compound according to claim 7, wherein, in said chemical formula (2-1),
R^{2a} and R^{2b} are each a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, which is optionally further substituted or not substituted by an electron-withdrawing group,
alternatively, R^{2a} and R^{2b} may form, in conjunction with the nitrogen atom bonded thereto, a 5- or 6-membered non-aromatic ring, wherein said non-aromatic ring may or may not have a nitrogen atom, an oxygen atom or a sulfur atom besides said nitrogen atom, and may or may not further have a substituent,
an enantiomer, tautomer or stereoisomer thereof or a salt thereof.

9. The glycoside compound according to claim 7, wherein, in said chemical formula (2-1),
R^{2a} and R^{2b} are each a methyl group, an ethyl group, an isopropyl group, or a t-butyl group, or
R^{2a} and R^{2b} form, in conjunction with a nitrogen atom bonded thereto, a piperidyl group, a morpholino group, a pyrrolidyl group, a thiomorpholino group, or other nitrogen-containing alicyclic group,
an enantiomer, tautomer or stereoisomer thereof or a salt thereof.

10. The glycoside compound according to any one of claims 7 to 9, wherein,
in said chemical formula (2-1),
R^{2c} is a hydrogen atom, halogen, a straight chain or branched alkyl group, a straight chain or branched alkenyl group, a straight chain or branched alkynyl group, a straight chain or branched haloalkyl group, an aryl group, a heteroaryl group, a straight chain or branched arylalkyl group, a cycloalkyl group, a cycloalkenyl group, a straight chain or branched cycloalkylalkyl group, a straight chain or branched cyclylalkyl group, a straight chain or branched hydroxyalkyl group, a straight chain or branched alkoxyalkyl group, a straight chain or branched aminoalkyl group, a straight chain or branched heterocyclylalkenyl group, a straight chain or branched heterocyclylalkyl group, a straight chain or branched heteroarylalkyl group, a silyl group, a silyloxyalkyl group, a mono-, di- or trialkylsilyl group, or a mono-, di- or trialkylsilyloxyalkyl group, and further, may or may not be substituted by an electron-withdrawing group [D²],
an enantiomer, tautomer or stereoisomer thereof or a salt thereof.

11. The glycoside compound according to any one of claims 7 to 9, wherein, in said chemical formula (2-1),
R^{2c} is a straight chain or branched alkyl group substituted by an electron-withdrawing group [D²],
an enantiomer, tautomer or stereoisomer thereof or a salt thereof.

12. The glycoside compound according to any one of claims 7 to 11, wherein, in said chemical formula (2-1),
said electron-withdrawing group [D²] for R^{2c} is a cyano group, a nitro group, an alkylsulfonyl group, halogen, an arylsulfonyl group, or a trihalomethyl group,
an enantiomer, tautomer or stereoisomer thereof or a salt thereof.

13. The glycoside compound according to any one of claims 7 to 11, wherein,
in said chemical formula (2-1),
R^{2c} is an alkenyl group or an ethynyl group, or substituted by an electron-withdrawing group [D²] and form, together with [D²], a cyanoethyl group,
an enantiomer, tautomer or stereoisomer thereof or a salt thereof.

14. The glycoside compound according to any one of claims 1 to 5 and 7 to 13, wherein,
the glycoside compound represented by said chemical formula (1) is a glycoside compound represented by the following chemical formula (3),
an enantiomer, tautomer or stereoisomer thereof or a salt thereof: 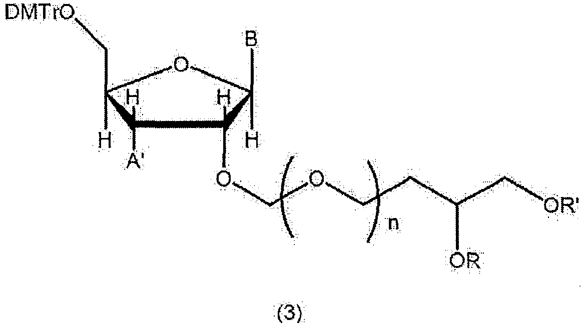 in said chemical formula (3),
R, R', B and n are as defined for said chemical formula. (1), and
DMTr is a 4,4'-dimethoxy(triphenylmethyl) group, and
A' is a group represented by the following chemical formula (3-1), (3-2-1) or (3-2-2):

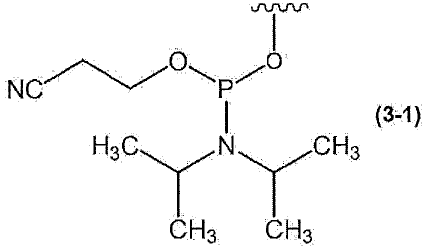

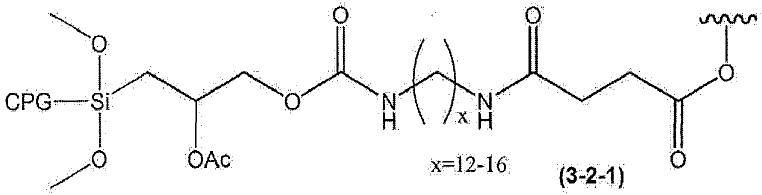

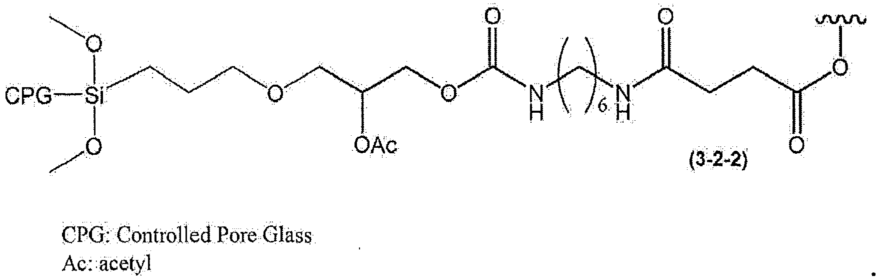

15. The glycoside compound according to claim 1, wherein the glycoside compound represented by said chemical formula (1) is a glycoside compound represented by the following chemical formula (AA5), (AB5), (C5), (G5) or (U5),
an enantiomer, tautomer or stereoisomer thereof or a salt thereof: 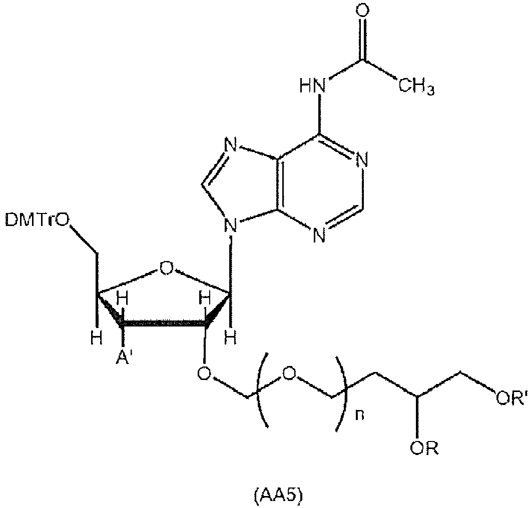 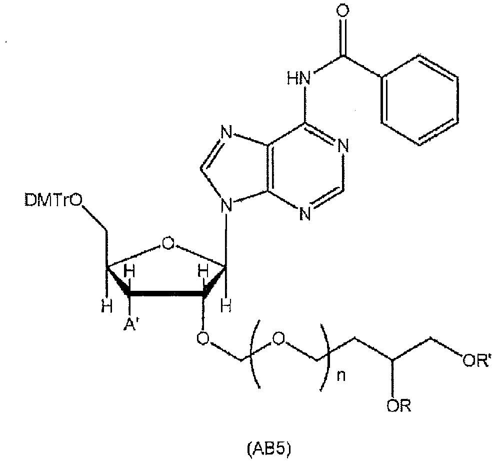 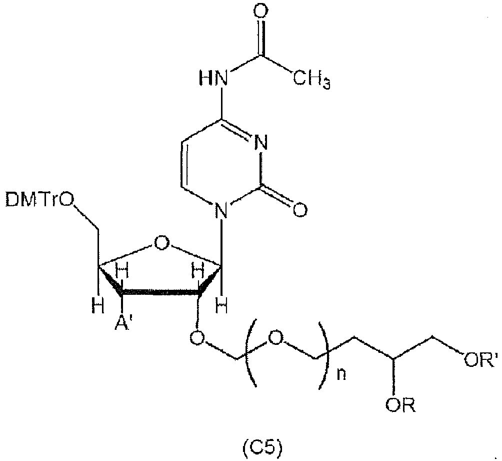 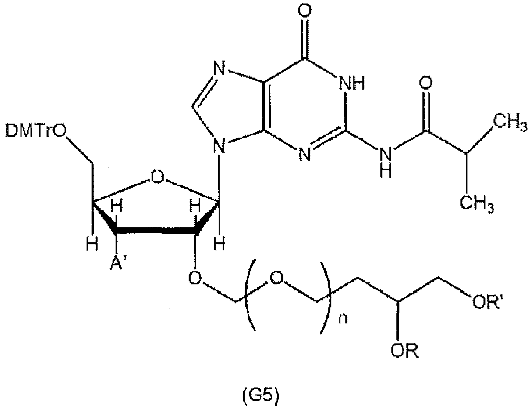 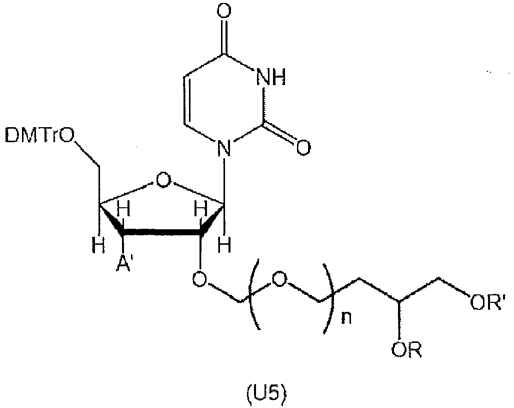 in said chemical formulas (AA5), (AB5), (C5), (G5) and (U5), R, R' and n are as defined for said chemical formula (1), DMTr is a 4,4'-dimethoxy(triphenylmethyl) group, A' is a group represented by the following chemical formula (3-1), (3-2-1) or (3-2-2):

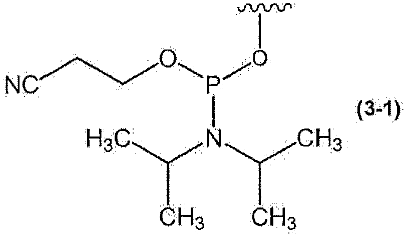

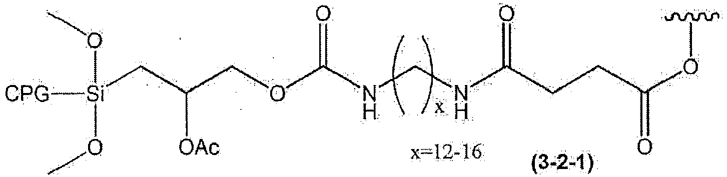

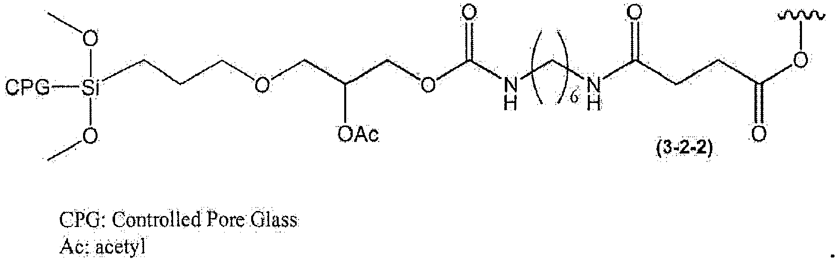

16. The glycoside compound according to any one of claims 1 to 15, wherein, in said chemical formula (1), n=1, an enantiomer, tautomer or stereoisomer thereof or a salt thereof.

17. A method for producing an ether represented by the following chemical formula (105), comprising
(1) methylthiomethylating a compound represented by the following chemical formula (102) to produce a compound represented by the following chemical formula (103);
(2) subjecting the compound represented by the following chemical formula (103) to a ring opening reaction to produce a compound represented by the following chemical formula (104); and
(3) protecting a hydroxyl group of the compound represented by the following chemical formula (104), and repeating, where necessary, a coupling reaction with methylthiomethanol in the presence of a halogenating agent and a Lewis acid: 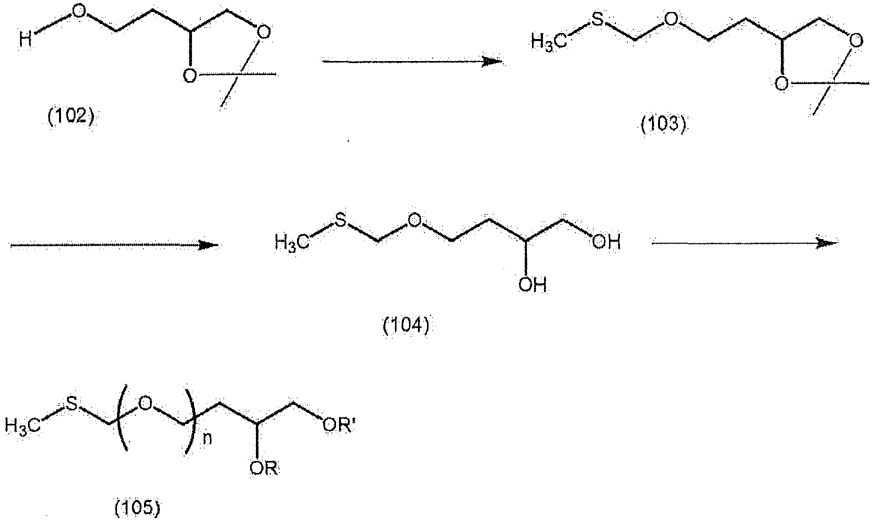 in each formula,
n is a positive integer, and
R and R' are the same or different and each is a hydrogen atom or a hydroxyl-protecting group.

18. The method according to claim 17, wherein the alcohol represented by the chemical formula (102) is obtained by protecting a diol represented by the following chemical formula (101) by cyclization:

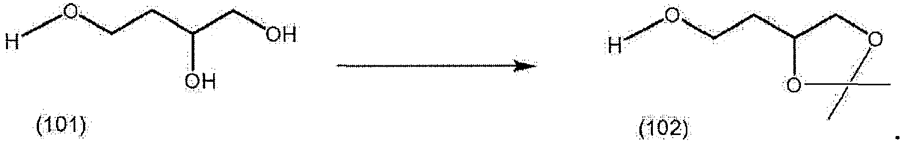

19. A method for producing a compound represented by the following chemical formula (102), comprising
(1) alkaline hydrolyzing a compound represented by the following chemical formula (101-1) to produce an alcohol represented by the following chemical formula (101-2);
(2) producing a compound represented by the following chemical formula (101-3) by protecting a hydroxyl group of the alcohol represented by the chemical formula (101-2);
(3) oxidizing the compound represented by the following chemical formula (101-3) to produce a compound represented by the following chemical formula (101-4);
(4) isopropylidenating the compound represented by the following chemical formula (101-4) to produce a compound represented by the following chemical formula (101-5); and
(5) deprotecting the compound represented by the following chemical formula (101-5): 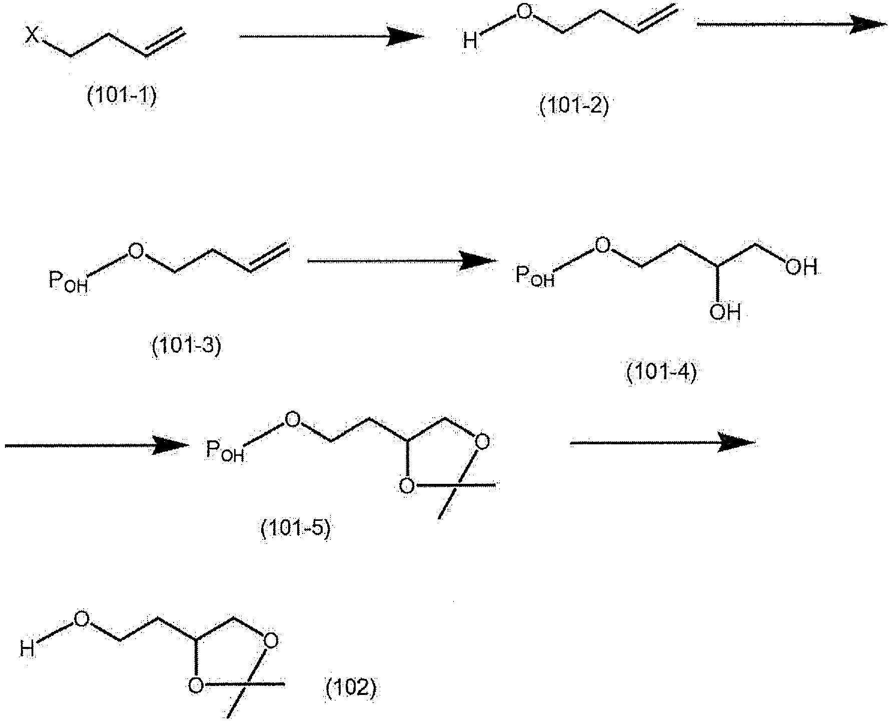 in each formula,
P_{OH} is a hydroxyl-protecting group, and
X is a halogen atom, an acetyloxy group, an optionally substituted carboxyl group or an optionally substituted carbamoyl group.

20. A method for producing ether represented by the following chemical formula (105), comprising
(1) protecting the 4-position hydroxyl group of a compound represented by the following chemical formula (104-1) to produce hydroxycarboxylic acid represented by the following chemical formula (104-2);
(2) protecting a hydroxyl group of the hydroxycarboxylic acid represented by the following chemical formula (104-2), and acylating same to produce a compound represented by the following chemical formula (104-3);
(3) deprotecting the 4-position of the compound represented by the following chemical formula (104-3) to produce a compound represented by the following chemical formula (104-4); and
(4) methylthiomethyling the compound represented by the following chemical formula (104-4), and repeating, where necessary, a coupling reaction with methylthiomethanol in the presence of a halogenating agent and a Lewis acid: 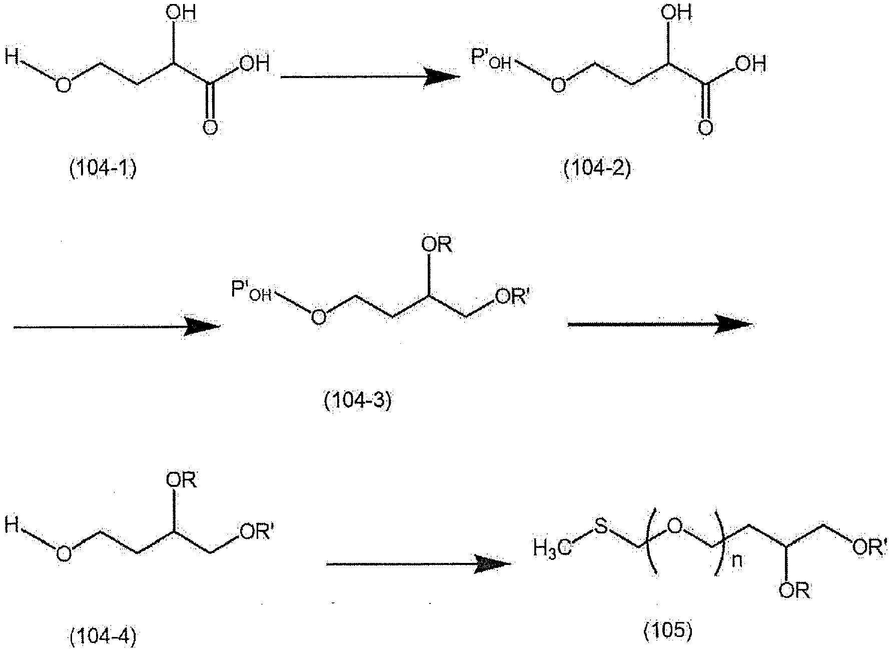 in each formula,
n is a positive integer,
P'_{OH} is a hydroxyl-protecting group, and
R and R' are the same or different and each is a hydrogen atom or a hydroxyl-protecting group.

21. The production method according to claim 17 or 20, wherein R and R' are acetyl groups.

22. Ether represented by the following chemical formula (105), an enantiomer, tautomer or stereoisomer thereof or a salt thereof: 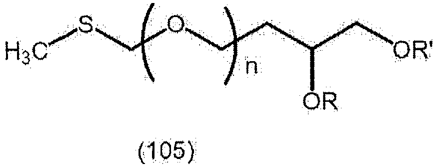 wherein,
n is a positive integer, and
R and R' are the same or different and each is a hydrogen atom or a hydroxyl-protecting group.

23. A production method of the glycoside compound according to any one of claims 1 to 16, an enantiomer, tautomer or stereoisomer thereof or a salt thereof, comprising a coupling step for producing a glycoside compound represented by the following chemical formula (1a) by subjecting a glycoside compound represented by the following chemical formula (106) and ether represented by the following chemical formula (105) to a coupling reaction in the presence of a halogenating agent and a Lewis acid, wherein
the glycoside compound represented by the following chemical formula (1a) is the glycoside compound wherein R¹ and R² in said chemical formula (1) in conjunction form an atomic group represented by said chemical formula (R¹R²A) or (R¹R²B): 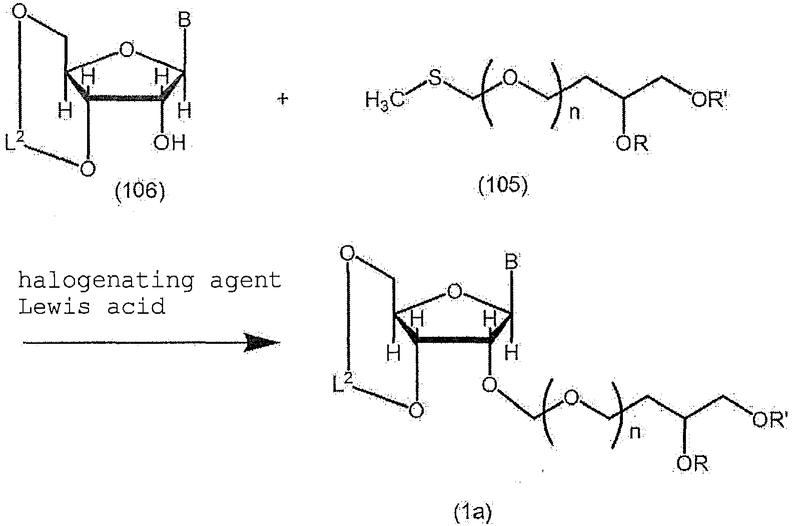 in said chemical formulas (106) and (1a),
L² is an atomic group represented by said chemical formula (R¹R²A) or (R¹R²B),
in said chemical formulas (105), (106) and (1a), B, n, R and R' are as defined for said chemical formula (1) .

24. The production method according to claim 23, further comprising a deprotection step for producing a glycoside compound represented by the following chemical formula (1b) by removing said atomic group L² from the glycoside compound represented by said chemical formula (1a), wherein
the glycoside compound represented by the following chemical formula (1b) is a glycoside compound of said chemical formula (1) wherein R¹ and R² are hydrogen atoms: 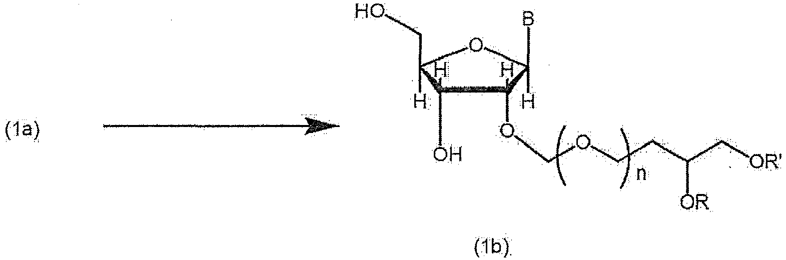 in said chemical formula (1b),
B, n, R and R' are as defined for said chemical formula (1) .

25. The production method according to claim 24, further comprising a protecting group introduction step for producing a glycoside compound represented by the following chemical formula (1c) by introducing protecting groups R¹ and R² into said chemical formula (1b), wherein
the glycoside compound represented by the following chemical formula (lc) is a glycoside compound of said chemical formula (1) wherein R¹ and R² are other than a hydrogen atom and said chemical formulas (R¹R²A) and (R¹R²B) : 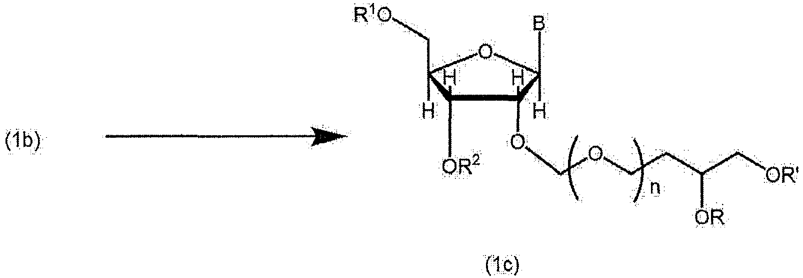 in said chemical formula (1c),
R¹ and R² are R¹ and R² in said chemical formula (1) and other than a hydrogen atom and said chemical formulas (R¹R²A) and (R¹R²B), and
B, n, R and R' are as defined for said chemical formula (1) .

26. The production method according to any one of claims 23 to 25, wherein,
said halogenating agent is at least one selected from the group consisting of N-chlorosuccinimide, N-bromosuccinimide, N-iodosuccinimide, iodine, bromine and chlorine.

27. The production method according to any one of claims 23 to 26, wherein said Lewis acid is at least one selected from the group consisting of perfluoroalkyl carboxylic acid, perfluoroalkyl sulfonic acid, alkyl sulfonic acid and a salt thereof.

28. The production method according to any one of claims 23 to 27, wherein said Lewis acid is a trifluoromethanesulfonic acid or methanesulfonic acid.

29. The production method according to any one of claims 23 to 28, wherein said coupling reaction is performed in the co-presence of a molecular sieve.

30. A nucleic acid comprising a structure represented by the following chemical formula (I), an enantiomer, tautomer or stereoisomer thereof: 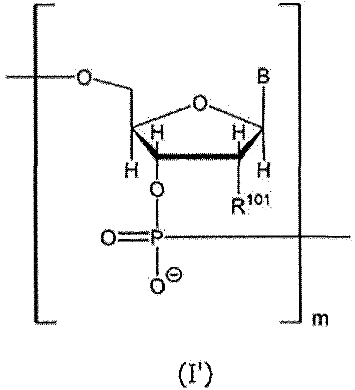 in said chemical formula (I'), B is the same as said chemical formula (1), (2) or (3),
R¹⁰¹ is a group represented by the following chemical formula (R³) or (R⁴), 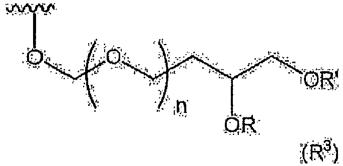 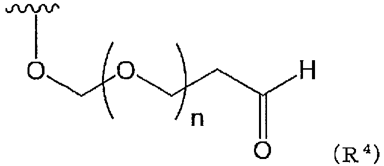 in said chemical formula,
n is a positive integer, R and R' are the same or different and each is a hydrogen atom or a hydroxyl-protecting group,
each B may be the same or different and each R¹⁰¹ may be the same or different, and
m is a positive integer.

31. A method for producing a nucleic acid comprising a structure represented by the following chemical formula (I), comprising
a condensation step for performing a condensation reaction of the glycoside compound according to any one of claims 7 to 16: 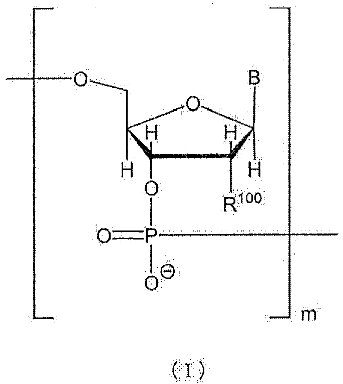 in said chemical formula (I), B is the same as said chemical formula (1), (2) or (3),
R¹⁰⁰ is a hydrogen atom or a hydroxyl group, or a group represented by the following chemical formula (R³) or (R⁴): 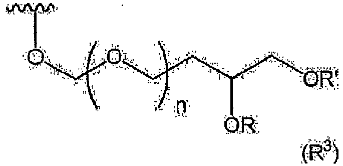 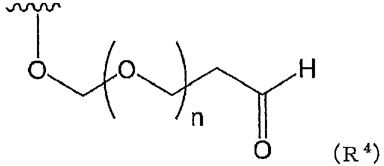 in said chemical formula,
n is a positive integer,
R and R' are the same or different and each is a hydrogen atom or a hydroxyl-protecting group,
each B may be the same or different, and each R¹⁰⁰ may be the same or different, and
m is a positive integer.

32. The method according to claim 31, comprising a step for deprotecting the 2'-position of the condensation compound obtained by the condensation reaction.

33. The method according to claim 32, wherein the deprotection is performed by (i) an ammonia treatment step, (ii) a periodic acid treatment step, and (iii) a tetraalkylammonium halide treatment step.

34. The method according to claim 33, comprising a step for protecting the 2'-position formyl group between (ii) the periodic acid treatment step and (iii) the tetraalkylammonium halide treatment step.

35. The method according to claim 34, wherein an amine compound is reacted in the step for protecting the 2'-position formyl group.

36. The method according to claim 35, wherein the amine compound is a benzylamine compound having an electron-withdrawing group.

37. The method according to any one of claims 31 to 36, wherein, in said chemical formula (I), each R¹⁰⁰ is a hydroxyl group.

38. The method according to any one of claims 31 to 36, wherein, in said chemical formula (I), each R¹⁰⁰ is a hydrogen atom, and the method further comprises
a reverse transcription step for producing a nucleic acid represented by said chemical formula (I) by reverse transcription from the nucleic acid obtained said condensation step.

39. The method according to claim 31, comprising a step for obtaining the nucleic acid comprising a structure represented by said chemical formula (I) wherein each R¹⁰⁰ is a hydroxyl group, by treating a nucleic acid comprising a structure represented by said chemical formula (I) wherein each R¹⁰⁰ is a group represented by the chemical formula (R⁴) with tetraalkylammoniumhalide.
